(19) Europäisches Patentamt
European Patent Office
Office européen des brevets

(11) EP 4 039 674 A1

(12) EUROPEAN PATENT APPLICATION
published in accordance with Art. 153(4) EPC

(43) Date of publication:
**10.08.2022 Bulletin 2022/32**

(21) Application number: **20872504.4**

(22) Date of filing: **29.09.2020**

(51) International Patent Classification (IPC):
***C07C 309/32*** (2006.01) ***C07D 213/71*** (2006.01)
***A61K 31/4412*** (2006.01) ***A61K 31/167*** (2006.01)
***A61P 31/12*** (2006.01) ***A61P 35/00*** (2006.01)
***A61P 37/02*** (2006.01)

(52) Cooperative Patent Classification (CPC):
**C07C 317/44; A61K 31/167; A61K 31/4412; A61P 31/12; A61P 35/00; A61P 37/02; C07C 309/32; C07C 317/46; C07D 213/71;**
C07C 2601/02

(86) International application number:
**PCT/CN2020/118905**

(87) International publication number:
**WO 2021/063362 (08.04.2021 Gazette 2021/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2019 CN 201910938067**
**15.09.2020 CN 202010966741**

(71) Applicant: **Shanghai Litedd Co., Ltd.**
**Shanghai 201203 (CN)**

(72) Inventors:
- **CHENG, Yaobang**
  **Shanghai 201203 (CN)**
- **HUANG, Yafei**
  **Shanghai 201203 (CN)**
- **ZHOU, Juan**
  **Shanghai 201203 (CN)**
- **DONG, Zhiqiang**
  **Shanghai 201203 (CN)**

(74) Representative: **Bianchetti & Minoja SRL**
**Via Plinio, 63**
**20129 Milano (IT)**

# (54) SULFO-SUBSTITUTED BIARYL COMPOUND OR SALT THEREOF, PREPARATION METHOD THEREFOR, AND USE THEREOF

(57) The present disclosure relates to a sulfo-substituted biaryl derivative compound or a salt thereof, a preparation method and use thereof, in particular to the compound of formula (I) wherein $R_1$, $R_2$, $R_3$, $R_4$, $R_5$, $R_5$’, $R_6$, $R_7$ and X as defined in the specification or its stereoisomers, tautomers, stable isotopic derivatives, pharmaceutically acceptable salts or solvates, a method for their preparation, a pharmaceutical composition comprising the same, and use of the compounds in the manufacture of a medicament for the treatment or prevention of a disease associated with RORγt.

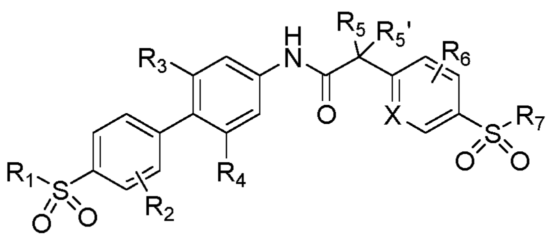

(I)

EP 4 039 674 A1

## Description

### Technical field

The disclosure belongs to the technical field of chemical medicines, in particular to a sulfo-substituted biaryl compound with RORγt agonistic activity, a preparation method thereof, a pharmaceutical composition containing the compound, and use of the sulfo-substituted biaryl compound in the preparation of a medicament for treating diseases related to RORγt.

### Background technique

Tumor immunotherapy has recently attracted much attention and has become the focus of the field of tumor therapy. Tumor immunotherapy is to control and kill tumor cells by mobilizing the body's immune system and enhancing the anti-tumor immunity of the tumor microenvironment. It targets the human immune system rather than the tumor directly. In recent years, good news of tumor immunotherapy keeps coming, showing strong anti-tumor activity so far in the treatment of some tumor types such as melanoma and non-small cell lung cancer, and there have been tumor-immune monoclonal antibody agents approved by FDA for clinical use. Tumor immunotherapy was named "The Most Important Scientific Breakthrough" of the year by the journal Science in 2013 due to its excellent efficacy and innovativeness. Due to their original discoveries in the field of tumor immunity, American scientist James P. Allison and Japanese scientist Tasuku Honjo were awarded the 2018 Nobel Prize in Physiology or Medicine. Therefore, tumor immunotherapy is expected to become an innovation in the field of tumor treatment, following surgery, chemotherapy, radiotherapy and targeted therapy.

Helper T cells 17 (Th17) is a newly discovered subtype of helper T cells, mainly secreting interleukin-17 (IL-17), and has been found to play an important role in the development of autoimmunity and inflammation. At present, researches have found that Th17 cells widely exist in tumor tissues, but nothing is known about the function of Th17 cells in them. In 2009, Professor Dong Chen published an article in *Immunity* (Immunity 2009, 31, 787-798), mainly analyzing that Th17 cells can promote the activation of cytotoxic T cells to exert tumor immune function. Their research found that mice deficient in IL-17A were more prone to develop pulmonary melanoma. If T-cell therapy is administered to mice, treatment with EL-17A-secreting T cells can effectively prevent tumor development. More importantly, with the aid of IL-17A, Th17 cells showed stronger therapeutic efficacy than Th1 cells. What is even more surprising is that, the use of Th17 cell therapy can also effectively activate tumor-specific CD8 $^+$ T cells, wherein, CD8 $^+$ T cells are essential cells for anti-tumor. Th17 cells can recruit dendritic cells into tumor tissue, and can induce CD8α+ dendritic cells to accumulate into tumor tissue. In addition, Th17 cells activate the chemokine CCL20 in tumor tissue. Overall, Th17 cells can effectively promote the activity of tumor-specific CD8$^+$T cells. These new findings broaden the horizons of tumor immunotherapy.

Retinoid acid receptor-related orphan receptor (ROR), also known as NF1R, is a member of the ligand-dependent transcription factor nuclear receptor (NR) superfamily. The RORs subfamily mainly includes three members: RORα, RORβ and RORγ. RORγ mainly includes two subtypes, RORγ1 (RORγ) and RORγ2 (RORγt), in which RORγ is distributed in skeletal muscle, thymus, testis, pancreas, prostate, heart and liver, etc., while RORγt is only expressed in some immune cells. Researches have reported that Th17 cells specifically express RORγt, and the activation of RORγt can promote the differentiation of Th17 cells and produce the pro-inflammatory cytokine IL-17. Therefore, it is theoretically possible to increase the differentiation of Th17 cells by activating RORγt, thereby promoting the activity of tumor-specific CD8$^+$T cells and exerting tumor immune function (ACS Chem. Biol. 2016, 11, 1012-1018).

On June 9, 2015, Celgene reached an agreement with Lycera for an anti-tumor T-cell drug at an upfront payment of $ 82.5 million plus a recent payment of $ 22.5 million. Lycera announced that substantial evidence has been achieved that its oral RORγt agonist can improve T cell efficacy, increase IL-17 production and promote Tc cell expression, thereby stimulating immune responses against cancer cells, resulting in durable tumor cell-killing effects (ONCOIMMUNOLOGY, 2016, 5, e1254854-1~e1254854-13). So far, Lycera's RORγt agonist small molecule compound LYC-55716 has entered phase 2 clinical trials, which fully demonstrates the great potential of RORγt agonists for small molecule tumor immunotherapy.

In 2018, Journal of Medicinal Chemistry published a review of small molecule RORγt agonists, detailing the structural types and characteristics of some RORγt agonists (J. Med. Chem. 2018, 61, 5794-5804). Most of these small-molecule compounds have defects in activity or druggability. So far, only one compound, LYC-55716, has entered clinical trials. Therefore, it is of great significance to find high-quality small-molecule RORγt agonists for this potential tumor immunotherapy target and apply them to virological infection and cancer treatment.

## SUMMARY OF THE INVENTION

In one aspect, the present disclosure provides compounds of formula (I) or stereoisomers, tautomers, stable isotopic derivatives, pharmaceutically acceptable salts or solvates thereof:

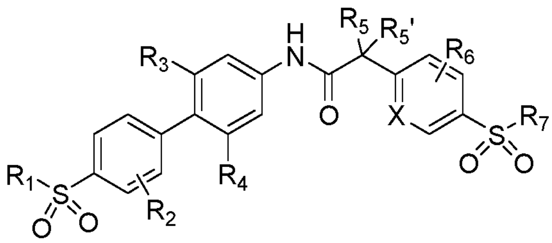

**(I)**

wherein,

$R_1$ is selected from optionally substituted $C_1$-$C_6$alkyl, optionally substituted $C_3$-$C_6$ cycloalkyl and optionally substituted 3-6 membered heterocycloalkyl, each of the substitutents is independently selected from hydrogen, halogen, cyano, nitro, hydroxyl, mercapto, $C_1$-$C_6$ alkyl, $C_1$-$C_6$ alkoxy, $C_1$-$C_6$ alkylthio and $C_3$-$C_6$ cycloalkyl;

$R_2$, $R_3$, $R_4$ are each independently selected from hydrogen, halogen, cyano, nitro, hydroxyl, mercapto, optionally substituted $C_1$-$C_6$ alkyl, optionally substituted $C_1$-$C_6$ alkoxy and an optionally substituted $C_1$-$C_6$ alkylthio group, each of the substitutents is independently selected from hydrogen, halogen, hydroxyl, mercapto, nitro and cyano;

$R_5$ and $R_5$' are each independently selected from hydrogen, hydroxy, halogen, nitro, cyano, optionally substituted $C_1$-$C_6$ alkyl, optionally substituted $C_1$-$C_6$ alkoxy, optionally substituted $C_1$-$C_6$ alkylthio and optionally substituted $C_3$-$C_6$ cycloalkyl, each of the substitutents is independently selected from hydrogen, halogen, nitro, cyano, hydroxyl, mercapto, $C_1$-$C_6$ alkoxy and $C_1$-$C_6$ alkylthio;

$R_6$ is selected from hydrogen, halogen, cyano, hydroxyl, mercapto, nitro, optionally substituted $C_1$-$C_6$ alkyl, optionally substituted $C_1$-$C_6$ alkoxy and optionally substituted $C_1$-$C_6$ alkylthio, each of the substituents is independently selected from hydrogen, halogen, nitro, cyano, hydroxyl and mercapto;

$R_7$ is selected from optionally substituted $C_1$-$C_6$ alkyl, optionally substituted $C_3$-$C_6$ cycloalkyl, optionally substituted 3-6 membered heterocycloalkyl, and an amino optionally substituted by $C_1$-$C_6$ alkyl, $C_3$-$C_6$ cycloalkyl or 3-6 membered heterocycloalkyl, each of the substitutents is independently selected from hydrogen, halogen, cyano, nitro, hydroxyl, mercapto, $C_1$-$C_6$ alkoxy, $C_1$-$C_6$ alkylthio, $C_3$-$C_6$ cycloalkyl, and an amino optionally substituted with $C_1$-$C_6$ alkyl; and

X is selected from CH and N.

In some preferred embodiments, $R_1$ is selected from optionally substituted $C_1$-$C_6$ alkyl and optionally substituted $C_3$-$C_6$ cycloalkyl, each of the substituents is independently selected from halogen, $C_3$-$C_6$ cycloalkyl and $C_1$-$C_6$ alkoxy, such as halogen substituted $C_1$-$C_6$ alkyl.

Preferably, $R_1$ is selected from $C_1$-$C_6$ alkyl and $C_3$-$C_6$ cycloalkyl, such as but not limited to methyl, ethyl, n-propyl, isopropyl, n-butyl, isobutyl, sec-butyl, tert-butyl, n-pentyl, isopentyl, neopentyl, hexyl, cyclopropyl, cyclobutyl, cyclopentyl and cyclohexyl.

More preferably, $R_1$ is selected from $C_1$-$C_3$ alkyl and $C_3$-$C_5$ cycloalkyl.

More preferably, $R_1$ is selected from methyl, ethyl, propyl and cyclopropyl.

In some preferred embodiments, $R_2$ is selected from H and halogen, such as fluorine, chlorine, bromine, iodine; preferably, $R_2$ is H.

In some preferred embodiments, $R_3$ and $R_4$ are each independently selected from hydrogen, halogen, cyano, $C_1$-$C_6$ alkyl optionally substituted with halogen and $C_1$-$C_6$ alkoxy optionally substituted with halogen; such as but not limited to fluorine, chlorine, bromine, iodine, cyano, and 1, 2 or 3 halogens (preferably fluorine)-substituted methyl, ethyl, propyl, isopropyl, methoxy, ethoxy, propoxy or isopropoxy.

More preferably, $R_3$ and $R_4$ are each independently selected from hydrogen, halogen, methyl, trifluoromethyl,

trifluoromethoxy and difluoromethoxy; further preferably, $R_3$ and $R_4$ are each independently selected from halogen; most preferably, both $R_3$ and $R_4$ are chlorine.

In some preferred embodiments, $R_5$ and $R_5$' are each independently selected from hydrogen, substituted $C_1$-$C_3$ alkyl, and substituted $C_3$-$C_6$ cycloalkyl, each of the substituetns is independently selected from hydroxy, $C_1$-$C_3$ alkoxy and halogen, such as halogen substituted ($C_1$-$C_3$ alkoxy $C_1$-$C_3$ alkyl); such as but not limited to hydroxy substituted methyl, ethyl, propyl, isopropyl, cyclopropyl, cyclobutyl, cyclopentyl; halogen and/or methoxy or ethoxy substituted methyl, ethyl, cyclopropyl or cyclobutyl; preferably, $R_5$ is selected from hydrogen, $-CH_2OH$, $-CH_2OCH_3$,

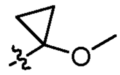 and 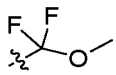,

and $R_5$' is H; more preferably, $R_5$ is selected from hydrogen and $-CH_2OCH_3$, and $R_5$' is H.

In some preferred embodiments, $R_6$ is selected from hydrogen and halogen, preferably, $R_6$ is hydrogen.

In some preferred embodiments, $R_7$ is selected from $C_1$-$C_6$ alkyl; $C_1$-$C_6$ alkyl substituted with hydroxy, $C_1$-$C_6$ alkoxy or $C_3$-$C_6$ cycloalkyl; $C_3$-$C_6$ cycloalkyl; and $C_3$-$C_6$ cycloalkyl substituted with hydroxy, $C_1$-$C_6$ alkyl or $C_1$-$C_6$ alkoxy.

Preferably, $R_7$ is selected from $C_1$-$C_6$ alkyl, $C_1$-$C_6$ alkyl substituted with hydroxy or $C_3$-$C_6$ cycloalkyl, and $C_3$-$C_6$ cycloalkyl; such as but not limited to methyl, ethyl, n-propyl, isopropyl, n-butyl, isobutyl, sec-butyl, tert-butyl, pentyl, hydroxy; methyl, ethyl or propyl substituted with cyclopropyl or cyclobutyl; cyclopropyl, cyclobutyl, cyclopentyl or cyclohexyl.

More preferably, $R_7$ is selected from methyl, ethyl, cyclopropylmethyl and $-CH_2CH_2-OH$, most preferably methyl, ethyl and $-CH_2CH_2-OH$.

In some embodiments of the disclosure, the compound has the structure of formula (II):

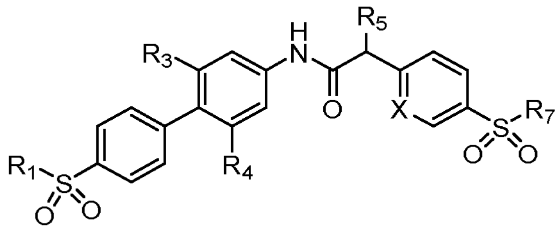

**(II)**

wherein: $R_1$, $R_3$, $R_4$, $R_5$, $R_7$ and X are as defined above for formula (I).

Most preferably, the compound of formula (I) or (II) of the present disclosure is selected from the following specific compound examples:

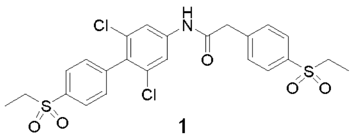

1

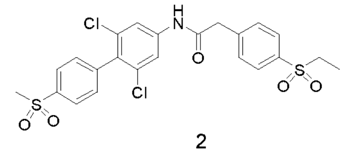

2

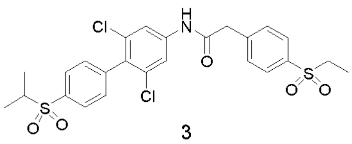

3

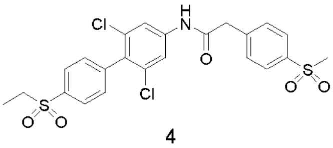

4

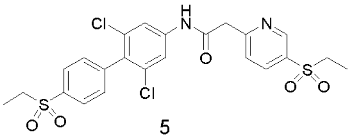

5

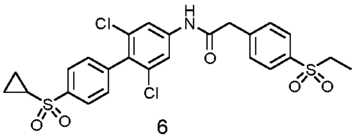

6

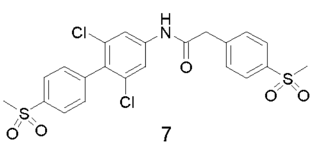

7

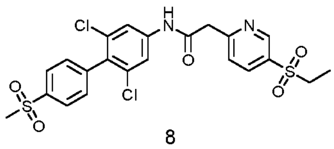

8

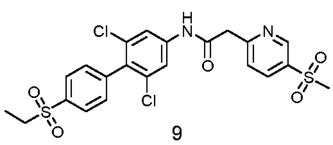

9

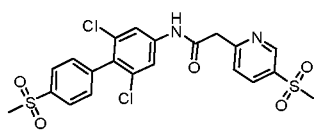

10

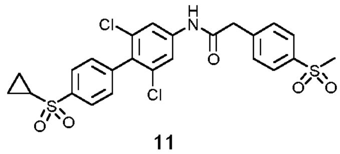

11

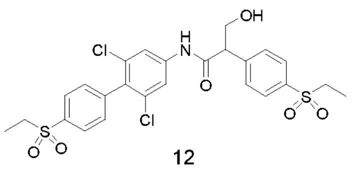

12

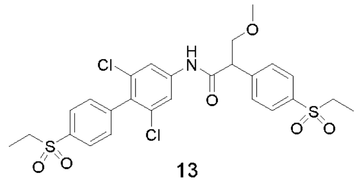

13

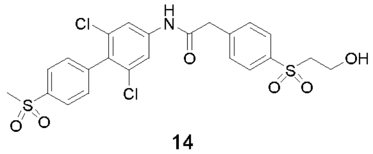

14

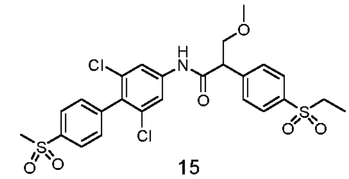

15

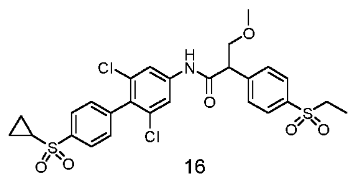

16

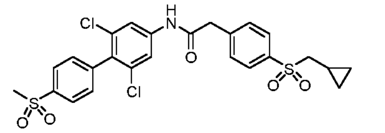

17

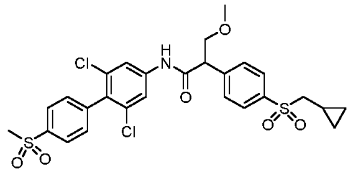

18

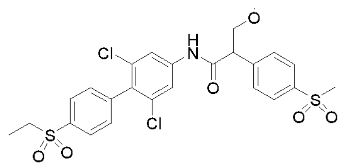

19

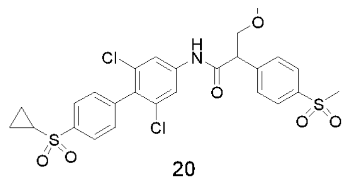

20

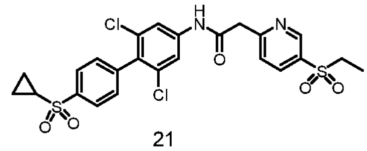

21

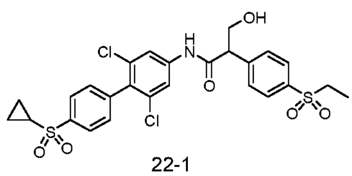

22-1

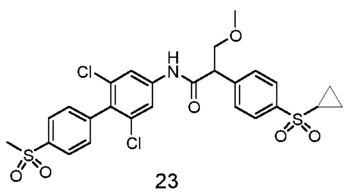

23

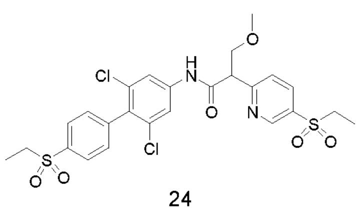

24

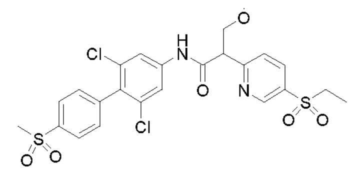

25

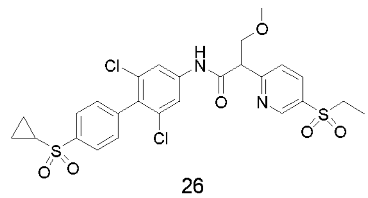

26

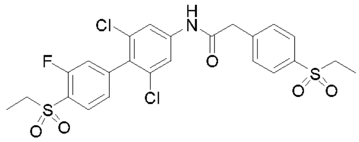

27

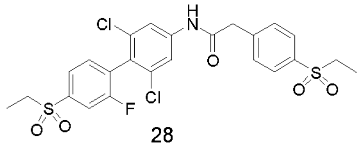

28

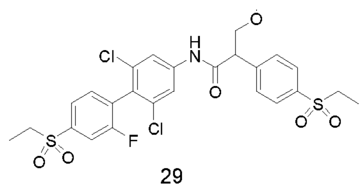

29

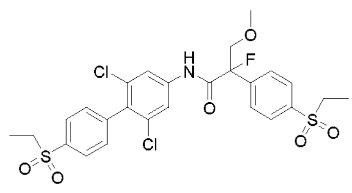

30

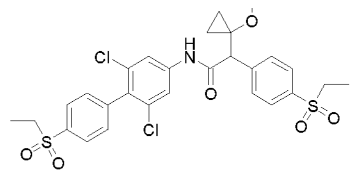

31

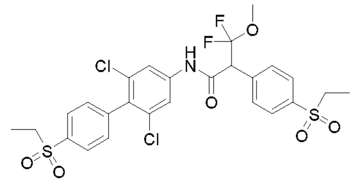

32

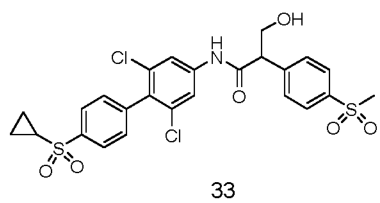

33

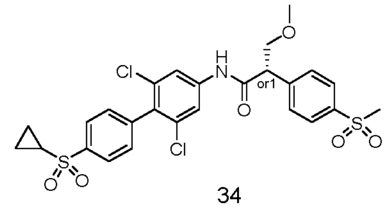

34

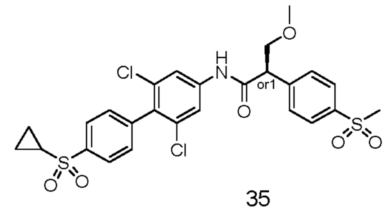

35

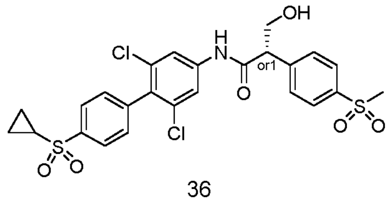

36

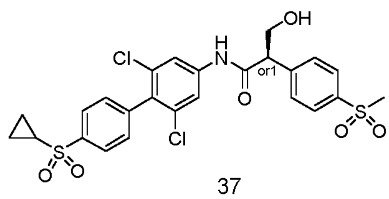

37

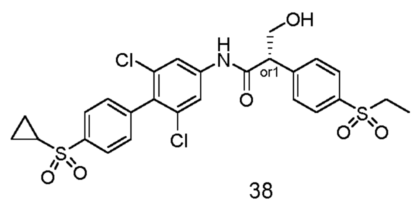

38

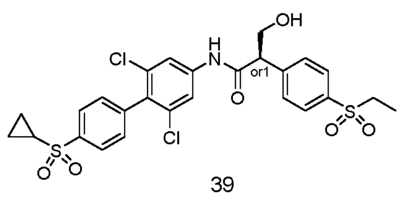

39

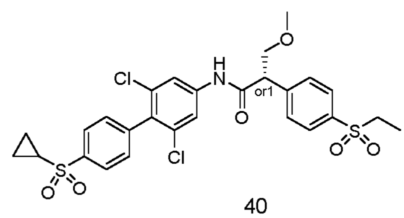

40

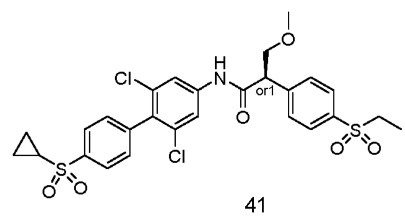

41

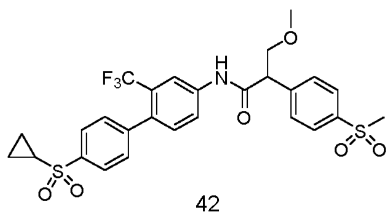

42

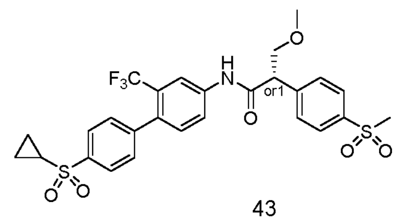

43

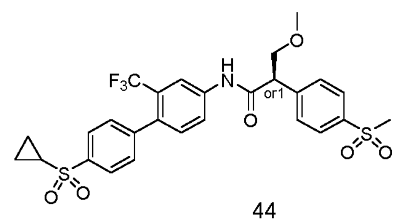

44

.

In a second aspect, the present disclosure also provides a process for the preparation of compounds of formula (I). General synthetic schemes for synthesizing the compounds of the present disclosure are exemplified below. For each reaction step, appropriate reaction conditions are known to those skilled in the art or can be routinely determined. The starting materials and reagents used in the preparation of these compounds are generally commercially available unless otherwise specified, or can be prepared by the methods below, the methods analogous to those given below, or the methods known in the art. If necessary, the starting materials and intermediates in the synthetic schemes can be separated and purified by conventional techniques, including but not limited to filtration, distillation, crystallization, chromatography and the like. The materials can be characterized using conventional methods including physical constants and spectral data.

In one embodiment, the process includes the steps of:

## Synthesis Scheme 1

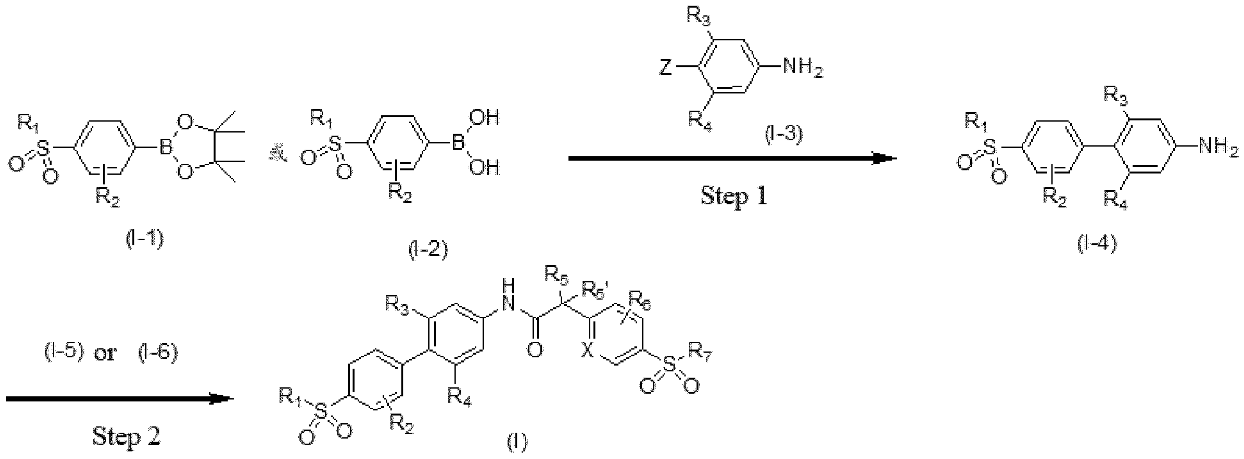


(I-1) (I-2) (I-4) (I)

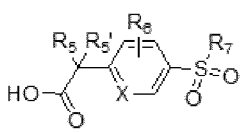

(I-5)

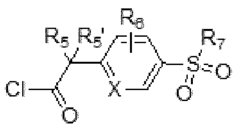

(I-6)

Z= Br or I

wherein $R_1$, $R_2$, $R_3$, $R_4$, $R_5$, $R_5$', $R_6$, $R_7$ and X are as defined above for formula (I), and Z is Br or I.

Step 1: a compound of formula (1-1) or formula (1-2) is reacted with a compound of formula (I-3) by coupling reaction such as Suzuki coupling reaction in the presence of a catalyst such as a palladium catalyst, to obtain compound of formula (1-4).

In Step 1, the palladium catalyst is a well-known palladium catalyst for Suzuki coupling in the art, including but not limited to $PdCl_2$(dtbpf), Pd(dppf)$Cl_2$ etc.; the reaction is preferably carried out in a suitable organic solvent which can be selected from tetrahydrofuran, ethers (such as diethyl ether, ethylene glycol monomethyl ether, etc.), N-methylpyrrolidone, N,N-dimethylformamide, dimethylacetamide, dichloromethane, 1,4- dioxane, dimethyl sulfoxide and any combination thereof, preferably 1,4- dioxane; the reaction is preferably carried out in the presence of a suitable base which can be selected from sodium hydroxide, potassium hydroxide, sodium carbonate, potassium carbonate and cesium carbonate, preferably, the base is potassium carbonate; the reaction is preferably carried out at a suitable temperature, such as 50-200°C, 80-150°C, preferably 90-120°C.

Step 2: the compound of formula (1-4) is reacted with a carboxylic acid of formula (1-5) in the presence of a condensing agent, or the compound of formula (1-4) is reacted with an acyl chloride of formula (1-6) under the action of a base, to obtain a compound of formula (I).

In Step 2, the condensing agent is one well known in the art for coupling carboxylic acid with amine, including but not limited to EDC, DCC, HATU, etc.; the reaction is preferably carried out in a suitable organic solvent which can be selected from tetrahydrofuran, ethers (such as diethyl ether, ethylene glycol monomethyl ether, etc.), N-methylpyrrolidone, N,N-dimethylformamide, dimethylacetamide, dichloromethane, 1,4- dioxane, dimethyl sulfoxide and any combination thereof; the reaction is preferably carried out in the presence of a suitable base including but not limited to sodium carbonate, potassium carbonate, cesium carbonate, *N,N*-diisopropyl ethylamine, triethylamine, HOBt or pyridine, preferably, the base is *N,N*-diisopropyl ethylamine; the reaction is preferably carried out at a suitable temperature, such as 0-200°C, 10-100°C or 20-50°C, preferably room temperature (20-25°C).

In another embodiment, the process includes the steps of:

## Synthesis Scheme 2

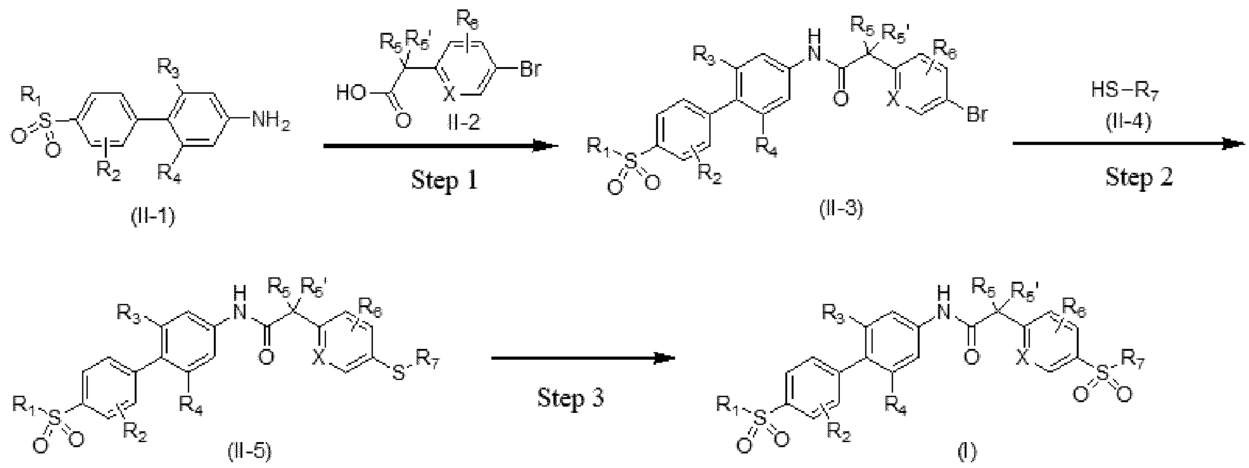


wherein $R_1$, $R_2$, $R_3$, $R_4$, $R_5$, $R_5$', $R_6$, $R_7$ and X are as defined above for formula (I), and Z is Br or I.

Step 1: a compound of formula (II-1) is condensed with a carboxylic acid of formula (II-2) in the presence of a condensing agent to obtain a compound of formula (II-3).

In Step 1, the condensing agent is one well known in the art for coupling carboxylic acid with amine, including but not limited to EDC, DCC, HATU, etc.; the reaction is preferably carried out in a suitable organic solvent which can be selected from tetrahydrofuran, ethers (such as diethyl ether, ethylene glycol monomethyl ether, etc.), N-methylpyrrolidone, N,N-dimethylformamide, dimethylacetamide, dichloromethane, 1,4- dioxane, dimethyl sulfoxide and any combination thereof; the reaction is preferably carried out in the presence of a suitable base including but not limited to sodium carbonate, potassium carbonate, cesium carbonate, *N*,*N*-diisopropyl ethylamine, triethylamine, HOBt or pyridine, preferably, the base is *N*,*N*-diisopropyl ethylamine; the reaction is preferably carried out at a suitable temperature, such as 0-200°C, 10-100°C or 20-50°C, preferably room temperature (20-25°C).

Step 2: the compound of formula (II-3) is reacted with a thiol of formula (II-4) under the action of a catalyst such as a palladium catalyst, to obtain a compound of formula (II-5).

In Step 2, the palladium catalyst includes but is not limited to $Pd_2(dba)_3$; the reaction is preferably carried out in a suitable organic solvent which can be selected from tetrahydrofuran, ethers (such as diethyl ether, ethylene glycol monomethyl ether, etc.), N-methylpyrrolidone, N,N-dimethylformamide, dimethylacetamide, dichloromethane, 1,4-dioxane, dimethyl sulfoxide and any combination thereof, preferably 1,4-dioxane; the reaction is preferably carried out in the presence of a suitable base which can be selected from *N*,*N*-diisopropyl ethylamine, triethylamine, HOBt and pyridine, preferably, the base is *N*,*N*-diisopropyl ethylamine; the reaction is preferably carried out in the presence of xantphos, and preferably at a suitable temperature, such as 50-200°C or 80-150°C, preferably 90-120°C.

Step 3: The compound of formula (II-5) is oxidized to give a compound of formula (I).

In Step 3, the oxidizing agent that can be used for the above-mentioned oxidation is well known in the art, including but not limited to m-chloroperoxybenzoic acid, peroxyacetic acid or hydrogen peroxide; the reaction is preferably carried out in a suitable organic solvent selected from tetrahydrofuran, ethers (such as diethyl ether, ethylene glycol monomethyl ether, etc.), N-methylpyrrolidone, N,N-dimethylformamide, dimethylacetamide, dichloromethane, 1,4-dioxane, dimethyl sulfoxide and any combination thereof, preferably dichloromethane; the reaction is preferably carried out at a suitable temperature, such as -50-100°C, 0-80°C or 20-50°C, preferably room temperature (20-25°C).

The above synthetic schemes only exemplify the preparation of some compounds of the present disclosure. The compounds of the present disclosure, or stereoisomers, tautomers, stable isotopic derivatives, pharmaceutically acceptable salts or solvates thereof can be prepared by a variety of methods, including the methods given above, the methods given in the Examples or a method analogous to them, by those of ordinary skill in the art on the basis of the above-mentioned synthetic schemes in combination with conventional techniques in the art.

In the third aspect, the present disclosure provides a compound of formula (I) or (II) as defined above, or a stereoisomer, tautomer, stable isotopic derivatives, pharmaceutically acceptable salts or solvates, for use as a medicament, in particule as a RORγt agonist.

In the fourth aspect, the present disclosure also provides a pharmaceutical composition comprising the compound of formula (I) or (II) as described above or a stereoisomer, tautomer, stable isotopic derivative, pharmaceutically acceptable salt or solvate thereof as the active ingredient, and one or more pharmaceutically acceptable carriers.

In the fifth aspect, the present disclosure provides the use of a compound of formula (I) or (II) as described

above or a stereoisomer, tautomer, stable isotope derivative, pharmaceutically acceptable salt or solvate thereof in the manufacture of a medicament with RORyt receptor agonistic activity.

The present disclosure preferably provides, in the fifth aspect, the use of a compound of formula (I) or (II) as described above or a stereoisomer, tautomer, stable isotopic derivative, pharmaceutically acceptable salt or solvate thereof or a pharmaceutical composition comprising the same in the manufacture of a medicament for the treatment or prevention of a disease associated with RORyt, such as a disease treatable or preventable by activation of RORyt, wherein the above compound or pharmaceutical composition is optionally combined with one or more chemotherapies or immunotherapies.

In the sixth aspect, the present disclosure also provides a method of preventing or treating a disease associated with RORyt (eg, a disease treatable or preventable by activation of RORyt), comprising administering to an individual in need thereof an effective dose of the compound of formula (I) or (II) as described above or its stereoisomers, tautomers, stable isotopic derivatives, pharmaceutically acceptable salts or solvates thereof or pharmaceutical compositions comprising them.

In the seventh aspect, the present disclosure provides a pharmaceutical combination, comprising the concurrent or sequential administration of a compound of formula (I) or (II) as described above or a stereoisomer, tautomer, stable isotope derivative, pharmaceutically acceptable salts or solvates thereof or pharmaceutical compositions comprising them, and one or more other agents that act by the same or a different mechanism of action. Such other agents are those which, when used in combination with the compounds of the present disclosure, produce a combined beneficial effect.

For the various aspects of the disclosure described above, the disease associated with RORyt is selected from tumors and cancers, including but not limited to colon cancer, pancreatic cancer, breast cancer, ovarian cancer, prostate cancer, squamous cell carcinoma, basal cell carcinoma, adenocarcinoma, sweat gland cancer, sebaceous gland cancer, lung cancer, leukemia, bladder cancer, stomach cancer, cervical cancer, testicular cancer, skin cancer, rectal cancer, thyroid cancer, kidney cancer, uterine cancer, pemphigus cancer, liver cancer, acoustic neuroma, oligodendroglioma, meningioma, neuroblastoma, eye cancer.

For the above-mentioned compounds of the disclosure for use as medicaments, prevention or treatment methods of the disclosure, pharmaceutical compositions, pharmaceutical combinations or uses, the respective preferred embodiments of the compounds of formula (I) or (II) as defined above are preferred, more preferrably the specific compounds listed above, namely Compound 1 to Compound 44.

## Definitions

Unless otherwise defined herein, all technical and scientific terms used herein are intended to have the same meaning as commonly understood by those skilled in the art. References to techniques used herein are intended to refer to techniques commonly understood in the art, including those variations or substitutions of equivalent techniques that would be apparent to those skilled in the art. While the following terms are believed to be well understood by those skilled in the art, the following definitions are set forth to better illustrate the present disclosure.

The term "halogen" as used herein refers to fluorine, chlorine, bromine or iodine, preferably fluorine or chlorine.

The term "hydroxyl" as used herein refers to -OH.

The term "alkyl" as used herein refers to a straight or branched chain saturated hydrocarbon group consisting of carbon atoms and hydrogen atoms. Alkyl groups have 1 to 10, such as 1 to 6, such as 1 to 4, or 1 to 3 carbon atoms. For example, as used herein, the term "$C_1$-$C_6$ alkyl" refers to a straight or branched chain saturated hydrocarbon group having 1 to 6 carbon atoms, such as methyl, ethyl, propyl (including n-propyl and isopropyl), butyl (including n-butyl, isobutyl, sec-butyl or tert-butyl), pentyl (including n-pentyl, isopentyl, neopentyl), n-hexyl, 2-methylpentyl, etc.; the alkyl group can be unsubstituted or substituted by one or more substituents, including but not limited to alkyl, alkoxy, alkylthio, cyano, hydroxyl, mercapto, carbonyl, carboxyl, aryl, heteroaryl, amino, halogen, sulfonyl, sulfinyl, phosphoryl.

The term "alkoxy" as used herein, refers to an alkyl group as defined above which is attached to the parent molecular moiety via an oxygen atom, eg, $C_1$-$C_6$ alkoxy or $C_1$-$C_3$ alkoxy. Representative examples of alkoxy include but are not limited to methoxy, ethoxy, propoxy (including n-propoxy, isopropoxy), butoxy (including n-butoxy, isobutoxy, tert-butoxy), pentyloxy (including n-pentyloxy, isopentyloxy, neopentyloxy), hexyloxy (including n-hexyloxy, isohexyloxy) and the like.

The term "alkylthio" as used herein refers to a -S-alkyl group, wherein the alkyl group is as defined above for "alkyl".

The term "halogen-substituted $C_1$-$C_6$ alkyl" as used herein refers to the $C_1$-$C_6$ alkyl groups described above wherein one or more (eg. 1, 2, 3, 4 or 5) hydrogen atoms are replaced by halogens. It will be understood by those skilled in the art that when there is more than one halogen substituent, the halogens may be the same or different, and may be located on the same or different C atoms. Examples of "halogen substituted $C_1$-$C_6$ alkyl" are eg. $-CH_2F$, $-CHF_2$, $-CF_3$, $-CCl_3$, $-C_2F_5$, $-C_2Cl_5$, - $CH_2CF_3$, $-CH_2Cl$ or $-CH_2CH_2CF_3$, etc.

The term "halogen-substituted $C_1$-$C_6$ alkoxy" as used herein refers to the $C_1$-$C_6$alkoxy groups described above wherein one or more (eg. 1, 2, 3, 4 or 5) hydrogen atoms are replaced by halogens. It will be understood by those skilled in the art that when there is more than one halogen substituent, the halogens may be the same or different, and may be located on the same or different C atoms. Examples of "halogen substituted $C_1$-$C_6$ alkoxy" are eg. $-OCH_2F$, $-OCHF_2$, $-OCF_3$, $-OCCl_3$, $-OC_2F_5$, $-OC_2Cl_5$, $-OCH_2CF_3$, $-OCH_2Cl$ or $-OCH_2CH_2CF_3$, etc..

Similarly, the term "halogen substituted ($C_1$-$C_3$ alkoxy $C_1$-$C_3$ alkyl)-" refers to a $C_1$-$C_3$ alkoxy $C_1$-$C_3$ alkyl group wherein one or more (eg. 1, 2, 3, 4 or 5) hydrogen atoms of the $C_1$-$C_3$alkoxy or $C_1$-$C_3$ alkyl moiety, preferably the $C_1$-$C_3$ alkyl moiety, are replaced by halogen, preferably fluorine.

The term "cycloalkyl" as used herein refers to a saturated or unsaturated non-aromatic monocyclic or polycyclic (such as bicyclic) hydrocarbon ring (eg. monocyclic, such as cyclopropyl, cyclobutyl, cyclopentyl, cyclohexyl, cyclopentenyl, cycloheptyl, cyclooctyl, cyclononyl, or bicyclic, including spiro, fused or bridged systems (such as bicyclo[1.1.1]pentyl, bicyclo[2.2.1]heptyl, spiro[3.4]octyl, bicyclo[3.1.1]hexyl, etc.). The cycloalkyl group has 3 to 10 carbon atoms, such as 3 to 8 carbon atoms, 3 to 7 carbon atoms, 3 to 6 carbon atoms or 3 to 5 carbon atoms. For example, as used herein, the term "$C_3$-$C_6$ cycloalkyl" refers to a saturated or unsaturated non-aromatic monocyclic or polycyclic (such as bicyclic) hydrocarbon rings having 3 to 6 ring carbon atoms, preferably the term "$C_3$-$C_6$ cycloalkyl" refers herein to saturated monocyclic rings having 3 to 6 ring carbon atoms, such as cyclopropyl, cyclobutyl, cyclopentyl, cyclohexyl.

The term "heterocycloalkyl" as used herein refers to a monocyclic or polycyclic non-aromatic group having 2, 3, 4, 5, 6, 7, 8, 9 carbon atoms and one or more (eg. 1, 2, 3 or 4) groups selected from C(=O), O, S, S(=O), $S(=O)_2$, N and NR (R represents hydrogen atom or substituents such as but not limited to alkyl or cycloalkyl) in the ring. As used herein, the term "3-6 membered heterocycloalkyl" means a heterocyclyl group containing 3-6 ring atoms, wherein 1 or more ring atoms are selected from N, O (optionally oxidized) and S (optionally oxidized) heteroatoms, this term includes 3, 4, 5 or 6 membered heterocycloalkyl, examples of which include but are not limited to oxiranyl, aziridinyl, azetidinyl, oxetanyl, tetrahydrofuranyl, pyrrolidinyl, pyrrolidinonyl, imidazolidinyl, pyrazolidinyl, tetrahydropyranyl, piperidinyl, morpholinyl, thiomorpholinyl, piperazinyl, piperazino, morpholino, piperidino, etc..

The term "aryl" as used herein refers to an all-carbon monocyclic or fused ring having a fully conjugated π-electron system, and typically having 6-14 carbon atoms, preferably 6-12 carbon atoms, most preferably 6 carbon atoms. Aryl groups may be unsubstituted or substituted with one or more substituents including, but not limited to alkyl, alkoxy, cyano, hydroxyl, carbonyl, carboxyl, aryl, aralkyl, amino, halogen, sulfonyl, sulfinyl, phosphoryl. Examples of unsubstituted aryl groups include but are not limited to phenyl, naphthyl and anthracenyl.

The term "heteroaryl" as used herein refers to a monocyclic or fused ring having 5-12 ring atoms, preferably a monocyclic ring of 5-6 ring atoms, containing 1-4 (eg. 1, 2, 3 or 4) heteroatoms selected from N, O (optionally oxidized), and S (optionally oxidized), the remaining ring atoms being C, and having a fully conjugated π-electron system, including but not limited to pyrrolyl, furyl, thienyl, imidazolyl, oxazolyl, isoxazolyl, pyrazolyl, pyridyl, pyrimidinyl, pyrazinyl, pyridazinyl, quinolinyl, isoquinolinyl, triazolyl, tetrahydropyrrolyl. Heteroaryl groups can be unsubstituted or substituted, and the substituents include but are not limited to alkyl, alkoxy, aryl, aralkyl, amino, halogen, hydroxy, cyano, nitro, carbonyl, and hetero-aliphatic ring group.

If a substituent is described as "optionally substituted", the substituent may be (1) unsubstituted or (2) replaced by one or more (eg., 1, 2, 3, 4, or 5, preferably 1) of the list of substituents. Unless specifically defined, groups used in the definitions of compounds herein have meanings well known to those skilled in the art.

The term "pharmaceutically acceptable salts" as used herein includes conventional salts formed with pharmaceutically acceptable inorganic or organic acids, or inorganic or organic bases. Illustrative examples of suitable salts include but are not limited to organic salts derived from amino acids such as glycine and arginine, ammonia, primary amines, secondary amines and tertiary amines, and cyclic amines such as piperidine, morpholine and piperazine, and inorganic salts derived from sodium, calcium, potassium, magnesium, manganese, iron, copper, zinc, aluminum and lithium.

Exemplary acid addition salts include, for example, inorganic acid salts such as hydrochloride, hydrobromide, hydroiodide, sulfate, nitrate, phosphate (eg, phosphate, hydrogen phosphate or dihydrogen phosphate), carbonate, bicarbonate or perchlorate; organic acid salts such as acetate, propionate, butyrate, valerate, caproate, heptanoate, octanoate, cyclopentane propionate, undecanoate, lactate, malate, oxalate, fumarate, tartrate, maleate, citrate, nicotinate, benzoate, salicylate or ascorbate; sulfonates such as mesylate, ethanesulfonate, 2-hydroxyethanesulfonate, benzenesulfonate, p-toluenesulfonate, 2-naphthalenesulfonate, 3-phenylsulfonate or camphorsulfonate; acidic amino acid salts such as aspartate or glutamate.

The present disclosure also includes all pharmaceutically acceptable isotopic compounds that are identical to the compounds of the present disclosure, except that one or more atoms are replaced by atoms that have the same atomic number but an atomic mass or mass number different from the atomic mass or mass number that predominates in nature. Examples of isotopes suitable for inclusion in the compounds of the present disclosure include but are not limited to isotopes of hydrogen (eg., 2H, 3H); isotopes of carbon (eg., 11C, 13C, and 14C); isotopes of chlorine (eg., 36Cl); isotopes of fluorine (eg., 18F); isotopes of iodine (eg. 1231 and 1251); isotopes of nitrogen (eg. 13N and 15N);

isotopes of oxygen (eg. 15O, 17O and 18O); isotopes of phosphorus (eg. 32P); and isotopes of sulfur (eg. 35S).

The term "stereoisomer" as used herein refers to isomers formed due to at least one asymmetric center. In compounds having one or more (eg, 1, 2, 3 or 4) asymmetric centers, it can give rise to racemic mixtures, single enantiomers, diastereomeric mixtures and individual diastereomers. Certain individual molecules may also exist as geometric isomers (cis/trans). Similarly, the compounds of the present disclosure may exist as mixtures of two or more different structural forms in rapid equilibrium (often referred to as tautomers). Representative examples of tautomers include keto-enol tautomers, phenol-ketone tautomers, nitroso-oxime tautomers, imine-enamine tautomers etc.. For example, a nitroso-oxime can exist in solution in equilibrium in the following tautomeric forms:

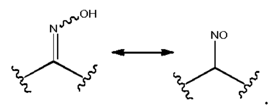

It is to be understood that the scope of this disclosure covers all such isomers or mixtures thereof in any ratio (eg. 60%, 65%, 70%, 75%, 80%, 85%, 90%, 95%, 96%, 97%, 98%, 99%).

Unless otherwise indicated, compounds of the present disclosure are intended to be available as stereoisomers (which include cis and trans isomers, optical isomers (eg, R and S enantiomers), diastereomers, geometric isomers, rotational isomers, conformational isomers, atropisomers and mixtures thereof). The compounds of the present disclosure may exhibit more than one type of isomerism and consist of mixtures thereof (eg., racemic mixtures and pairs of diastereomers).

The compounds of the present disclosure may exist in the form of solvates, preferably hydrates, wherein the compounds of the present disclosure comprise a polar solvent as a structural element of the compound crystal lattice, in particular for example water, methanol or ethanol. The amount of polar solvent, especially water, may be present in stoichiometric or non-stoichiometric ratios.

The term "treatment" as used herein refers to any treatment of a disease in a mammal, including: (1) preventing the disease, i.e. causing symptoms of the clinical disease not to develop; (2) inhibiting the disease, i.e. preventing the development of the clinical symptoms; (3) alleviating the disease, that is, causing the subsidence of clinical symptoms.

The term "cancer" or "tumor" as used herein refers to the growth and proliferation of neoplastic cells, either malignant or benign, and all precancerous cells and cancer cells and tissues. For the compounds, methods, pharmaceutical compositions, pharmaceutical combinations and uses of the present disclosure, the cancer or tumor includes but is not limited to colon cancer, pancreatic cancer, breast cancer, ovarian cancer, prostate cancer, squamous cell carcinoma, basal cell carcinoma, adenocarcinoma, sweat gland cancer, sebaceous gland cancer, lung cancer, leukemia, bladder cancer, stomach cancer, cervical cancer, testicular cancer, skin cancer, rectal cancer, thyroid cancer, kidney cancer, uterine cancer, pemphigus cancer, liver cancer, acoustic neuroma, oligodendroglioma, meningioma, neuroblastoma, eye cancer.

The term "effective dose" as used herein refers to the amount of a compound that, when administered, alleviates to some extent one or more symptoms of the disorder being treated.

The term "individual" as used herein includes human or non-human animals. Exemplary human subjects include human subjects with a disease (eg, a disease described herein) (referred to as patients) or normal subjects. "Non-human animals" in the present disclosure include all vertebrates such as non-mammals (eg. birds, amphibians, reptiles) and mammals such as non-human primates, livestock and/or domesticated animals (eg. sheep, dogs, cats, cows, pigs, etc.).

The "pharmaceutical composition" in the present disclosure refers to a composition comprising one or more compounds of formula (I) or (II) or their stereoisomers, tautomers, stable isotopic derivatives, pharmaceutically acceptable salts or solvates and carriers generally accepted in the art for the delivery of biologically active compounds to organisms such as humans.

The "pharmaceutically acceptable carrier" as used herein refers to a carrier with which the therapeutic agent is administered and which, within the scope of sound medical judgment, is suitable for contact with human and/or other animal tissues without undue toxicity, irritation, allergic reactions, or other problems or complications corresponding to a reasonable benefit/risk ratio, including but not limited to any glidants, sweetening agents, diluents, preservatives, dyes/colorants, flavor enhancers, surfactants, wetting agents, dispersing agents, disintegrating agents, suspending agents, stabilizers, isotonic agents, solvents or emulsifiers, such as including but not limited to calcium carbonate, calcium phosphate, various sugars and various starches, cellulose derivatives, gelatin, vegetable oils and polyethylene glycols.

The pharmaceutical compositions of the present disclosure can be formulated by techniques known to those skilled in the art, such as those disclosed in Remington's Pharmaceutical Sciences 20th Edition, such as conventional mixing, dissolving, granulating, methods for preparing dragees, grinding, emulsification, freeze-drying, etc., and can be formulated into solid, semi-solid, liquid or gaseous preparations, such as tablets, pills, capsules, powders, granules, ointments, emulsions, suspensions, solutions, suppositories, injections, inhalants, gels, microspheres and aerosols,

etc.. These pharmaceutical compositions may contain conventional ingredients in pharmaceutical formulations, such as diluents, carriers, pH adjusters, sweeteners, disintegrants, and the like.

The administration routes of the compounds of the present disclosure or their pharmaceutically acceptable salts or their pharmaceutical compositions include but are not limited to oral, rectal, transmucosal, enteral, or topical, transdermal, inhalation, parenteral, sublingual, intravaginal, intranasal, intraocular, intraperitoneal, intramuscular, subcutaneous, intravenous administration. The preferred route of administration is oral administration.

For oral administration, the pharmaceutical compositions can be formulated by admixing the active compound with pharmaceutically acceptable carriers well known in the art. These carriers enable the compounds of this disclosure to be formulated as tablets, pills, lozenges, dragees, capsules, liquids, gels, slurries, suspensions and the like. For example, for pharmaceutical compositions for oral administration, tablets may be obtained by combining the active ingredient with one or more solid carriers, granulating the resulting mixture if desired, and adding minor amounts of excipients if desired, to process into mixtures or granules to form tablets or tablet cores. The tablet core can be associated with an optional enteric coating material and processed into a coated formulation that is more conducive to absorption by an organism (eg, a human).

Dosage regimens can be adjusted to provide the optimal desired response. For example, a single bolus may be administered, several divided doses may be administered over time, or the dose may be proportionally reduced or increased as indicated by the exigencies of the therapeutic situation. It is noted that dosage amounts may vary with the type and severity of the condition to be alleviated, and may include single or multiple doses. It is further understood that for any particular individual, the specific dosing regimen should be adjusted over time according to the needs of the individual and the professional judgment of the person administering or supervising the administration of the composition.

The compounds of the present disclosure may be administered as the sole active ingredient or, as appropriate, in combination with additional drugs or therapies that may have or produce the same or different pharmacological effects, with the proviso that, when used in combination the compounds of the present disclosure, do not result in undesired reductions in activity, adverse interactions or side effects.

When a compound of formula (I) or (II) is administered in combination with other drugs, the dosage of the other drugs co-administered will of course depend on the type of co-drug used, the specific drug used, the condition to be treated, the general health of the patient, the judgment of the physician or the veterinarian.

The drugs used in combination with the present compounds, i.e., co-agents, may be administered simultaneously, separately or sequentially with the compounds of the present disclosure by the same or different route of administration. They may be contained in the same pharmaceutical composition, or may be in separate form, such as a combination product, preferably in the form of a kit. They may be prepared and/or formulated by the same or different manufacturers. Furthermore, the compound of the present disclosure and the additional drug may be added to the combination therapy (i) prior to delivering the combination product to a physician (eg., in the case of a kit comprising the compound of the present disclosure and the additional drug); (ii) immediately prior to administration by the physician himself (or under the direction of the physician); (iii) by the patient himself, eg. during sequential administration of the compound of the disclosure and the additional drug.

The amount of the compound of the disclosure administered will depend on the individual being treated, severity of the disorder or condition, rate of administration, disposition of the compound, and judgment of the prescribing physician. In general, an effective dose is about 0.0001 to about 5000 mg /kg body weight/day, eg, about 0.01 to about 1000mg/kg/day (single or divided administration). For a 70 kg person, this would add up to about 0.007mg/day to about 7000mg/day, eg. about 0.7mg/day to about 1500mg/day. In some cases, dose levels not higher than the lower limit of the foregoing ranges may be sufficient, while in other cases larger doses may be employed without causing any deleterious side effects, provided that the larger dose is first divided into several smaller doses to be administered throughout the day.

The content or amount of the compound of the present disclosure in the pharmaceutical composition can be about 0.01mg to about 1000mg, suitably 0.1-500mg, preferably 0.5-300mg, more preferably 1-150mg, particularly preferably 1-50mg, such as 1.5mg, 2mg, 4mg, 10mg, 25mg, etc..

## Beneficial Effects of Invention

The present disclosure provides a class of sulfo-substituted biaryl compounds with the characteristic structural formula (I). It has been found through research that such compounds can effectively activate RORγt protein receptor, thereby regulating the differentiation of Th17 cells and increasing the production of IL-17, so can be used as an immunomodulator for the treatment of diseases related to Th17 cell differentiation.

The compounds of the present disclosure have the following beneficial effects:

- High agonistic activity on RORγt receptor, as demonstrated in the Activity Examples 1 and 2 below;
- Regulating the differentiation of Th17 cells, increasing the production of IL-17, as demonstrated in Active Examples

3 and 4 below; and/or

- Good pharmacokinetic properties, such as longer $t_{1/2}$, allowing for example greater dosing intervals, longer half-life, and better patient compliance, as demonstrated in Active Example 5 below;
- Improved $AUC_{0\text{-last}}$ data, better druggability, higher bioavailability, as demonstrated in Active Example 6 below; and/or
- Good safety profile, eg. excellent properties such as membrane permeability, P450 (reduced risk of drug interactions), solubility, etc..

**Embodiments of carrying out the invention**

The technical solutions of the present disclosure are illustrated below with reference to specific embodiments, but the protection scope of the present disclosure is not limited to these embodiments. All modifications or equivalent substitutions that do not depart from the concept of the present disclosure are included in the protection scope of the present disclosure.

The experimental methods without specific conditions in the following examples are generally in accordance with the conventional conditions of this type of reaction, or in accordance with the conditions suggested by manufacturers. Percentages and parts are weight percentages and parts unless otherwise specified. Unless otherwise stated, ratios of liquids are by volume.

Unless otherwise specified, the experimental materials and reagents used in the following examples can be obtained from commercial sources, prepared according to methods in the prior art, or prepared according to methods analogous to those disclosed in this application.

Abbreviations used herein have the meanings commonly understood in the art unless clearly defined otherwise in the specification. The meanings of the abbreviations used in the specification are listed below:

$PdCl_2$ (dtbpf): 1,1'-di-tert-butylphosphinoferrocene palladium dichloride
Pd(dppf)$Cl_2$: [1,1'-bis(diphenylphosphino)ferrocene] palladium dichloride
xantphos: 4,5-Bisdiphenylphosphine-9,9-dimethylxanthene
$pd_2(dba)_3$: Tri(dibenzylideneacetone) dipalladium
HATU: 2-(7-Azobenzotriazole)-*N*,*N*,*N*',*N*',-tetramethylurea hexafluorophosphate
DIEA: *N*,*N*-diisopropylethylamine
DMF: N,N-dimethylformamide
DCM: dichloromethane
EA: Ethyl acetate
PE: Petroleum ether
rt: room temperature
LC-MS: Liquid Chromatography Mass Spectrometry
ESI: Electrospray ionization
*m/z:* mass-to-charge ratio
DTT: Dithiothreitol
EDC: 1-(3-dimethylaminopropyl)-3-ethylcarbodiimide hydrochloride
DCC: Dicyclohexylcarbodiimide
HOBt: 1-Hydroxybenzotriazole
TLC: Thin Layer Chromatography

**Synthesis Examples**

In the preparation of the target compounds provided by the present disclosure, column chromatography was carried out with silica gel (300-400mesh) produced by Rushan Sun Desiccant Co., Ltd.; thin-layer chromatography was carried out with GF254 (0.25mm); nuclear magnetic resonance spectroscopy (NMR) was carried out with Varian-400 nuclear magnetic resonance spectrometer; liquid mass spectrometry (LC/MS) was carried out with Agilent TechnologiESI 6120 liquid mass spectrometer.

In addition, all operations involving easily oxidizable or easily hydrolyzed raw materials are carried out under nitrogen protection. Unless otherwise specified, the raw materials used in the present disclosure are all commercially available which can be used directly without further purification, and the temperatures used in the present disclosure are all in degrees Celsius °C.

Where the structure of the compound of the present disclosure is inconsistent with the name thereof, the

structural formula generally controls, unless it can be determined from the context that the name of the compound is correct.

**Example 1:** *N*-(2,6-Dichloro-4'-(ethylsulfonyl)-[1,1'-biphenyl]-4-yl)-2-(4-(ethylsulfonyl) phenyl) acetamide

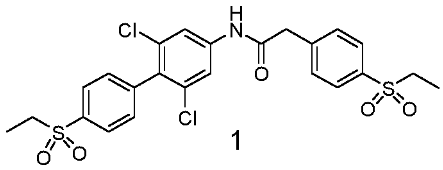

1

The title compound was synthesized according to the following scheme:

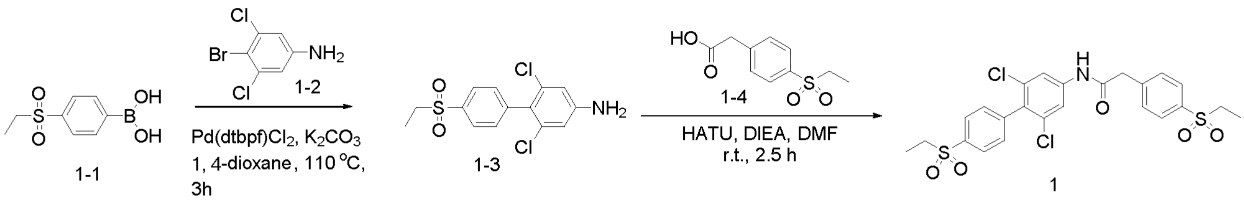


**Step** 1: Synthesis of 2,6-dichloro-4'-(ethylsulfonyl)-[1,1'-biphenyl]-4-amine

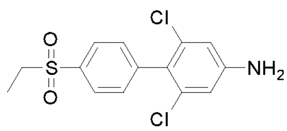

1-3

4-Ethylsulphonylbenzeneboronic acid (514 mg, 2.4 mmol), 4-bromo-3,5-dichloroaniline (482 mg, 2 mmol), $PdCl_2$(dtbpf) (65 mg, 0.1 mmol), potassium carbonate (816 mg, 6 mmol) were added to 1,4-dioxane/water (5mL/1mL). The resulting mixture was stirred at 110°C for 3 hours under nitrogen protection, the solvent was removed under reduced pressure, and the residue was separated and purified by silica gel column (PE:EA = 3:2) to obtain the target compound (320 mg, yield 48.5%, white solid). LC-MS (ESI) *m/z* : 330.0 $[M+H]^+$.

**Step** 2: Synthesis of *N*-(2,6-dichloro-4'-(ethylsulfonyl)-[1,1'-biphenyl]-4-yl)-2-(4-(ethylsulfonyl)phenyl)acetamide

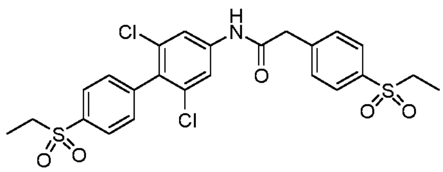

2,6-Dichloro-4'-(ethylsulfonyl)-[1,1'-biphenyl]-4-amine (30 mg, 0.091 mmol), 2-(4-(ethylsulfonyl)phenyl)acetic acid (24.9 mg, 0.109 mmol), HATU (41.4 mg, 0.109 mmol), DIEA (35.3 mg, 0.273 mmol) were added sequentially to DMF (1.5 mL). The reaction mixture was stirred at room temperature for 2.5 hours. Water (10 mL) was added to the reaction mixture, which was extracted with EA (10 mL ×2). The combined organic phases were washed with saturated brine, dried over anhydrous sodium sulfate, and filtered. The filtrate was concentrated under reduced pressure to obtain the crude product. Separation and purification by preparative plate (silica gel, EA:PE = 1.5:1) gave the target compound

(13.3 mg, yield 27.0%, white solid). LC-MS (ESI) *m/z* : 538.17 $[M-H]^-$. $^1H$ NMR (400 MHz, DMSO-$d_6$) $\delta$ 10.71 (s, 1H), 7.99 (d, *J* = 8.4 Hz, 2H), 7.91 - 7.82 (m, 4H), 7.62 (d, *J* = 8.3 Hz, 2H), 7.57 (d, *J* = 8.4 Hz, 2H), 3.87 (s, 2H), 3.38 (q, *J* = 7.4 Hz, 2H), 3.30 - 3.26 (m, 2H), 1.17 - 1.08 (m, 6H).

**Example 2:** *N*-(2,6-dichloro-4'-(methylsulfonyl)-[1,1'-biphenyl]-4-yl)-2-(4-(ethylsulfonyl) phenyl) acetamide

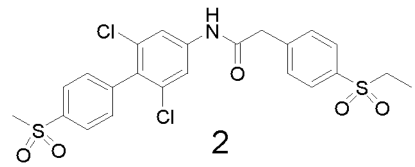

2

The title compound was synthesized according to the following scheme:

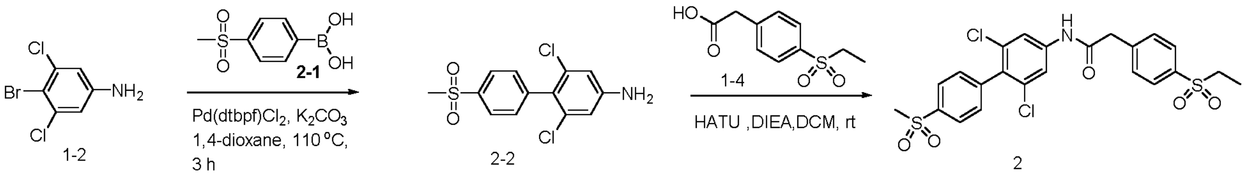


1-2 2-2 2

**Step** 1: Synthesis of 2,6-dichloro-4'-(methylsulfonyl)-[1,1'-biphenyl]-4-amine

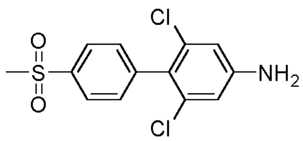

4-Methylsulfonylphenylboronic acid (240 mg, 1.2 mmol), 4-bromo-3,5-dichloroaniline (241 mg, 1 mmol), $PdCl_2$ (dtbpf) (33 mg, 0.05 mmol) and potassium carbonate (408 mg, 3 mmol) were added to a mixture of 1,4-dioxane/water (5 mL/1 mL). The resulting mixture was stirred at 110°C for 3 hours under nitrogen protection. The reaction was completed, and was cooled to room temperature, added with water (20 mL), extracted with ethyl acetate (20 mL $\times$ 3), and the solvent was removed under reduced pressure. The residue was separated and purified on a silica gel column (PE:EA = 3:2) to obain the target compound (168 mg, yield 53.1%, yellow solid). LC-MS (ESI) *m/z:* 317.9$[M+H]^+$.

**Step 2:** Synthesis of *N*-(2,6-dichloro-4'-(methylsulfonyl)-[1,1'-biphenyl]-4-yl)-2-(4-(ethylsulfonyl)phenyl)acetamide

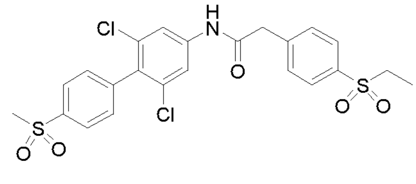

2,6-Dichloro-4'-(methylsulfonyl)-[1,1'-biphenyl]-4-amine (58 mg, 0.18 mmol), HATU (84 mg, 0.22 mmol), 2-(4-(ethylsulfonyl)phenyl)acetic acid (50 mg, 0.22 mmol) and DIEA (70 mg, 0.54 mmol) were successively added to DCM (3mL). The reaction solution was reacted at room temperature overnight. TLC showed that the raw materials had been run out of and the reaction was completed.

The reaction was washed with saturated aqueous sodium bicarbonate solution (20 mL) and saturated sodium chloride (20 mL) successively, and the crude product was separated via an automated chromatographic system using

silica gel column (PE:EA = 1:4), to obtain the target product (70 mg, 71.4 % yield, white solid). LC-MS (ESI) *m/z* : $[M+H]^+/[M+18]^+$ 526.0/543.0.

$^1H$ NMR (400 MHz, DMSO-$d_6$) δ 10.70 (s, 1H), 8.04 (d, *J* = 8.2 Hz, 2H), 7.87 (d, *J* = 8.2 Hz, 2H), 7.85 (s, 2H), 7.62 (d, *J* = 8.2 Hz, 2H), 7.57 (d, *J* = 8.2 Hz, 2H), 3.87 (s, 2H), 3.31 (s, 3H), 3.31 - 3.25 (m, 2H), 1.11 (t, *J* = 7.3 Hz, 3H).

**Example 3:** *N*-(2,6-Dichloro-4'-(isopropylsulfonyl)-[1,1'-biphenyl]-4-yl)-2-(4-(ethylsulfonyl)phenyl) acetamide

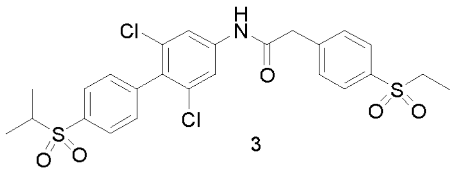

3

The title compound was synthesized according to the following scheme:

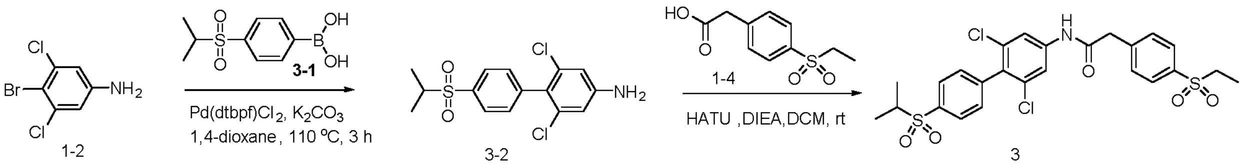


**Step 1:** Synthesis of 2,6-dichloro-4'-(isopropylsulfonyl)-[1,1'-biphenyl]-4-amine

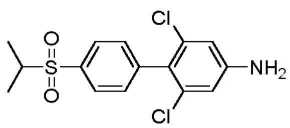

4-Isopropanesulfonylbenzeneboronic acid (252 mg, 1.2 mmol), 4-bromo-3,5-dichloroaniline (241 mg, 1 mmol), $PdCl_2$ (dtbpf) (33 mg, 0.05 mmol) and potassium carbonate (408 mg, 3 mmol) were added to a mixture of 1,4-dioxane/water (5 mL/1 mL). The resulting mixture was stirred at 110°C for 3 hours under nitrogen protection. The reaction was completed, and was cooled to room temperature, added with water (20 mL), and extracted with EA (20 mL × 3). The solvent was removed under reduced pressure, and the residue was separated and purified by silica gel column (PE:EA = 3:2) to obtain the target compound (230 mg, yield 66.8%, yellow solid). LC-MS (ESI) *m/z*: 344.0$[M+H]^+$.

**Step 2:** Synthesis of *N*-(2,6-Dichloro-4'-(isopropylsulfonyl)-[1,1'-biphenyl]-4-yl)-2-(4-(ethylsulfonyl) phenyl)acetamide

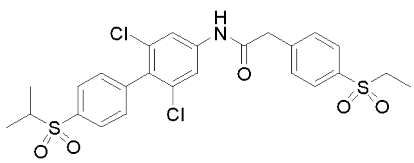

2,6-Dichloro-4'-(isopropylsulfonyl)-[1,1'-biphenyl]-4-amine (62 mg, 0.18 mmol), HATU (84 mg, 0.22 mmol), 2-(4-(ethylsulfonyl)phenyl)acetic acid (50 mg, 0.22 mmol) and DIEA (70 mg, 0.54 mmol) were successively added in DCM (3mL). The reaction solution was reacted at room temperature overnight. TLC showed that the raw materials had been run out of and the reaction was completed. The reaction was washed with saturated aqueous sodium bicarbonate solution (20 mL) and saturated sodium chloride (20 mL) successively, and the crude product was separated by chromatography using silica gel column (PE:EA = 1:2) to obtain the target product (70 mg, 70.0% yield, white solid). LC-MS

(ESI) *m/z*: 571.0[M+18]$^+$.

$^1$H NMR (400 MHz, DMSO-$d_6$) δ 10.70 (s, 1H), 7.96 (d, *J* = 8.2 Hz, 2H), 7.91 - 7.81 (m, 4H), 7.62 (d, *J*= 8.2 Hz, 2H), 7.58 (d, *J*= 8.2 Hz, 2H), 3.87 (s, 2H), 3.55 - 3.46 (m, 1H), 3.31 - 3.25 (m, 2H), 1.19 (d, *J* = 6.8 Hz, 6H), 1.11 (t, *J* = 7.3 Hz, 3H).

**Example 4:** *N*-(2,6-Dichloro-4'-(ethylsulfonyl)-[1,1'-biphenyl]-4-yl)-2-(4-(methylsulfonyl) phenyl)acetamide

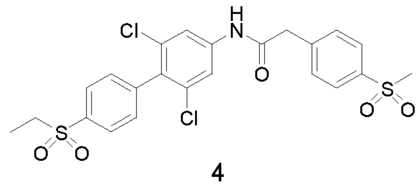

**4**

The title compound was synthesized according to the following scheme:

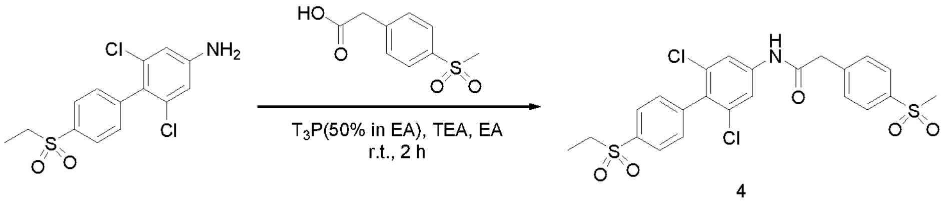


2,6-Dichloro-4'-(ethylsulfonyl)-[1,1'-biphenyl]-4-amine (30 mg, 0.091 mmol), 2-(4-(methylsulfonyl)phenyl)acetic acid (23.4 mg, 0.109 mmol), 1-propylphosphoric anhydride (50% in ethyl acetate, 139 mg, 0.218 mmol), triethylamine (33.2 mg, 0.328 mmol) were successively added to EA (1.5 mL). The reaction mixture was stirred at room temperature for 2 hours. Water (10 mL) and EA (10 mL) were added to the reaction mixture. The aqueous phase was separated and extracted with EA (10 mL). The combined organic phases were washed with saturated brine, dried over anhydrous sodium sulfate, and filtered. The filtrate was concentrated under reduced pressure to obtain the crude product. Separation and purification by preparative plate (silica gel, dichloromethane : methanol = 30:1) to obtain the target compound (30.0 mg, yield 62.6%, white solid). LC-MS (ESI) *m/z*: 543.0 [M+18]$^+$.

$^1$H NMR (400 MHz, DMSO-$d_6$) δ 10.71 (s, 1H), 7.99 (d, *J* = 8.2 Hz, 2H), 7.91 (d, *J* = 8.2 Hz, 2H), 7.85 (s, 2H), 7.61 (d, *J* = 8.2 Hz, 2H), 7.57 (d, *J* = 8.2 Hz, 2H), 3.86 (s, 2H), 3.38 (q, *J* = 14.7, 7.3 Hz, 2H), 3.21 (s, 3H), 1.14 (t, *J* = 7.3 Hz, 3H).

**Example 5:** *N*-(2,6-Dichloro-4'-(ethylsulfonyl)-[1,1'-biphenyl]-4-yl)-2-(5-(ethylsulfonyl)pyridin-2-yl)acetamide

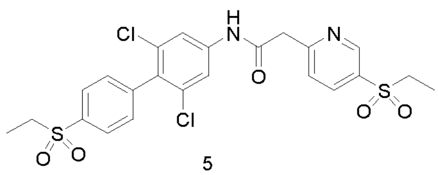

5

The title compound was synthesized according to the following scheme:

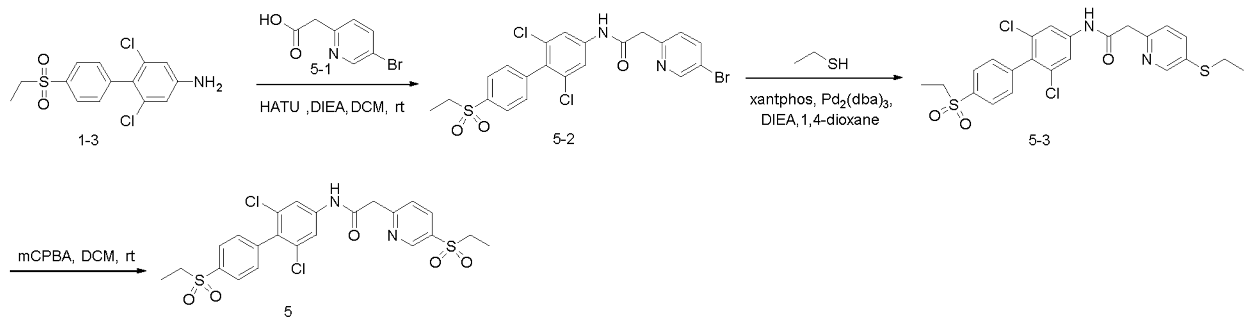


**Step 1:** Synthesis of 2-(5-Bromopyridin-2-yl)-N-(2,6-dichloro-4'-(ethylsulfonyl)-[1,1'-biphenyl]-4-yl)acetamide

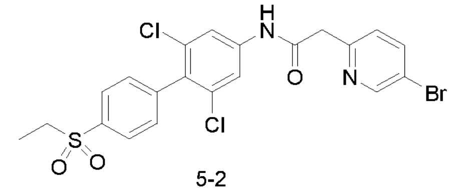

5-2

2,6-Dichloro-4'-(ethylsulfonyl)-[1,1'-biphenyl]-4-amine (120 mg, 0.35 mmol), HATU (160 mg, 0.42 mmol), 2-(5-Bromopyridin-2-yl)acetic acid (91 mg, 0.42 mmol) and DIEA (135 mg, 1.05 mmol) were successively added in DCM (3mL). The reaction solution was reacted at room temperature overnight. TLC showed that the raw materials had been run out of and the reaction was completed. The reaction solution was washed with saturated aqueous sodium bicarbonate solution (20 mL) and saturated sodium chloride (20 mL) successively, and the crude product (200 mg) was directly used in the next step.

**Step 2:** Synthesis of *N*-(2,6-Dichloro-4'-(ethylsulfonyl)-[1,1'-biphenyl]-4-yl)-2-(5-(ethylthio)pyridine-2-yl)acetamide

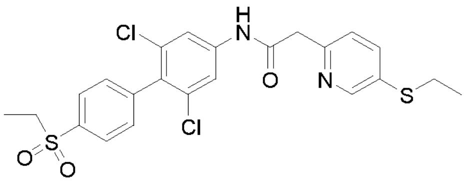

2-(5-Bromopyridin-2-yl)-N-(2,6-dichloro-4'-(ethylsulfonyl)-[1,1'-biphenyl]-4-yl)acetamide crude (200 mg) synthasized in the previous step, DIEA (135 mg, 1.05 mmol), xantphos (9.8 mg, 0.017 mmol), $Pd_2(dba)_3$ (16 mg, 0.017 mmol) and 1,4-dioxane (3 mL) were added to a microwave reaction tube, bubbled with nitrogen for 5 minutes, and then reacted under microwave at 120°C for 2 hours. TLC showed that the reaction was completed, and the reaction was separated via an automated chromatographic system using silica gel column (PE:EA = 1:2) to obtain the target product (130 mg, 70.3% over two steps, yellow solid). LC-MS (ESI) *m/z* : 509.0$[M+H]^+$.

**Step 3:** Synthesis of *N*-(2,6-Dichloro-4'-(ethylsulfonyl)-[1,1'-biphenyl]-4-yl)-2-(5-(ethylsulfonyl)pyridine-2-yl)acetamide

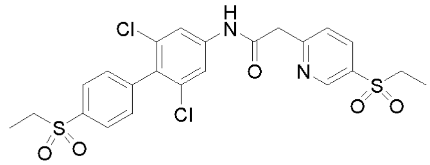

At room temperature, *N*-(2,6-dichloro-4'-(ethylsulfonyl)-[1,1'-biphenyl]-4-yl)-2-(5-(ethylthio)pyridine-2-yl)acetamide (130 mg, 0.27 mmol), m-chloroperoxybenzoic acid (107 mg, 0.53 mmol, 85% content) were added to DCM (5mL), and the reaction solution was reacted at room temperature for 1 hour. The reaction was completed, and was washed once with 2N sodium carbonate solution (10 mL), and separated via an automated chromatographic system using silica gel column (PE:EA = 1:3) to obtain the target product (42 mg, yield 28.8%, yellow solid). LC-MS (ESI) *m/z*: 541.0 $[M+H]^+$.

$^1H$ NMR (400 MHz, DMSO-$d_6$) $\delta$ 10.77 (s, 1H), 8.98 (d, *J*= 2.3 Hz, 1H), 8.29 (dd, *J*= 8.2, 2.4 Hz, 1H), 7.99 (d, *J*= 8.3 Hz, 2H), 7.85 (s, 2H), 7.73 (d, *J*= 8.2 Hz, 1H), 7.58 (d, *J*= 8.3 Hz, 2H), 4.08 (s, 2H), 3.45 -3.35 (m, 4H), 1.17 - 1.12 (m, 6H).

**Example 6:** *N*-(2,6-Dichloro-4'-(cyclopropylsulfonyl)-[1,1'-biphenyl]-4-yl)-2-(4-ethylsulfonyl) phenyl)acetamide

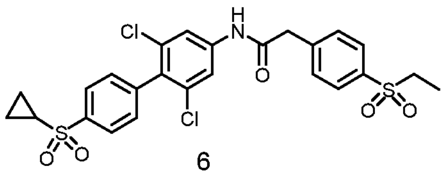

6

The title compound was synthesized according to the following scheme:

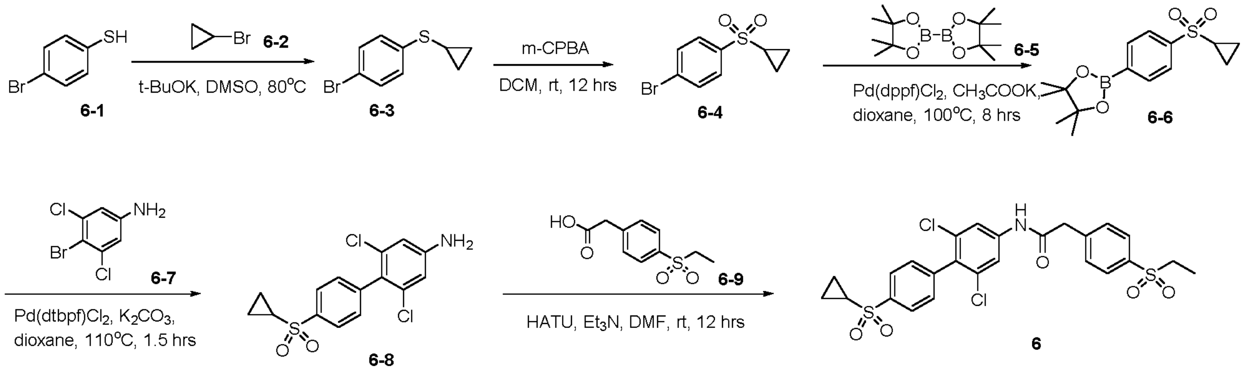


**Step 1:** Synthesis of 1-bromo-4-cyclopropylthiobenzene

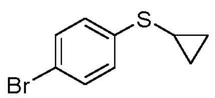

Potassium tert-butoxide (1.48 g, 13.2 mmol) was dissolved in dimethyl sulfoxide (5 mL), and 4-bromobenzenethiol (1.64 mL, 13.2 mmol) was added dropwise to the reaction solution at room temperature. Upon the dropwise addition was completed, the reaction mixture was stirred at room temperature for reaction for 15 minutes, and then bromocyclopropane (3.18 mL, 39.7 mmol) was added to the reaction solution. The tube was sealed and the reaction mixture was stirred at 80°C for reaction for 24 hours. TLC showed that the raw materials was reacted completely, and a new main spot was formed (Rf= 0.7, PE:EA=10:1). The reaction solution was poured into ice water (50 mL) and extracted with methyl tert-butyl ether (50 mL × 2). The organic phases were combined, washed with saturated brine (30 mL), dried over anhydrous sodium sulfate, filtered, and the filtrate was concentrated under reduced pressure to

obtain the target compound (2.8 g, yield 92.4%, brown liquid).

**Step 2:** Synthesis of 1-bromo-4-cyclopropylsulfonylbenzene

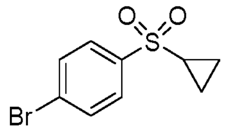

1-Bromo-4-cyclopropylthiobenzene (6-3) (3.00 g, 13.1 mmol) was dissolved in DCM (50 mL) and m-chloroperoxybenzoic acid (5.65 g, 32.7 mmol) was slowly added to the reaction solution under ice bath. After the addition was completed, the ice bath was removed and the reaction mixture was stirred at room temperature overnight. TLC showed that the raw materials were reacted completely, and a new main spot was formed (Rf= 0.5, PE: EA=10:1). The reaction solution was filtered, the filtrate was poured into saturated aqueous sodium bicarbonate solution (50 mL), the organic phase was washed with saturated brine (30 mL), dried over anhydrous sodium sulfate, filtered, and the filtrate was concentrated under reduced pressure to obtain the crude product, which was separated and purified on a silica gel column (PE:EA = 100:1-30:1) to obtain the target compound (1.50 g, 43.9% yield, yellow solid).

**Step 3:** Synthesis of 2-(4-(cyclopropylsulfonyl)phenyl)-4,4,5,5-tetramethyl-1,3,2-dioxaborolane

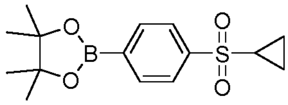

1-Bromo-4-cyclopropylsulfonylbenzene (6-4) (500 mg, 1.91 mmol), Bis(pinacolato)diboron (729 mg, 2.87 mmol), potassium acetate (564 mg, 5.74 mmol)) and Pd(dppf)$Cl_2$ (140 mg, 0.192 mmol) were added to 1,4-dioxane (5 mL). Under nitrogen protection, the reaction mixture was stirred at 90°C overnight. Then the reaction mixture was cooled to room temperature, filtered, and the filtrate was concentrated under reduced pressure to obtain crude product, which was separated and purified by silica gel column (PE:EA = 100:1-30:1) to obtain the target compound (200 mg, yield 3.39%, yellow liquid).

**Step 4:** Synthesis of 2,6-dichloro-4'-(cyclopropylsulfonyl)-[1,1'-biphenyl]-4-amine

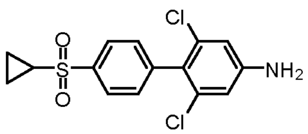

2-(4-(cyclopropylsulfonyl)phenyl)-4,4,5,5-tetramethyl-1,3,2-dioxaborolane (200 mg, 0.649 mmol), 4-bromo-3,5-dichloroaniline (130 mg, 0.541 mmol), potassium carbonate (187 mg, 1.35 mmol) and $PdCl_2$ (dtbpf) (59.1 mg, 0.081 mmol) were added to a mixed solvent of 1,4-dioxane (8 mL) and water (1 mL). Under nitrogen protection, the reaction mixture was stirred at 110°C for 1.5 hours, and monitored by TLC for completion. The reaction mixture was cooled to room temperature, filtered, and the filtrate was concentrated under reduced pressure to obtain crude product, which was separated and purified by silica gel column (PE:EA = 8:1-4:1) to obtain the target compound (64.0 mg, yield 34.6%, brown solid). LC-MS (ESI) *m/z* 342.0 $[M+H]^+$.

**Step 5:** Synthesis of *N*-(2,6-Dichloro-4'-(cyclopropylsulfonyl)-[1,1'-biphenyl]-4-yl)-2-(4-ethylsulfonyl)phenyl)acetamide

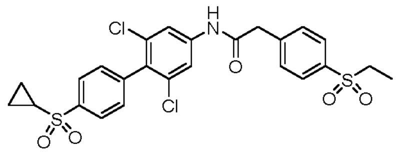

2,6-Dichloro-4'-(cyclopropylsulfonyl)-[1,1'-biphenyl]-4-amine (50.0 mg, 0.146 mmol), 2-[4-(ethylsulfonyl))phenyl]acetic acid (40.0 mg, 0.175 mmol), DIEA (0.0724 mL, 0.438 mmol) and HATU (83.3 mg, 0.219 mmol) were added to DMF (3 mL). The reaction mixture was stirred at room temperature overnight. The reaction solution was poured into water (20 mL) and extracted with EA (10 mL × 2). The organic phases were combined, washed successively with saturated aqueous sodium bicarbonate solution (15 mL) and saturated brine (15 mL), dried over anhydrous sodium sulfate, filtered, and the filtrate was concentrated under reduced pressure to obtain the crude product. Crystallization from methanol gave the target compound (20.0 mg, 24.8% yield, white solid). LC-MS (ESI) *m/z* 550.0 $[M-1]^-$.

$^1$H NMR (400 MHz, $CDCl_3$) δ 7.97 (d, J = 8.3 Hz, 2H), 7.91 (d, J = 8.2 Hz, 2H), 7.67 (s, 2H), 7.54 (d, J = 8.2 Hz, 2H), 7.48 (s, 1H), 7.43 (d, J = 8.3 Hz, 2H), 3.84 (s, 2H), 3.14 (q, J = 7.5 Hz, 2H), 2.59 - 2.50 (m, 1H), 1.44 - 1.37 (m, 2H), 1.30 (t, J = 7.5 Hz, 3H), 1.13 - 1.05 (m, 2H).

**Example 7:** *N-(*2,6-Dichloro-4'-(methylsulfonyl)-[1,1'-biphenyl]-4-yl)-2-(4-(methylsulfonyl) phenyl)acetamide

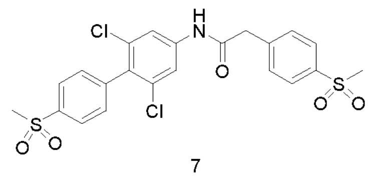

7

2,6-Dichloro-4'-(methylsulfonyl)-[1,1'-biphenyl]-4-amine (64 mg, 0.2 mmol), HATU (91 mg, 0.24 mmol), 2-(4-(methylsulfonyl)phenyl)acetic acid (52 mg, 0.24 mmol) and DIEA (77 mg, 0.6 mmol) were successively added in DCM (3mL), and the reaction solution was reacted at room temperature overnight. TLC showed that the raw materials had been run out of and the reaction was completed. The reaction solution was washed with saturated aqueous sodium bicarbonate solution (20 mL) and saturated sodium chloride (20 mL) successively, and the crude product was separated via aotomated chromatographic system using silica gel column (PE: EA = 1:2) to obtain the target product (90 mg, 86.5% yield, white solid). LC-MS (ESI) *m/z*: 529.0 $[M+18]^+$.

$^1$H NMR (400 MHz, DMSO-$d_6$) δ 10.70 (s, 1H), 8.04 (d, *J* = 8.4 Hz, 2H), 7.91 (d, *J* = 8.4 Hz, 2H), 7.85 (s, 2H), 7.62 (d, *J* = 8.3 Hz, 2H), 7.57 (d, *J* = 8.4 Hz, 2H), 3.86 (s, 2H), 3.32 (s, 3H), 3.22 (s, 3H).

**Example 8:** *N*-(2,6-Dichloro-4'-(methylsulfonyl)-[1,1'-biphenyl]-4-yl)-2-(5-(ethylsulfonyl) pyridin-2-yl)acetamide

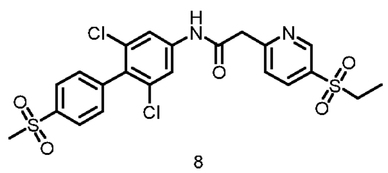

8

The title compound was synthesized according to the following scheme:

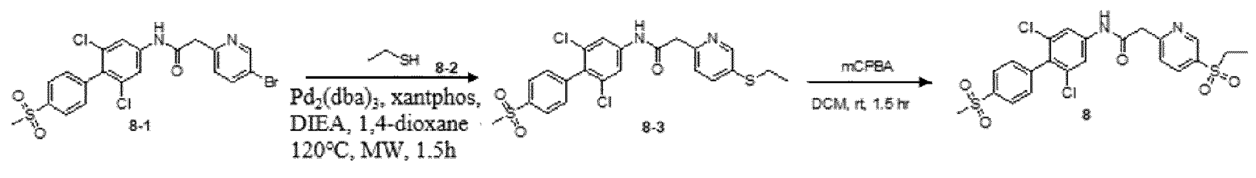

**Step 1:** Synthesis of *N*-(2,6-Dichloro-4'-(methylsulfonyl)-[1,1'-biphenyl]-4-yl)-2-(6-(ethylthio)pyridine-3-yl)acetamide

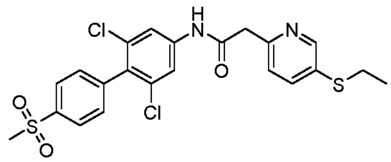

2-(5-Bromopyridin-2-yl)-*N*-(2,6-dichloro-4'-(methylsulfonyl)-[1,1'-biphenyl]-4-yl)acetamide (60.0 mg, 0.117 mmol), $pd_2(dba)_3$ (10.7mg, 0.0117mmol), xantphos (6.77 mg, 0.0117 mmol), triethylamine (0.0324 mL, 0.233 mmol) and ethanethiol (0.0437 mL, 0.584 mmol) were added to 1,4-dioxane (1.5 mL). Under nitrogen protection, the reaction mixture was reacted under microwave at 120°C for 1.5 hours, and monitored by LC-MS for completion. The reaction mixture was cooled to room temperature, filtered, and the filtrate was concentrated under reduced pressure to obtain crude product, which was separated and purified by silica gel column (PE:EA= 4:1-2:1) to obtain the target compound (29.0 mg, yield 50.2%, yellow oil). LC-MS (ESI) *m*/*z* : 495.0 $[M+H]^+$.

**Step 2:** Synthesis of *N*-(2,6-Dichloro-4'-(methylsulfonyl)-[1,1'-biphenyl]-4-yl)-2-(5-(ethylsulfonyl)pyridin-2-yl)acetamide

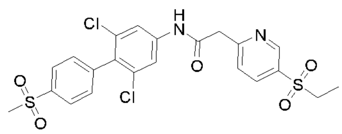

*N-(*2,6-Dichloro-4'-(methylsulfonyl)-[1,1'-biphenyl]-4-yl)-2-(6-(ethylthio)pyridin-3-yl) acetamide (8-3) (29.0 mg, 0.0585 mmol) was dissolved in DCM (2 mL) and m-chloroperoxybenzoic acid (23.8 mg, 0.117 mmol) was slowly added to the reaction under ice bath. Upon the addition was complete, the ice bath was removed, and the reaction was continued under stirring at room temperature for 1.5 hours, and monitored by LC-MS for completion. The reaction solution was poured into saturated aqueous sodium bicarbonate solution (10 mL), extracted with DCM (10 mL×2), the organic phases were combined, dried over anhydrous sodium sulfate, filtered, and the filtrate was concentrated under reduced pressure to obtain a crude product, which was separated and purified by a preparative plate (PE:EA = 1:3) to obtain the target compound (10.0 mg, yield 32.4%, white solid). LC-MS (ESI) *m*/*z* 527.0 $[M+H]^+$.

$^1$H NMR (400 MHz, $CDCl_3$) δ 9.65 (s, 1H), 9.14 (d, *J* = 2.1 Hz, 1H), 8.24 (dd, *J* = 8.1, 2.3 Hz, 1H), 8.02 (d, *J* = 8.2 Hz, 2H), 7.71 (s, 2H), 7.57 (d, *J* = 8.1 Hz, 1H), 7.46 (d, *J* = 8.2 Hz, 2H), 4.03 (s, 2H), 3.20 (q, *J*= 7.4 Hz, 2H), 3.13 (s, 3H), 1.36 (t, *J*= 7.4 Hz, 3H).

**Example** 9: *N*-(2,6-Dichloro-4'-(ethylsulfonyl)-[1,1'-biphenyl]-4-yl)-2-(5-(methylsulfonyl)pyridin-2-yl)acetamide

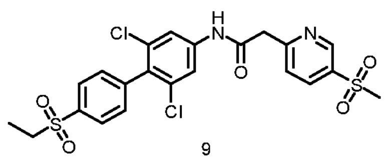

9

The title compound was synthesized according to the following scheme:

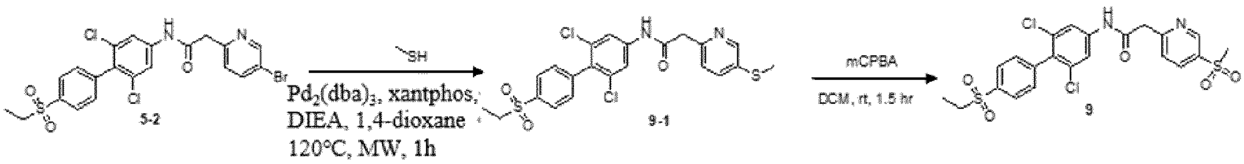


**Step 1:** Synthesis of *N*-(2,6-Dichloro-4'-(ethylsulfonyl)-[1,1'-biphenyl]-4-yl)-2-(5-(methylthio)pyridin-2-yl)acetamide

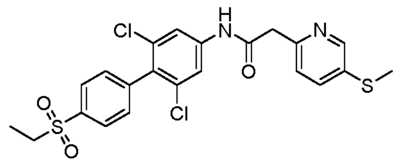

2-(5-Bromopyridin-2-yl)-N-(2,6-dichloro-4'-(ethylsulfonyl)-[1,1'-biphenyl]-4-yl)acetamide (100 mg, 0.189 mmol), methyl mercaptan (18.2 mg, 0.379 mmol), xantphos (10.9 mg, 0.019 mmol), Pd$_2$(dba)$_3$ (10.9 mg, 0.019 mmol) and DIEA (48.9 mg, 0.379 mmol) were dissolved in 1,4-dioxane (4 mL). The reaction mixture was stirred under microwave at 100°C for 1 hour, and monitored by LC-MS for completion. The reaction mixture was cooled to room temperature, filtered, and the filtrate was concentrated under reduced pressure to obtain a crude product, which was separated and purified by a preparative plate (EA:PE=1:1) to obtain the target compound (36.0 mg, yield 38.4%, white solid). LC-MS (ESI) *m*/*z* : 495.0 $[M+H]^+$.

**Step 2:** Synthesis of *N*-(2,6-Dichloro-4'-(ethylsulfonyl)-[1,1'-biphenyl]-4-yl)-2-(5-(methylsulfonyl)pyridin-2-yl)acetamide

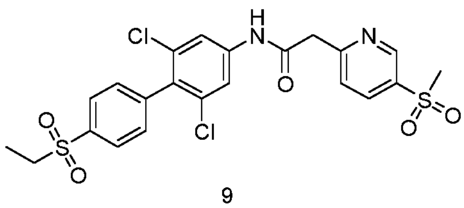

9

*N-(*2,6-Dichloro-4'-(ethylsulfonyl)-[1,1'-biphenyl]-4-yl)-2-(5-(methylthio)pyridin-2-yl)acetamide (9-1) (30.0 mg, 0.06 mmol) was dissolved in DCM (3 mL), and m-chloroperoxybenzoic acid (21.0 mg, 0.121 mmol) was slowly added to the reaction solution under ice bath. The ice bath was removed and the reaction mixture was stirred for an additional 1.5 hours at room temperature, and monitored by LC-MS for completion. The reaction solution was poured into saturated aqueous sodium bicarbonate solution (15 mL), extracted with DCM (15 mL ×2). The combined organic phases were washed with saturated brine (20 mL), dried over anhydrous sodium sulfate, concentrated under reduced pressure to obtain a crude product, which was separated and purified by a preparative plate (EA:PE = 2:1) to obtain the target compound (10.0 mg, yield 31.3%, white solid). LC-MS (ESI) *m*/*z :* 527.0 $[M+H]^+$.

$^1$H NMR (400 MHz, $CDCl_3$) δ 9.60 (s, 1H), 9.19 (s, 1H), 8.28 (d, J = 6.1 Hz, 1H), 7.98 (d, J = 8.1 Hz, 2H), 7.71 (s, 2H), 7.57 (d, J = 8.1 Hz, 1H), 7.45 (d, J = 8.0 Hz, 2H), 4.03 (s, 2H), 3.27 - 3.11 (m, 5H), 1.33 (t, J = 7.4 Hz, 3H).

**Example 10:** *N*-(2,6-Dichloro-4'-(methylsulfonyl)-[1,1'-biphenyl]-4-yl)-2-(5-(methylsulfonyl) pyridin-2-yl)acetamide

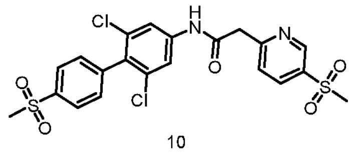

10

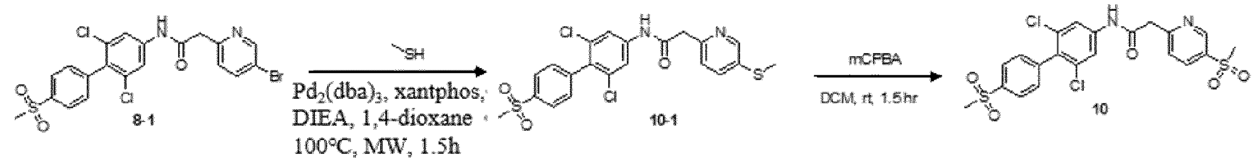


**Step 1:** Synthesis of *N*-(2,6-Dichloro-4'-(methylsulfonyl)-[1,1'-biphenyl]-4-yl)-2-(5-(methylthio) pyridin-2-yl)acetamide

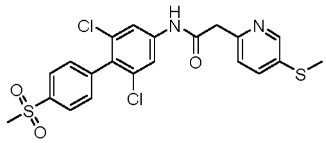

2-(5-Bromopyridin-2-yl)-N-(2,6-dichloro-4'-(methylsulfonyl)-[1,1'-biphenyl]-4-yl)acetamide (60.0 mg, 0.117 mmol), $Pd_2(dba)_3$ (10.7 mg, 0.0117 mmol), xantphos (6.77 mg, 0.0117 mmol), triethylamine (0.0487 mL, 0.350 mmol) and methyl mercaptan (281 mg, 0.584 mmol) were added to 1,4-dioxane (1.5 mL). Under nitrogen protection, the reaction mixture was stirred under microwave at 100°C for 1.5 hours, and monitored by LC-MS for completion. The reaction mixture was cooled to room temperature, filtered, and the filtrate was concentrated under reduced pressure to obtain a crude product, which was separated and purified by silica gel column (PE:EA= 4:1-2:1) to obtain the target compound (30.0 mg, yield 53.4%, yellow oil). LC-MS (ESI) *m/z* : 480.9 $[M+H]^+$.

**Step 2:** Synthesis of *N*-(2,6-Dichloro-4'-(methylsulfonyl)-[1,1'-biphenyl]-4-yl)-2-(5-(methylsulfonyl)pyridin-2-yl)acetamide

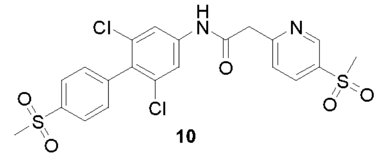

**10**

*N-(*2,6-Dichloro-4'-(methylsulfonyl)-[1,1'-biphenyl]-4-yl)-2-(5-(methylthio)pyridin-2-yl) acetamide (10-1) (30.0 mg, 0.0623 mmol) was dissolved in DCM (2 mL) and m-chloroperoxybenzoic acid (25.3 mg, 0.125 mmol) was slowly added to the reaction solution under ice bath. Upon the addition was complete, the ice bath was removed, and the reaction was continued under stirring at room temperature for 1.5 hours, and monitored by LC-MS for completion. The reaction solution was poured into saturated aqueous sodium bicarbonate solution (10 mL), extracted with DCM (10 mL×2), the organic phases were combined, dried over anhydrous sodium sulfate, filtered, and the filtrate was concentrated under reduced pressure to obtain a crude product, which was separated and purified by a preparative plate (PE:EA = 1:3) to obtain the target compound (3.00 mg, yield 9.38%, white solid). LC-MS (ESI) *m/z* 513.0 $[M+H]^+$.

$^1H$ NMR (400 MHz, $CDCl_3$) $\delta$ 9.61 (s, 1H), 9.19 (d, *J* = 2.0 Hz, 1H), 8.28 (dd, *J* = 8.1, 2.2 Hz, 1H), 8.02 (d, *J* = 8.3 Hz, 2H), 7.71 (s, 2H), 7.58 (d, *J* = 8.1 Hz, 1H), 7.46 (d, *J* = 8.2 Hz, 2H), 4.03 (s, 2H), 3.15 (s, 3H), 3.13 (s, 3H).

**Example 11:** *N*-(2,6-Dichloro-4'-(cyclopropylsulfonyl)-[1,1'-biphenyl]-4-yl)-2-(4-(methylsulfonyl) phenyl)acetamide

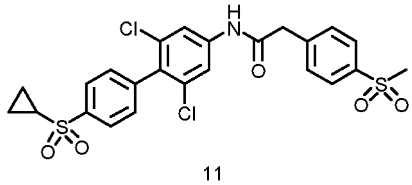

11

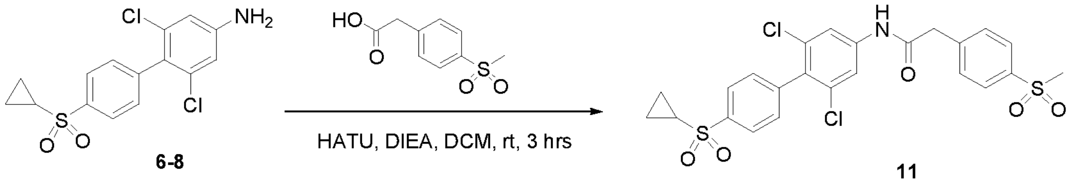


6-8 11

2,6-Dichloro-4'-(cyclopropylsulfonyl)-[1,1'-biphenyl]-4-amine (70.0 mg, 0.205 mmol), 2-[4-(methylsulfonyl)phenyl]acetic acid (52.6 mg, 0.245 mmol), HATU (117 mg, 0.307 mmol) and DIEA (79.3 mg, 0.614 mmol) were added to DCM (3 mL). The reaction mixture was stirred at room temperature for 3 hours. The reaction solution was poured into saturated aqueous ammonium chloride solution (20 mL) and extracted with DCM (15 mL $\times$ 2). The organic phases were combined, washed successively with saturated aqueous sodium bicarbonate solution (20 mL) and saturated brine (20 mL), dried over anhydrous sodium sulfate, filtered, and the filtrate was concentrated under reduced pressure to obtain a crude product, which was separated and purified by a preparative plate (EA:PE = 3:1) to obtain the target compound (14.5 mg, yield 13.2%, white solid). LC-MS (ESI) m/z 536.0 $[M-H]^-$.

$^1H$ NMR (400 MHz, $CDCl_3$) $\delta$ 8.03 (s, 1H), 7.96 (d, J = 8.1 Hz, 2H), 7.90 (d, J = 8.0 Hz, 2H), 7.68 (s, 2H), 7.53 (d, J = 7.9 Hz, 2H), 7.42 (d, J = 8.0 Hz, 2H), 3.82 (s, 2H), 3.07 (s, 3H), 2.61 - 2.49 (m, 1H), 1.43 - 1.37 (m, 2H), 1.17 - 1.06 (m, 2H).

**Example 12:** *N*-(2,6-Dichloro-4'-(ethylsulfonyl)-[1,1'-biphenyl]-4-yl)-2-(4-(ethylsulfonyl)phenyl)-3-hydroxypropionamide

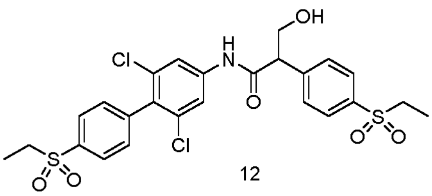

12

*N-(*2,6-Dichloro-4'-(ethylsulfonyl)-[1,1'-biphenyl]-4-yl)-2-(4-(ethylsulfonyl)phenyl)acetamide (450 mg, 0.83 mmol) and paraformaldehyde (25 mg, 0.83 mmol) were added to anhydrous dimethylsulfoxide (5 mL). To the reaction mixture was added sodium methoxide (1.78 mg, 0.03 mmol). The reaction mixture was stirred at room temperature overnight. EA (10 mL) and water (10 mL) were added. The organic phase was separated, the aqueous phase was extracted with EA (10 mL $\times$ 2), the combined organic phases were washed with saturated brine, dried over anhydrous sodium sulfate, and filtered. The filtrate was concentrated under reduced pressure to obtain a crude product, which was separated and purified by column chromatography (silica gel, EA/(DCM/PE = 1/2) = 0 ~ 100%) to obtain the target compound (200 mg, yield 42.4%, white solid). LC-MS (ESI) *m/z* : 586.9 $[M+NH_4]^+$.

$^1H$ NMR (500 MHz, DMSO-$d_6$) $\delta$ 10.76 (s, 1H), 8.06 (d, *J* = 8.4 Hz, 2H), 7.98 - 7.91 (m, 4H), 7.74 (d, *J* = 8.4 Hz, 2H), 7.63 (d, *J* = 8.4 Hz, 2H), 5.22 (t, *J* = 5.2 Hz, 1H), 4.23 - 4.13 (m, 1H), 4.09 - 4.03 (m, 1H), 3.83 - 3.68 (m, 1H), 3.45 (q, *J* = 7.3 Hz, 2H), 3.34 (q, *J* = 7.4 Hz, 2H), 1.21 (t, *J* =7.3 Hz, 3H), 1.17 (t, *J* = 7.3 Hz, 3H).

**Example 12-1:** 3-(2,6- Dichloro-4'-(ethylsulfonyl)-[1,1'-biphenyl]-4-amino)-2-(4-(ethylsulfonyl) phenyl)propionic acid

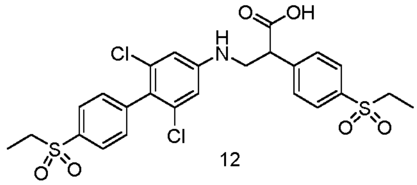

12

Dichloromethane (1 mL), 2,6-dichloro-4'-(ethylsulfonyl)-[1,1'-biphenyl]-4-amine (45 mg, 0.139 mmol), 2-(4-(ethylsulfonyl)phenyl)-3-hydroxypropionic acid (30 mg, 0.116 mmol), EDC (33 mg, 0.174 mmol), HOBt (24 mg, 0.174 mmol) and DIEA (45 mg, 0.348 mmol) were successively added to a 25 mL single-necked flask. The resulting reactant was stirred at room temperature for 48 hours under nitrogen protection, the solvent was then removed under reduced pressure, and the residue was purified by preparative chromatography to obtain the target compound (18.0 mg, yield 27.2%, white solid). LC-MS (ESI) *m/z* : 570.0$[M+H]^+$.

$^1H$ NMR (400 MHz, DMSO-$d_6$) $\delta$ 7.93 (d, *J*= 8.4 Hz, 2H), 7.85 (d, *J*= 8.4 Hz, 2H), 7.65 (d, *J*= 8.4 Hz, 2H), 7.50 (d, *J*= 8.4 Hz, 2H), 6.78 (s, 2H), 6.53 (s, 1H), 3.92 (t, *J*= 6.8 Hz, 1H), 3.78 - 3.73 (m, 1H), 3.44 (dd, *J* = 12.2, 5.4 Hz, 1H), 3.39 - 3.34 (m, 2H), 3.29 - 3.25 (m, 2H), 1.16 - 1.07 (m, 6H).

**Example 13:** <u>*N*-(2,6-Dichloro-4'-(ethylsulfonyl)-[1,1'-biphenyl]-4-yl)-2-(4-(ethylsulfonyl) phenyl)-3-methoxypropionamide</u>

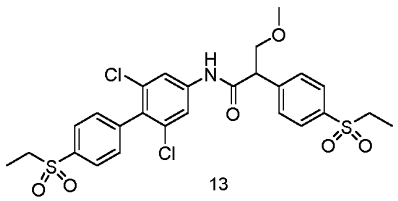

13

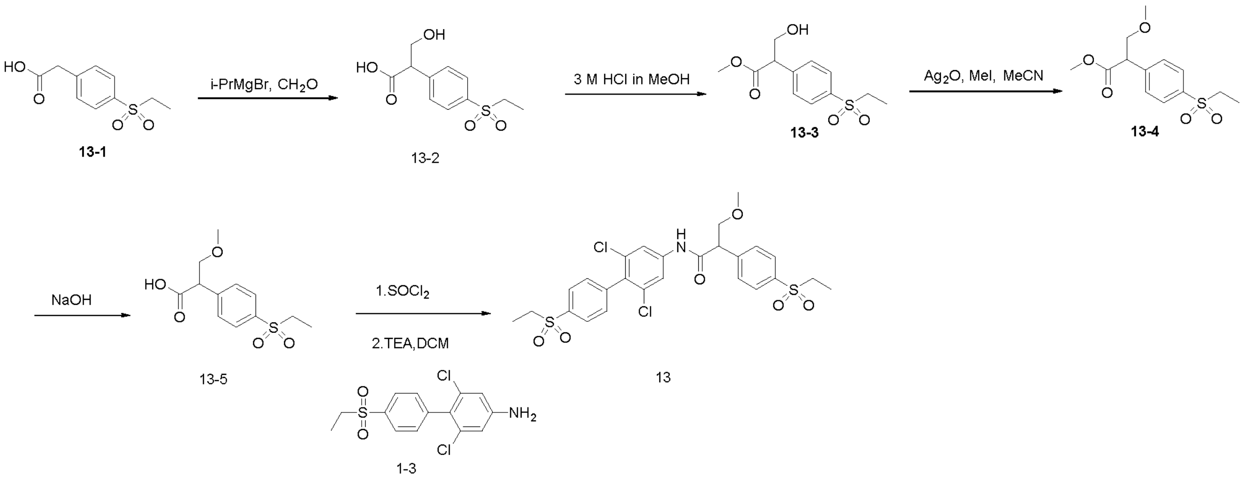


13-1 13-2 13-3 13-4 13-5 13 1-3

**Step 1:** Synthesis of 2-(4-(ethylsulfonyl)phenyl)-3-hydroxypropionic acid

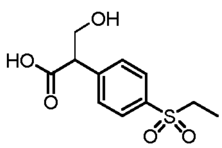

To a 250mL there-necked flask were added 4-ethylsulfonyl phenylacetic acid (2.50 g, 10.9mmol) and tetrahydrofuran (35mL), the reaction solution was cooled to -20°C and then added dropwise isopropylmagnesium bromide (32.8

mL, 32.8 mmol, 1mol/L). Upon the addition was complete, the reaction solution was reacted at room temperature for 6 hours, and then paraformaldehyde (2.95 g, 32.8 mmol) was added. The reaction continued at room temperature for 18 hours, then cooled to 0°C, quenched with saturated aqueous ammonium chloride solution (40mL), and acidified to pH=2~3 with hydrochloric acid (1mol/L) at 0°C. The separated aqueous phase was extracted again with EA (50 mL×2), the organic layers were combined, and the solvent was rotary dried under reduced pressure to obtain a crude product. The crude product was separated with a silica gel column (dichloromethane : methanol=1:0-4:1) to obtain the target product (2.00 g, yield 71.0%, colorless oil). LC-MS (ESI) *m/z* 276.1 $[M+18]^+$.

$^1$H NMR (400 MHz, MeOH-$d_4$) $\delta$ 7.90 - 7.85 (m, 2H), 7.65 - 7.60 (m, 2H), 4.13 - 4.07 (m, 1H), 3.94 - 3.87 (m, 1H), 3.87 - 3.81 (m, 1H), 3.20 (q, *J*= 7.4 Hz, 2H), 1.21 (t, *J*= 7.4 Hz, 3H).

**Step 2:** Synthesis of methyl 2-(4-(ethylsulfonyl)phenyl)-3-hydroxypropanoate

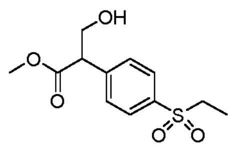

2-(4-(ethylsulfonyl)phenyl)-3-hydroxypropionic acid (1.88 g, 7.27 mmol) and 3mol/L hydrogen chloride methanol solution (60 mL) were added to a 100 mL single-necked flask. The reaction was carried out at 60°C for 18 hours, and rotary dried under reduced pressure to obtain a crude product. The crude product was subjected to silica gel column (EA:PE = 0:1-1:1) to obtain the target product (1.30 g, yield 65.6%, white solid). LC-MS (ESI) m/z: 290.1 $[M+18]^+$.

**Step 3:** Methyl 2-(4-(ethylsulfonyl)phenyl)-3-methoxypropanoate

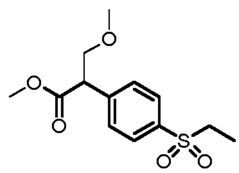

To a 50mL single-necked flask were successively added methyl 2-(4-(ethylsulfonyl)phenyl)-3-hydroxypropionate (1.10 g, 4.03 mmol), acetonitrile (10mL), silver oxide (1.21 g, 5.25 mmol), then cooled to 0°C. Methyl iodide (1.72 g, 12.1 mmol) was added, and the reaction solution was stirred at room temperature for 7 days. The solvent was removed under reduced pressure by rotary evaporation, and the crude product was separated by silica gel column (EA:PE = 0:1-2:1) to obtain the target product (800 mg, yield 69.3%, oil). LC-MS (ESI) m/z: 287.0 $[M+H]^+$.

**Step 4:** 2-(4-(Ethylsulfonyl)phenyl)-3-methoxypropionic acid

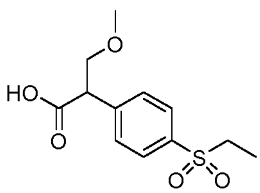

To a 50 mL single-necked flask were added methyl 2-(4-(ethylsulfonyl)phenyl)-3-methoxypropionate (800 mg, 2.79 mmol), methanol (5 mL), water (5 mL), lithium hydroxide (200 mg, 8.38 mmol), and the reaction solution was stirred at 50°C for 2 hours. The solvent was removed under reduced pressure by rotary evaporation, and the crude product was re-diluted with EA (20 mL) and water (20 mL). The separated aqueous phase was acidified with hydrochloric acid

(1 mol/L) to pH=3, and then extracted with EA (20 mL×3). The organic phases from the last three extractions were combined, and the filtrate was rotary dried under reduced pressure to obtain a crude product. The crude product was prepared by high pressure liquid chromatography to obtain the target product (270 mg, yield 35.5%, white solid). LC-MS (ESI) m/z: 273.0 $[M+H]^+$.

$^1H$ NMR (400 MHz, MeOH-$d_4$) δ 7.90 - 7.84 (m, 2H), 7.65 - 7.60 (m, 2H), 4.04 - 3.98 (m, 1H), 3.97 - 3.92 (m, 1H), 3.73 - 3.68 (m, 1H), 3.34 (s, 3H), 3.20 (q, *J* = 7.6 Hz, 2H), 1.21 (t, *J* = 7.6 Hz, 3H). **Step 5:** *N*-(2,6-Dichloro-4'-(ethylsulfonyl)-[1,1'-biphenyl]-4-yl)-2-(4-(ethylsulfonyl)phenyl)-3-methoxypropionamide

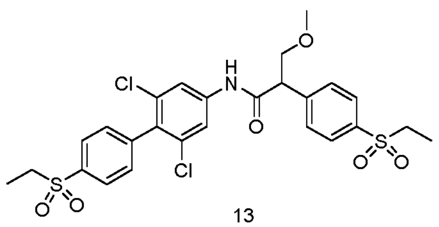

13

To a 50 mL single-necked flask were added 2-(4-(ethylsulfonyl)phenyl)-3-methoxypropionic acid (12-5) (40 mg, 0.146 mmol), DCM (3mL) and thionyl chloride (87 mg, 0.734 mmol), the reaction solution was stirred at room temperature for 3 hours. The solvent was removed under reduced pressure by rotary evaporation, and the crude product was re-dissolved in DCM (3mL), added with DIEA (113 mg, 0.881 mmol), 3,5-dichloro-4-(4-ethylsulfonylphenyl)aniline (58 mg, 0.176 mmol). The reaction solution was further stirred at room temperature for 4 hours. The crude product was obtained by rotary evaporation under reduced pressure, which was subject to high pressure liquid chramatography to obtain the target product (40 mg, yield 46.7%, white solid). LC-MS (ESI) m/z: 584.0 $[M+H]^+$.

$^1H$ NMR (400 MHz, DMSO-$d_6$) δ 10.73 (s, 1H), 7.99 (d, *J* = 8.4 Hz, 2H), 7.89 (d, *J* = 8.4 Hz, 2H), 7.84 (s, 2H), 7.68 (d, *J*= 8.4 Hz, 2H), 7.56 (d, *J*= 8.4 Hz, 2H), 4.17 - 4.11 (m, 1H), 4.00 (t, *J*= 9.0 Hz, 1H), 3.66 - 3.60 (m, 1H), 3.38 (q, *J* = 7.4 Hz, 2H), 3.31 - 3.26 (m, 5H), 1.18 - 1.06 (m, 6H).

**Example 14:** *N*-(2,6-Dichloro-4'-(methylsulfonyl)-[1,1'-biphenyl]-4-yl)-2-(4-(2-hydroxyethyl sulfonyl)phenyl)acetamide

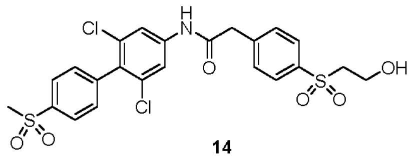

14

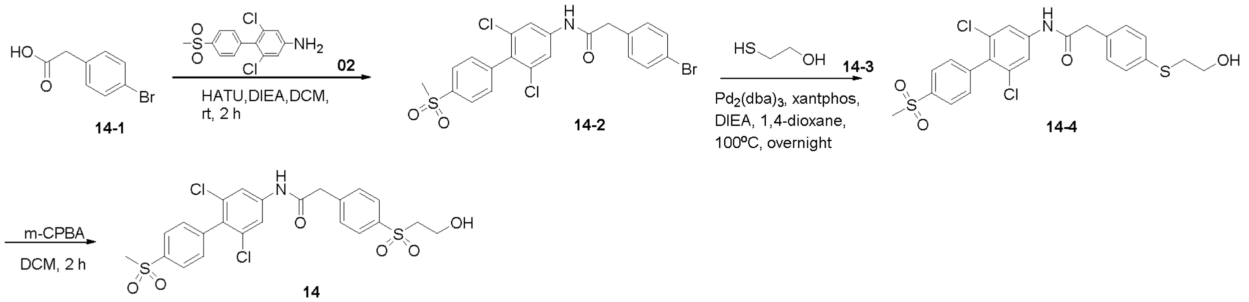


**Step 1:** Synthesis of 2-(4-Bromophenyl)-N-(2,6-dichloro-4'-(methylsulfonyl)-[1,1'-biphenyl]-4-yl)acetamide

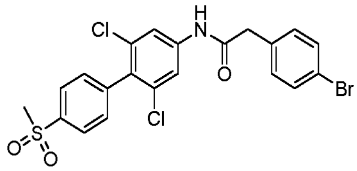

2,6-Dichloro-4'-(methylsulfonyl)-[1,1'-biphenyl]-4-amine (100 mg, 0.316 mmol), 2-(4-bromophenyl)acetic acid (81.6 mg, 0.379 mmol), DIEA (0.157 mL, 0.949 mmol) and HATU (180 mg, 0.474 mmol) were successively added to DCM (3 mL). The reaction mixture was stirred at room temperature for 2 hours and monitored by LC-MS for completion. The reaction solution was poured into water (20 mL) and extracted with DCM (15 mL × 2). The organic phases were combined, washed successively with saturated ammonium chloride (20 mL) and saturated aqueous sodium bicarbonate solution (20 mL), dried over anhydrous sodium sulfate, filtered, and the filtrate was concentrated under reduced pressure to obtain the target compound (196 mg, crude product, white solid). LC-MS (ESI) *m/z* : 509.9 $[M-H]^-$.

**Step 2:** Synthesis of *N*-(2,6-Dichloro-4'-(methylsulfonyl)-[1,1'-biphenyl]-4-yl)-2-(4-(2-hydroxyethylthio)phenyl)acetamide

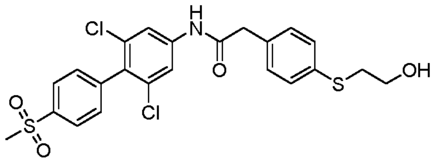

2-(4-Bromophenyl)-N-(2,6-dichloro-4'-(methylsulfonyl)-[1,1'-biphenyl]-4-yl)acetamide (196 mg, 0.382 mmol), $Pd_2(dba)_3$ (35.0 mg, 0.0382 mmol), xantphos (22.1 mg, 0.0382 mmol), triethylamine (0.159 mL, 1.15 mmol) and 2-mercaptoethanol (0.0538 mL, 0.764 mmol) were added into 1,4-dioxane (3 mL). Under nitrogen protection, the reaction mixture was stirred at 100°C overnight, and monitored by TLC for completion. The reaction mixture was cooled to room temperature, filtered, and the filtrate was concentrated under reduced pressure to obtain the target compound (600 mg, crude, yellow oil).

**Step 3:** Synthesis of *N*-(2,6-Dichloro-4'-(methylsulfonyl)-[1,1'-biphenyl]-4-yl)-2-(4-(2-hydroxyethylsulfonyl)phenyl)acetamide

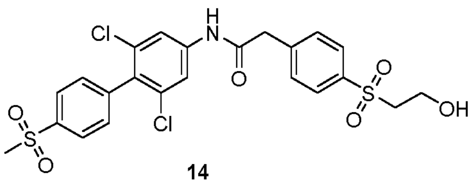

**14**

*N-(*2,6-Dichloro-4'-(methylsulfonyl)-[1,1'-biphenyl]-4-yl)-2-(4-(2-hydroxyethylthio)phenyl) acetamide (600 mg, 1.18 mmol) was dissolved in DCM (10 mL), and m-chloroperoxybenzoic acid (507 mg, 2.94 mmol) was slowly added to the reaction under ice bath. Upon the addition was complete, the ice bath was removed, and the reaction was continued under stirring at room temperature for 2 hours, and monitored by LC-MS for completion. The reaction solution was poured into saturated aqueous sodium bicarbonate solution (30 mL), extracted with DCM (20 mL×2), the organic phases were combined, dried over anhydrous sodium sulfate, filtered, and the filtrate was concentrated under reduced pressure to obtain a crude product, which was separated and purified on silica gel column (PE:EA=2:1-1:2) to obtain the target compound (30.0 mg, 17.5% yield over three steps, white solid). LC-MS (ESI) *m/z* : 541.9 $[M + H]^+$.

$^1H$ NMR (400 MHz, $CDCl_3$) δ 8.02 (d, *J*= 8.0 Hz, 2H), 7.94 (d, *J*= 7.9 Hz, 2H), 7.66 (s, 2H), 7.60 - 7.53 (m, 3H), 7.45 (d, *J*= 7.9 Hz, 2H), 4.06 - 4.00 (m, 2H), 3.84 (s, 2H), 3.42 - 3.33 (m, 2H), 3.14 (s, 3H).

**Example 15:** *N*-(2,6-Dichloro-4'-(methylsulfonyl)-[1,1'-biphenyl]-4-yl)-2-(4-(ethylsulfonyl) phenyl)-3-methoxypropionamide

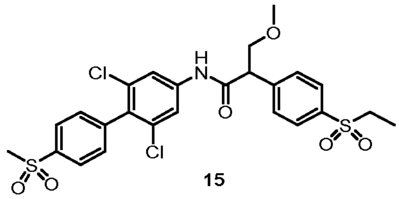

**15**

2-[4-(Ethylsulfonyl)phenyl]-3-methoxypropionic acid (40.0 mg, 0.147 mmol) was dissolved in dry DCM (3 mL), and thionyl chloride (0.0533 mL, 0.735 mmol) was added dropwise to the reaction solution at room temperature. Upon the addition was complete, the reaction was continued under stirring at room temperature for 3 hours. The intermediate obtained by concentrating the reaction solution under reduced pressure was dissolved in dry DCM (3 mL), and 2,6-dichloro-4'-(methylsulfonyl)-[1,1'-biphenyl]-4-amine (46.5 mg, 0.147 mmol) and DIEA (0.159 mL, 0.955 mmol) were added successively. The reaction mixture was stirred at room temperature for 2 hours. The reaction solution was poured into water (20 mL) and extracted with DCM (15 mL × 2). The organic phases were combined, washed successively with saturated ammonium chloride (20 mL) and saturated aqueous sodium bicarbonate solution (20 mL), dried over anhydrous sodium sulfate, filtered, and the filtrate was concentrated under reduced pressure to obtain a crude product, which was separated and purified by silica gel column (PE:EA=1:9) to obtain the target compound (20.0 mg, yield 23.9%, white solid). LC-MS (ESI) *m/z* : 569.9 $[M + H]^+$.

$^1H$ NMR (400 MHz, $CDCl_3$) δ 8.47 (s, 1H), 8.02 (d, *J* = 8.1 Hz, 2H), 7.90 (d, *J* = 8.1 Hz, 2H), 7.70 (s, 2H), 7.58 (d, *J* = 7.9 Hz, 2H), 7.45 (d, *J* = 8.1 Hz, 2H), 4.07 - 3.99 (m, 1H), 3.98 - 3.92 (m, 1H), 3.82 - 3.74 (m, 1H), 3.49 (s, 3H), 3.19 - 3.07 (m, 5H), 1.30 (t, *J* = 7.4 Hz, 3H).

**Example 16:** *N*-(2,6-Dichloro-4'-(cyclopropylsulfonyl)-[1,1'-biphenyl]-4-yl)-2-(4-(ethylsulfonyl)phenyl)-3-methoxypropionamide

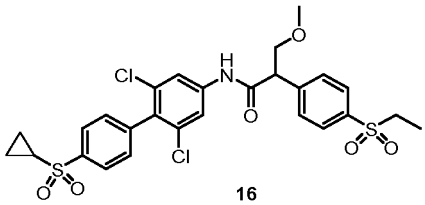

**16**

2-[4-(Ethylsulfonyl)phenyl]-3-methoxypropionic acid (40.0 mg, 0.147 mmol) was dissolved in dry DCM (3 mL), and thionyl chloride (0.0533 mL, 0.735 mmol) was added dropwise to the reaction solution at room temperature. Upon the addition was complete, the reaction was continued under stirring at room temperature for 3 hours. The intermediate obtained by concentrating the reaction solution under reduced pressure was dissolved in dry DCM (3 mL), and 2,6-dichloro-4'-(cyclopropylsulfonyl)-[1,1'-biphenyl]-4-amine (50.3 mg, 0.147 mmol) and DIEA (0.158 mL, 0.955 mmol) were added successively. The reaction mixture was stirred at room temperature for 2 hours. The reaction solution was poured into water (20 mL) and extracted with DCM (15 mL × 2). The organic phases were combined, washed successively with saturated ammonium chloride (20 mL) and saturated aqueous sodium bicarbonate solution (20 mL), dried over anhydrous sodium sulfate, filtered, and the filtrate was concentrated under reduced pressure to obtain a crude product, which was separated and purified by silica gel column (PE:EA=1:9) to obtain the target compound (27.0 mg, yield 30.8%, white solid). LC-MS (ESI) *m/z* : 596.1 $[M + H]^+$.

$^1H$ NMR (400 MHz, DMSO-$d_6$) δ 10.72 (s, 1H), 7.98 (d, *J* = 8.1 Hz, 2H), 7.87 (d, *J* = 8.0 Hz, 2H), 7.82 (s, 2H), 7.66 (d, *J* = 8.0 Hz, 2H), 7.53 (d, *J* = 8.1 Hz, 2H), 4.15 - 4.08 (m, 1H), 3.98 (t, *J* = 9.0 Hz, 1H), 3.65 - 3.57 (m, 1H), 3.29 - 3.25 (m, 5H), 2.98 - 2.89 (m, 1H), 1.18 - 1.13 (m, 2H), 1.11 - 1.04 (m, 5H).

**Example 17:** 2-(4-((Cyclopropylmethyl)sulfonyl)phenyl)-N-(2,6-dichloro-4'-(methylsulfonyl)-[1,1'-biphenyl]-4-yl)acetamide

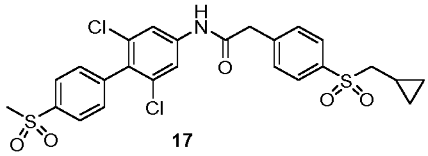

17

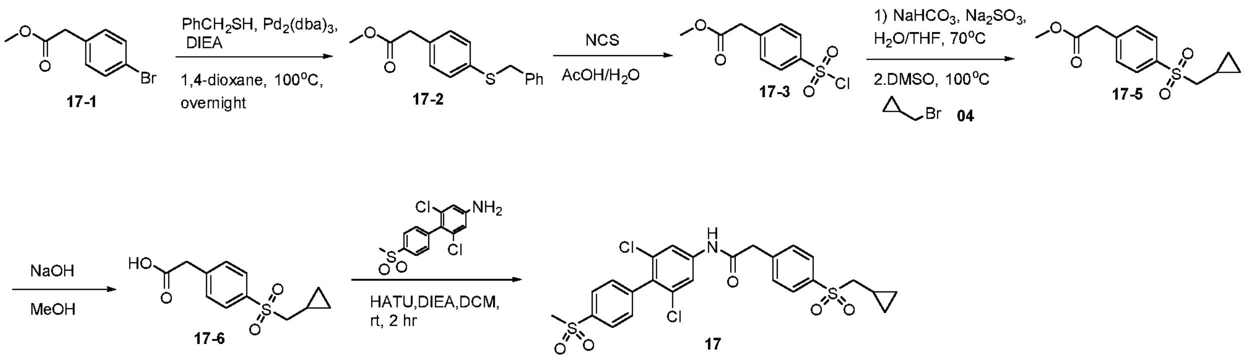


**Step 1**: Synthesis of methyl 2-(4-(benzylthio)phenyl)acetate

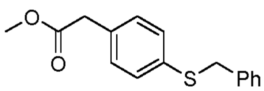

Methyl 2-(4-bromophenyl)acetate (9.00 g, 39.3 mmol), pd$_2$(dba)$_3$ (2.88 g, 3.14 mmol), xantphos (1.82 g, 3.14 mmol), DIEA (13.0 mL, 78.6 mmol) and benzyl mercaptan (9.22 mL, 78.6 mmol) were added to 1,4-dioxane (120 mL). Under nitrogen protection, the reaction mixture was stirred at 100°C overnight, and monitored by TLC for completion. The reaction mixture was cooled to room temperature, filtered, and the filtrate was concentrated under reduced pressure to obtain a crude product, which was separated and purified by silica gel column (PE:EA = 20:1-10:1) to obtain the target compound (10.2 g, yield 95.3%, yellow oily).

**Step 2**: Synthesis of methyl 2-[4-(chlorosulfonyl)phenyl]acetate

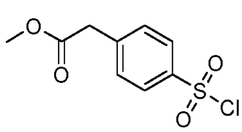

Methyl 2-(4-(benzylthio)phenyl)acetate (10.2 g, 37.4 mmol) was dissolved in a mixed solvent of acetic acid (100 mL) and water (25 mL). Under an ice bath, *N*-chlorosuccinimide (20.0 g, 150 mmol) was slowly added to the reaction solution. After the addition was complete, the ice bath was removed, and the reaction was continued under stirring at room temperature for 2 hours, and monitored by TLC for completion. The reaction solution was poured into water (200 mL), extracted with EA (50 mL×2), the organic phases were combined, dried with anhydrous sodium sulfate, filtered, and the filtrate was concentrated under reduced pressure to obtain a crude product, which was separated and purified by silica gel column (PE:EA = 10:1 - 5:1) to obtain the target compound (8.5 g, yield 91.3%, colorless oil).

**Step 3**: Synthesis of methyl 2-(4-cyclopropylmethylsulfonylphenyl)acetate

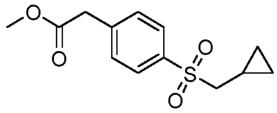

Methyl 2-[4-(chlorosulfonyl)phenyl]acetate (5.00 g, 20.1 mmol) was dissolved in a mixed solvent of tetrahydrofuran (60 mL) and water (40 mL). Sodium bicarbonate (3.38 g, 40.2 mmol) and sodium sulfite (3.04 g, 24.1 mmol) were added to the reaction at room temperature. Upon the addition was complete, the reaction solution was heated to 70°C and stirred for 2 hours, and monitored by LC-MS for completion. The reaction solution was concentrated under reduced pressure to obtain the intermediate, which was dissolved in anhydrous dimethylsulfoxide (90mL), and added with (bromomethyl)cyclopropane (5.90 mL, 60.3 mmol). Under nitrogen protection, the reaction mixture was stirred at 100°C for 3 hours, and monitored by TLC for completion. The reaction solution was poured into water (150 mL), extracted with EA (80 mL×2), the organic phases were combined, washed with saturated brine (100 mL), dried over anhydrous sodium sulfate, filtered, and the filtrate was concentrated under reduced pressure to obtain a crude product, which was separated and purified by silica gel column (PE:EA = 7:3) to obtain the target compound (2.80 g, yield 51.9%, light brown solid).

**Step 4**: Synthesis of 2-(4-cyclopropylmethylsulfonylphenyl)acetic acid

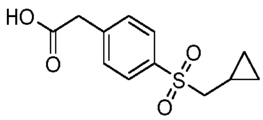

Methyl 2-(4-cyclopropylmethylsulfonylphenyl)acetate (58.0 mg, 0.216 mmol) was dissolved in methanol (3 mL). At room temperature, a 1M aqueous sodium hydroxide solution (1 mL) was added dropwise to the reaction solution. Upon the addition was complete, the reaction was continued under stirring at room temperature for 2 hours, and monitored by LC-MS for completion. The reaction solution was poured into water (10 mL) and adjusted to pH~4 with 1M dilute hydrochloric acid. The reaction was extracted with EA (15 mL×2), the organic phases were combined, dried over anhydrous sodium sulfate, filtered, and the filtrate was concentrated under reduced pressure to obtain the target compound (45.0 mg, crude product, colorless oil). LC-MS (ESI) *m/z* : 507.1 $[2M-H]^-$.

**Step 5**: Synthesis of 2-(4-((Cyclopropylmethyl)sulfonyl)phenyl)-N-(2,6-dichloro-4'-(methylsulfonyl)-[1,1'-biphenyl]-4-yl)acetamide

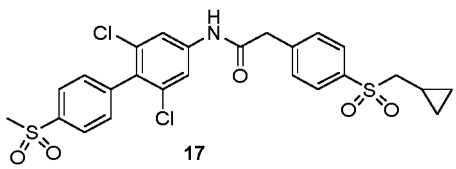

**17**

2,6-Dichloro-4'-(methylsulfonyl)-[1,1'-biphenyl]-4-amine (56.0 mg, 0.177 mmol), 2-(4-cyclopropylmethylsulfonylphenyl)acetic acid (45.0 mg, 0.177 mmol), DIEA (0.0878 mL, 0.531 mmol) and HATU (101 mg, 0.266 mmol) were successively added to DCM (3 mL). The reaction mixture was stirred at room temperature for 2 hours and monitored by LC-MS for completion. The reaction solution was poured into water (20 mL) and extracted with DCM (15 mL × 2). The organic phases were combined, washed successively with saturated ammonium chloride (20 mL) and saturated aqueous sodium bicarbonate solution (20 mL), dried over anhydrous sodium sulfate, filtered, and the filtrate was concentrated under reduced pressure to obtain a crude product, which was separated and purified by reverse phase column (20% water/80% acetonitrile) to obtain the target compound (35.0 mg, 29.4% over two steps, white solid). LC-MS (ESI) *m/z* : 550.0 $[M-H]^-$.

$^1$H NMR (400 MHz, DMSO-$d_6$) δ 10.69 (s, 1H), 8.02 (d, J = 8.1 Hz, 2H), 7.85 (d, J = 8.1 Hz, 2H), 7.82 (s, 2H), 7.59 (d, J = 8.0 Hz, 2H), 7.55 (d, J = 8.1 Hz, 2H), 3.85 (s, 2H), 3.29 (s, 3H), 3.23 (d, J = 7.0 Hz, 2H), 0.86 - 0.76 (m, 1H), 0.48 - 0.39 (m, 2H), 0.14 - 0.06 (m, 2H).

**Example 18:** 2-(4-((Cyclopropylmethyl)sulfonyl)phenyl)-N-(2,6-dichloro-4'-(methylsulfonyl)-[1,1'-biphenyl]-4-yl)-3-methoxypropionamide

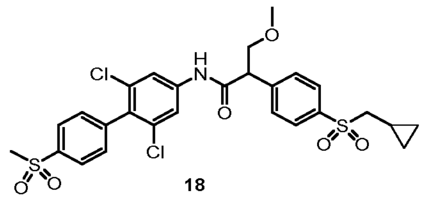

18

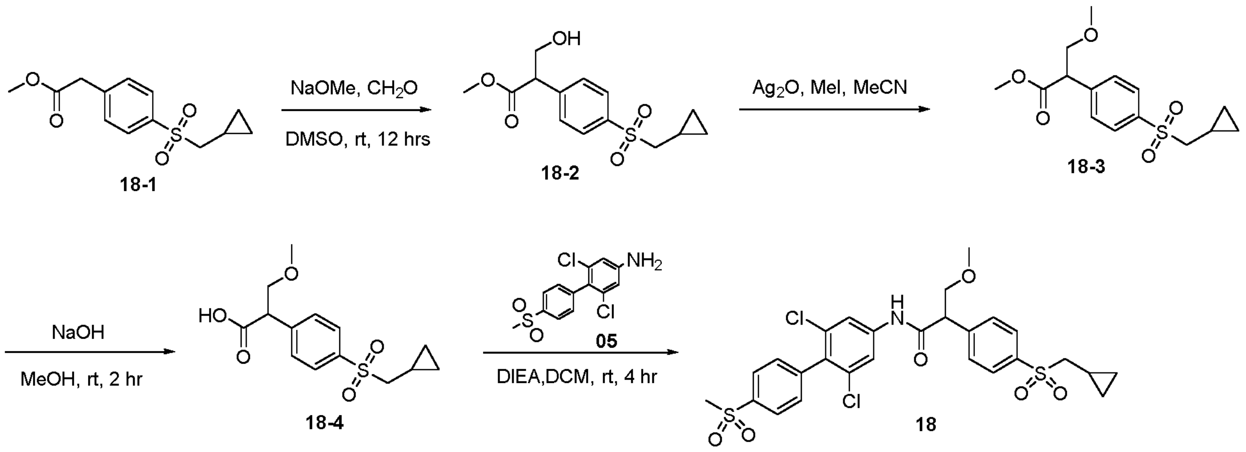


**Step 1:** Synthesis of methyl 2-(4-((cyclopropylmethyl)sulfonyl)phenyl)-3-hydroxypropanoate

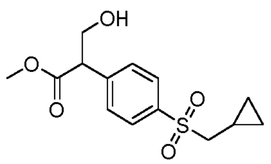

Methyl 2-(4-((cyclopropylmethyl)sulfonyl)phenyl)acetate (1000 mg, 3.73 mmol) and paraformaldehyde (302 mg, 3.35 mmol) were dissolved in anhydrous dimethylsulfoxide (10 mL). Sodium methoxide (8.05 mg, 0.149 mmol) was added to the reaction solution at room temperature. Upon the addition was complete, the reaction solution was stirred at room temperature overnight. The reaction solution was poured into ice water (25 mL), and neutralized with 1M dilute hydrochloric acid to pH~7. The resultant mixture was extracted with EA (15 mL×3), the combined organic phases was washed with saturated brine (30 mL), dried over anhydrous sodium sulfate, filtered, and the filtrate was concentrated under reduced pressure to obtain a crude product, which was separated and purified by silica gel column (PE:EA = 10:1-1:1) to obtain the target compound (765 mg, yield 65.7%, light brown solid), and the raw material (196 mg) was recovered. LC-MS (ESI) *m/z* : 297.1 [M-H]$^-$.

**Step 2**: Synthesis of methyl 2-(4-((cyclopropylmethyl)sulfonyl)phenyl)-3-methoxypropanoate

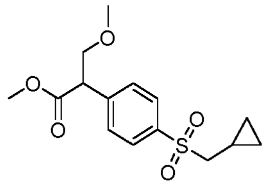

Methyl 2-(4-((cyclopropylmethyl)sulfonyl)phenyl)-3-hydroxypropanoate (490 mg, 1.64 mmol) and silver oxide (457 mg, 1.97 mmol) were dissllved in acetonitrile (4 mL) under a condition protected from light. Under an ice bath, iodomethane (0.153 mL, 2.46 mmol) was added dropwise to the reaction solution. Upon the addition was complete, the ice bath was removed and the reaction was stirred at room temperature for 3 days. The reaction solution was filtered, and the filtrate was concentrated under reduced pressure to obtain a crude product, which wad separated and purified by silica gel column (PE:EA=10:1-1:1) to obtain the target compound (70.0 mg, yield 13.6%, pale yellow solid), and the raw material (255 mg) was recovered. LC-MS (ESI) m/z : 313.1 $[M+H]^+$.

**Step 3:** Synthesis of 2-(4-((cyclopropylmethyl)sulfonyl)phenyl)-3-methoxypropionic acid

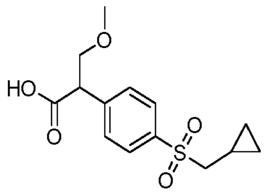

Methyl 2-(4-((cyclopropylmethyl)sulfonyl)phenyl)-3-methoxypropanoate (70.0 mg, 0.224 mmol) was dissolved in methanol (2 mL). At room temperature, a 1M aqueous sodium hydroxide solution (2 mL) was added dropwise to the reaction solution. Upon the addition was complete, the reaction was continued under stirring at room temperature for 2 hours, and monitored by LC-MS for completion. The reaction solution was poured into water (10 mL) and adjusted to pH~4 with 1M dilute hydrochloric acid. The reaction was extracted with EA (10 mL×2), the organic phases were combined, dried over anhydrous sodium sulfate, filtered, and the filtrate was concentrated under reduced pressure to obtain the target compound (60.0 mg, crude product, pale yellow oil). LC-MS (ESI) *m/z* : 297.0 $[M-H]^-$.

**Step 4:** 2-(4-((Cyclopropylmethyl)sulfonyl)phenyl)-N-(2,6-dichloro-4'-(methylsulfonyl)-[1,1'-biphenyl] Preparation of phenyl]-4-yl)-3-methoxypropionamide

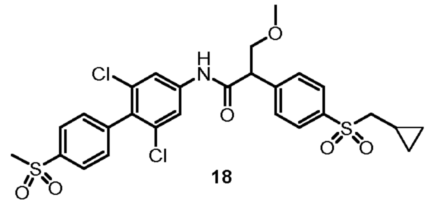

**18**

2-(4-((Cyclopropylmethyl)sulfonyl)phenyl)-3-methoxypropionic acid (60.0 mg, 0.201 mmol) was dissolved in dry DCM (3 mL). At room temperature, thionyl chloride (0.073 mL, 1.01 mmol) was added dropwise to the reaction solution. Upon the addition was complete, the reaction was continued under stirring at room temperature for 3 hours. The intermediate obtained by concentrating the reaction solution under reduced pressure was dissolved in dry DCM (3 mL), and 2,6-dichloro-4'-(methylsulfonyl)-[1,1'-biphenyl]-4-amine (63.6 mg, 0.201 mmol) and DIEA (0.216 mL, 1.31 mmol) was added successively. The reaction mixture was stirred at room temperature for 2 hours. The reaction solution was poured into water (20 mL) and extracted with DCM (15 mL × 2). The organic phases were combined, washed successively with saturated ammonium chloride (20 mL) and saturated aqueous sodium bicarbonate solution (20 mL), dried over anhydrous sodium sulfate, filtered, and the filtrate was concentrated under reduced pressure to obtain a crude product, which was separated and purified by reverse phase column (20% water/80% acetonitrile) to obtain the target

compound (26.0 mg, 19.5% yield over two steps, white solid). LC-MS (ESI) *m/z* : 594.1 [M-H]$^-$.

$^1$H NMR (400 MHz, DMSO-$d_6$) $\delta$ 10.88 (s, 1H), 8.01 (d, *J* = 8.1 Hz, 2H), 7.87 (d, *J* = 8.1 Hz, 2H), 7.84 (s, 2H), 7.66 (d, *J* = 8.0 Hz, 2H), 7.53 (d, *J* = 8.1 Hz, 2H), 4.18 - 4.11 (m, 1H), 3.98 (t, *J* = 8.9 Hz, 1H), 3.62 - 3.55 (m, 1H), 3.28 (s, 3H), 3.27 (s, 3H), 3.23 (d, *J*= 7.1 Hz, 2H), 0.84 - 0.75 (m, 1H), 0.47 - 0.36 (m, 2H), 0.14 - 0.04 (m, 2H).

**Example 19:** <u>*N*-(2,6-Dichloro-4'-(ethylsulfonyl)-[1,1'-biphenyl]-4-yl)-3-methoxy-2-(4-(methylsulfonyl)phenyl)propionamide</u>

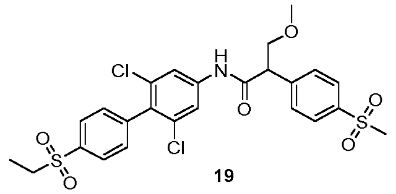

19

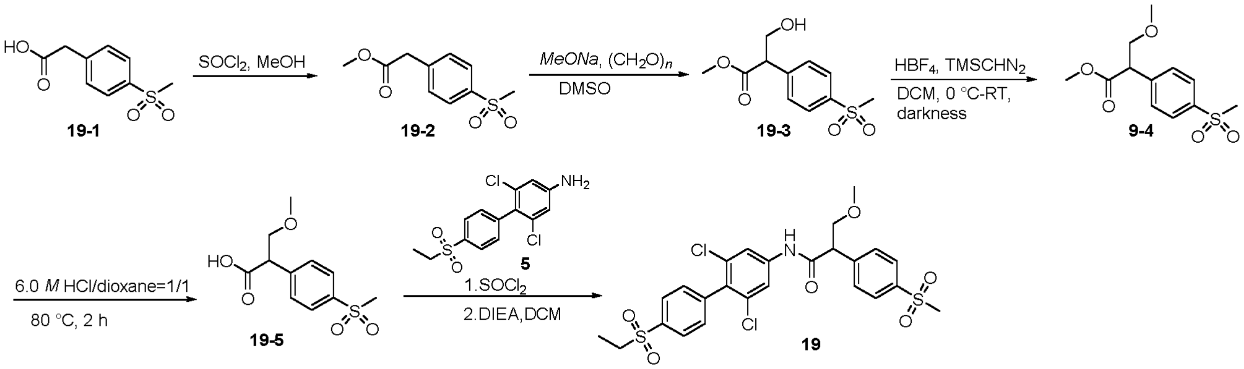


19-1 19-2 19-3 9-4 5 19-5 19

**Step 1:** Synthesis of methyl 2-(4-methylsulfonyl)phenylacetate

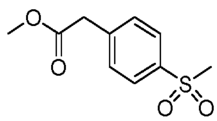

2-(4-Methylsulfonyl) phenylacetic acid (9.00 g, 42.0 mmol) was dissolved in methanol (100.0 mL), and thionyl chloride (15.0 g, 126 mmol) was added at 0°C. The reaction solution was warmed to room temperature and stirred for 1 hour, and then was stopped. The reaction solution was concentrated under reduced pressure, and the obtained residue was separated and purified by column chromatography (PE:EA = 3:1-2:1) to obtain the target compound (8.60 g, yield 89.7%, yellow solid). MS (ESI) m/z: 229.0 [M+H]$^+$.

$^1$H NMR (400 MHz, $CDCl_3$) $\delta$ 7.91 (d, *J* = 8.4 Hz, 2H), 7.49 (d, *J* = 8.5 Hz, 2H), 3.73 (s, 2H), 3.72 (s, 3H), 3.05 (s, 3H).

**Step 2:** Synthesis of methyl 2-(4-methylsulfonyl)phenyl-3-hydroxypropionate

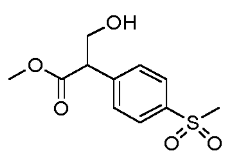

To a solution of methyl 2-(4-methylsulfonyl)phenylacetate (9.40 g, 41.2 mmol) and paraformaldehyde (1.17 g,

39.2 mmol) in anhydrous dimethyl sulfoxide (100.0 mL) was added sodium methoxide (89.0 mg, 1.65 mmol), and the reaction was stirred at room temperature for 16 hours, and then was stopped. The reaction solution was poured into ice water (300.0 mL), 1.0M hydrochloric acid solution was added, and extracted with EA (400 mL × 2). The organic phase was washed with saturated brine (60.0 mL × 3), dried over anhydrous sodium sulfate, filtered, and the filtrate was concentrated under reduced pressure. The obtained residue was separated and purified by column chromatography (PE:EA = 3:1-1:1) to obtain the target compound (6.15 g, yield 57.8%, pale yellow oil). MS (ESI) m/z: 290.1 $[M+NH_4]^+$.

$^1H$ NMR (400 MHz, DMSO-$d_6$) δ 7.87 (d, 2H), 7.57 (d, 2H), 5.08 (t, *J*= 4.9 Hz, 1H), 3.95 - 3.90 (m, 2H), 3.72 - 3.65 (m, 1H), 3.61 (s, 3H), 3.18 (s, 3H).

**Step 3:** Synthesis of methyl 2-(4-(methylsulfonyl)phenyl)-3-methoxypropanoate

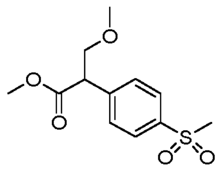

Methyl 2-(4-(methylsulfonyl)phenyl)-3-hydroxypropanoate (6.10 g, 23.6 mmol) was dissolved in DCM (140 mL) and cooled to 0°C with an ice-water bath. Fluoroboric acid (4.14 g, 23.6 mmol, 48% aqueous solution) and trimethylsilyl diazomethane (11.8 mL, 23.6 mmol, 2M) were successively added to the reaction solution under a condition protected from light. After 20 minutes, trimethylsilane diazomethane (11.8 mL, 23.6 mmol) was added again. After 20 minutes, trimethylsilane diazomethane (11.8 mL, 23.6 mmol) was added once again. After 20 minutes, trimethylsilane diazomethane (11.8 mL, 23.6 mmol) was added again. The reaction solution was poured into water (80.0 mL), extracted with DCM (200 mL × 3), and allowed to stand to separate the layers. The organic phase was dried over anhydrous sodium sulfate, filtered, and the filtrate was concentrated under reduced pressure. The obtained residue was separated and purified by column chromatography (PE:EA = 5:1-3:1) to obtain the target compound (4.62 g, yield 71.9%, colorless oil) . MS (ESI) m/z: 273.2 $[M+H]^+$.

$^1H$ NMR (400 MHz, $CDCl_3$) δ 7.90 (d, 2H), 7.53 (d, 2H), 4.00 - 3.92 (m, 2H), 3.71 (s, 3H), 3.69 - 3.65 (m, 1H), 3.35 (s, 3H), 3.04 (s, 3H).

**Step 4:** Synthesis of 2-(4-(methylsulfonyl)phenyl)-3-methoxypropionic acid

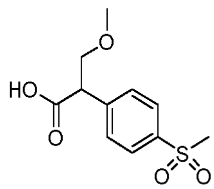

Methyl 2-(4-(methylsulfonyl)phenyl)-3-methoxypropanoate (1.00 g, 3.67 mmol) was dissolved in the mixture of 1,4-dioxane (30.0 mL) and 6.0 *M* hydrochloric acid (30.0 mL). The reaction solution was stirred at 80°C for 2 hours, and then was stopped. The reaction solution was cooled to room temperature, carefully poured into water (80.0 mL), extracted with EA (150 mL × 2), and allowed to stand to separate the layers. The organic phase was dried over anhydrous sodium sulfate, filtered, and the filtrate was concentrated under reduced pressure. The obtained residue was purified by reverse phase chromatography (acetonitrile/water containing 0.05% formic acid) to obtain the target compound (880 mg, yield 77.3%, white solid). MS (ESI) m/z: 276.0 $[M+ NH_4]^+$.

$^1H$ NMR (400 MHz, $CDCl_3$) δ 7.91 (d, *J*= 8.3 Hz, 2H), 7.54 (d, *J*= 8.3 Hz, 2H), 4.01 - 3.92 (m, 2H), 3.72 - 3.66 (m, 1H), 3.37 (s, 3H), 3.05 (s, 3H).

**Step 5:** Synthesis of *N*-(2,6-Dichloro-4'-(ethylsulfonyl)-[1,1'-biphenyl]-4-yl)-3-methoxy-2-(4-(methylsulfonyl)phenyl)propionamide

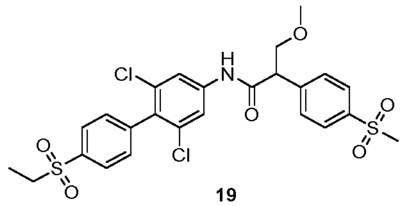

19

2-(4-(Methylsulfonyl)phenyl)-3-methoxypropionic acid (105 mg, 0.407 mmol) was dissolved in DCM (3.00 mL), and thionyl chloride (52.4 mg, 118.97 mmol) was added at 0°C. The reaction solution was stirred at room temperature for 2 hours, dried under reduced pressure to remove the solvent to obtain a crude product. The crude product was redissolved in DCM (3.0 mL) and carefully added dropwise to the ice-water bath cooled solution of DIEA (131 mg, 1.02 mmol) and 2,6-dichloro-4'-(ethylsulfonyl)-[1,1'-biphenyl]-4-amine (112 mg, 0.339 mmol) in DCM (2.00 mL). The reaction solution was stirred for 20 minutes in an ice-water bath. The crude product was obtained by drying under reduced pressure and removing the solvent, which was subject to high pressure liquid chromatography (acetonitrile/water containing 0.05% ammonia water) to obtain the target compound (51.3 mg, yield 26.6%, pale yellow solid). MS (ESI) m/z: 570.2 $[M+H]^+$.

$^1H$ NMR (400 MHz, $CDCl_3$) δ 8.52 (s, 1H), 7.97 (d, *J* = 8.4 Hz, 2H), 7.92 (d, *J* = 8.2 Hz, 2H), 7.70 (s, 2H), 7.59 (d, *J* = 8.3 Hz, 2H), 7.44 (d, *J* = 8.4 Hz, 2H), 4.06 - 4.01 (m, 1H), 4.00 - 3.94 (m, 1H), 3.80 - 3.74 (m, 1H), 3.48 (s, 3H), 3.19 (q, *J*= 7.4 Hz, 2H), 3.06 (s, 3H), 1.33 (t, *J*= 7.4 Hz, 3H).

**Example 20:** *N*-(2,6-Dichloro-4'-(cyclopropylsulfonyl)-[1,1'-biphenyl]-4-yl)-3-methoxy-2-(4-(methylsulfonyl)phenyl)propionamide

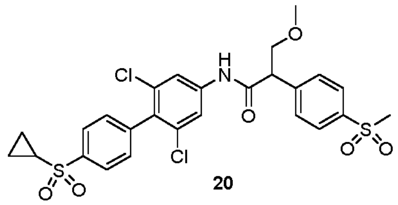

20

3-Methoxy-2-(4-methylsulfonyl) phenyl)propanoic acid (105 mg, 0.407 mmol) was dissolved in DCM (3.00 mL), and thionyl chloride (52.4 mg, 0.441 mmol) was added at 0°C. The reaction solution was stirred at room temperature for 2 hours, dried under reduced pressure to remove the solvent, the crude product was redissolved in DCM (3.0 mL), and carefully added dropwise to the ice-water bath cooled solution of DIEA (131 mg, 1.02 mmol) and 2,6-dichloro-4'-(cyclopropylsulfonyl)-[1,1'-biphenyl]-4-amine (116 mg, 0.339 mmol) in DCM (2.0 mL). The reaction solution was stirred for 20 minutes in an ice-water bath. The crude product was obtained by drying under reduced pressure and removing the solvent, which was subject to high pressure liquid chromatography (acetonitrile/water containing 0.05% ammonia water) to obtain the target compound (52.9 mg, yield 26.9%, off-white solid). MS (ESI) m/z: 582.0 $[M+H]^+$.

$^1H$ NMR (400 MHz, $CDCl_3$) δ 8.65 (s, 1H), 7.97 (d, *J* = 8.0 Hz, 2H), 7.93 (d, *J* = 7.6 Hz, 2H), 7.72 (s, 2H), 7.61 (d, *J*= 7.7 Hz, 2H), 7.43 (d, *J*= 8.0 Hz, 2H), 4.08 - 3.98 (m, 2H), 3.79 - 3.74 (m, 1H), 3.48 (s, 3H), 3.06 (s, 3H), 2.57 - 2.50 (m, 1H), 1.43 - 1.38 (m, 2H), 1.13 - 1.05 (m, 2H).

**Example 21:** *N*-(2,6-Dichloro-4'-(cyclopropylsulfonyl)-[1,1'-biphenyl]-4-yl)-2-(5-(ethylsulfonyl) pyridin-2-yl)acetamide

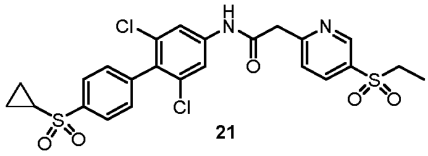

21

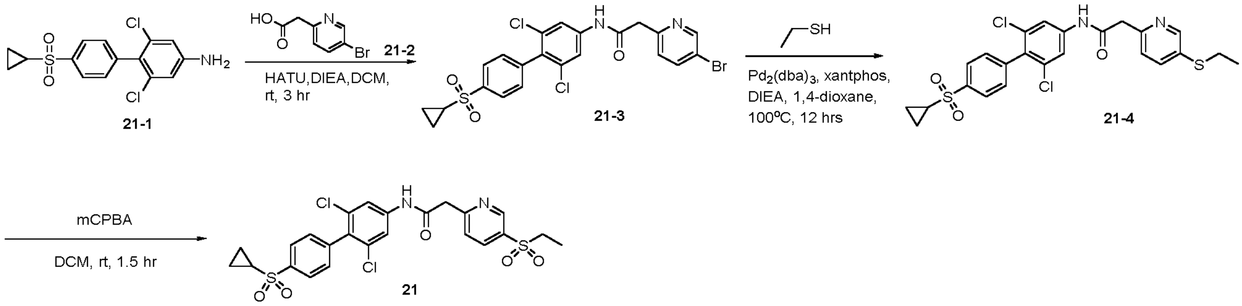


21-1 21-3 21-4 21

**Step 1:** Synthesis of 2-(5-Bromopyridin-2-yl)-*N*-(2,6-dichloro-4'-(cyclopropylsulfonyl)-[1,1'-biphenyl]-4-yl)acetamide

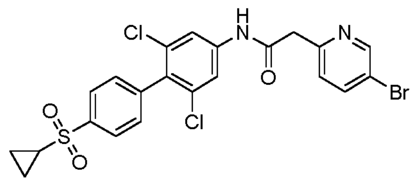

2,6-Dichloro-4'-(cyclopropylsulfonyl)-[1,1'-biphenyl]-4-amine (150 mg, 0.438 mmol), 2-(5-bromopyridine-2-yl)acetic acid (114 mg, 0.526 mmol), DIEA (0.217 mL, 1.31 mmol) and HATU (250 mg, 0.658 mmol) were added to DCM (5 mL). The reaction mixture was stirred at room temperature for 2 hours and monitored by LC- MS for completion. The reaction solution was poured into water (20 mL) and extracted with DCM (10 mL × 2). The organic phases were combined, washed successively with saturated ammonium chloride (15 mL) and saturated aqueous sodium bicarbonate solution (15 mL), dried over anhydrous sodium sulfate, filtered, and the filtrate was concentrated under reduced pressure to obtain the target compound (283 mg, crude product, yellow oil). LC-MS (ESI) *m/z* : 538.9 $[M+H]^+$.

**Step 2:** Synthesis of *N*-(2,6-Dichloro-4'-(cyclopropylsulfonyl)-[1,1'-biphenyl]-4-yl)-2-(5-(ethylthio)pyridin-2-yl)acetamide

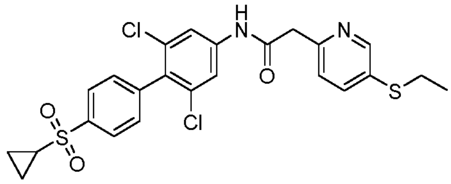

2-(5-Bromopyridin-2-yl)-N-(2,6-dichloro-4'-(cyclopropylsulfonyl)-[1,1'-biphenyl]-4-yl)acetamide (283 mg, 0.524 mmol), $Pd_2(dba)_3$ (48.0 mg, 0.0524 mmol), xantphos (30.3 mg, 0.0524 mmol), DIEA (0.173 mL, 1.05 mmol) and ethanethiol (0.0784 mL, 1.05 mmol) were added to 1,4-dioxane (2 mL). Under nitrogen protection, the reaction mixture was stirred in a sealed tube at 100°C overnight, and monitored by LC-MS for completion. The reaction mixture was cooled to room temperature, filtered, and the filtrate was concentrated under reduced pressure to obtain a crude product, which was separated and purified by silica gel column (PE:EA = 4:1-2:1) to obtain the target compound (170 mg, yield 74.6% over two steps, yellow oil). LC-MS (ESI) *m/z* : 520.9 $[M + H]^+$.

**Step 3:** Synthesis of *N*-(2,6-Dichloro-4'-(cyclopropylsulfonyl)-[1,1'-biphenyl]-4-yl)-2-(5-(ethylsulfonyl)pyridin-2-yl)acetamide

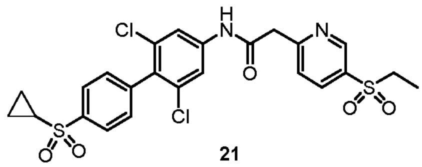

**21**

*N*-(2,6-Dichloro-4'-(cyclopropylsulfonyl)-[1,1'-biphenyl]-4-yl)-2-(5-(ethylthio)pyridin-2-yl)acetamide (170 mg, 0.326 mmol) was dissolved in DCM (5 mL), and m-chloroperoxybenzoic acid (132 mg, 0.652 mmol) was slowly added to the reaction solution under ice bath. Upon the addition was complete, the ice bath was removed, and the reaction was continued under stirring at room temperature for 1.5 hours, and monitored by LC-MS for completion. The reaction solution was poured into saturated aqueous sodium bicarbonate solution (20 mL), extracted with DCM (10 mL×2), the organic phases were combined, washed with saturated brine (15 mL), dried over anhydrous sodium sulfate, filtered, and the filtrate was concentrated under reduced pressure to obtain a crude product, which was separated and purified by silica gel column (PE:EA = 1:9) to obtain crude product (120 mg). Further separation and purification by reverse phase column (water : acetonitrile = 3:7) gave the target compound (76.0 mg, yield 42.1%, white solid). LC-MS (ESI) *m*/*z* : 552.9 $[M + H]^+$.

$^1$H NMR (500 MHz, MeOD) $\delta$ 8.90 (d, *J* = 2.4 Hz, 1H), 8.20 (dd, *J* = 8.2, 2.4 Hz, 1H), 7.91 (d, *J* = 8.5 Hz, 2H), 7.73 (s, 2H), 7.62 (d, *J*= 8.2 Hz, 1H), 7.42 (d, *J*= 8.5 Hz, 2H), 3.25 - 3.20 (m, 2H), 2.71 (s, 2H), 2.68 - 2.63 (m, 1H), 1.21 - 1.19 (m, 2H), 1.17 (t, *J* = 7.4 Hz 3H), 1.03 - 1.01 (m, 2H).

**Example 22: 3-**(2,6-Dichloro-4'-(cyclopropylsulfonyl)-[1,1'-biphenyl]-4-amino)-2-(4-(ethylsulfonyl) phenyl)propionic acid

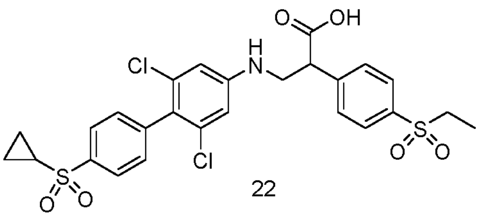

22

2,6-Dichloro-4'-(cyclopropylsulfonyl)-[1,1'-biphenyl]-4-amine (30 mg, 0.0877 mmol) and 4-(ethylsulfonyl)phenyl)-3-hydroxypropionic acid (27.1 mg, 0.105 mmol) were added to DCM (3 mL), and HOBt (17.8 mg, 0.132 mmol), EDC (25.3 mg, 0.132 mmol) and DIEA (0.043 mL, 0.263 mmol) were added successively. The reaction mixture was stirred at room temperature for 3 days. To the reaction mixture was added saturated aqueous sodium bicarbonate solution (10 mL) and DCM (10 mL). The aqueous phase was separated and extracted with DCM (10 mL). The combined organic phases were washed with saturated brine, dried over anhydrous sodium sulfate, and filtered. The filtrate was concentrated under reduced pressure to obtain a crude product, which was separated and purified by preparative plate (silica gel, dichloromethane : methanol = 15 : 1), to obtain the target compound (6 mg, yield 11.7%, white solid). LC-MS (ESI) m/z 582.0 $[M+H]^+$.

$^1$H NMR (400 MHz, MeOH-$d_4$) $\delta$ 7.95 (d, *J* = 8.4 Hz, 2H), 7.91 (d, *J* = 8.4 Hz, 2H), 7.68 (d, *J* = 8.4 Hz, 2H), 7.48 (d, *J*= 8.4 Hz, 2H), 6.70 (s, 2H), 4.09-4.04 (m, 1H), 3.90-3.84 (m, 1H), 3.60-3.54 (m, 1H), 3.20 (q, *J* = 7.6 Hz, 2H), 2.78-2.72 (m, 1H), 1.31-1.27 (m, 2H), 1.23 (t, *J*= 7.4 Hz, 3H), 1.15-1.08 (m, 2H).

**Example 22-1:** *N*-(2,6-Dichloro-4'-(cyclopropylsulfonyl)-[1,1'-biphenyl]-4-yl)-2-(4-(ethylsulfonyl) phenyl)-3-hydroxypropionamide

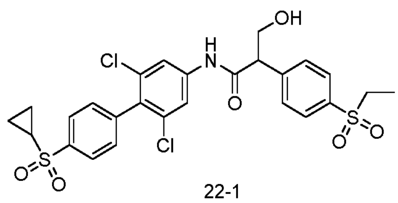

22-1

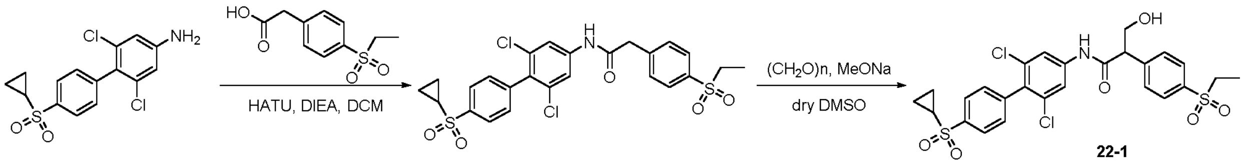


**Step 1**: Synthesis of *N*-(2,6-Dichloro-4'-(cyclopropylsulfonyl)-[1,1'-biphenyl]-4-yl)-2-(4-(ethylsulfonyl)phenyl)acetamide

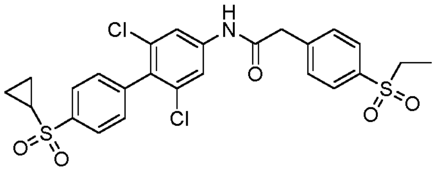

2,6-Dichloro-4'-(cyclopropylsulfonyl)-[1,1'-biphenyl]-4-amine (100 mg, 0.292 mmol) and 2-(4-(ethylsulfonyl)phenyl)acetic acid (79.9 mg, 0.350 mmol) were added to DCM (5 mL). To the reaction mixture was added successively HATU (133 mg, 0.350 mmol) and DIEA (0.145 mL, 0.876 mmol). The reaction mixture was stirred at room temperature for 2 hours. Saturated aqueous sodium bicarbonate solution (10 mL) and DCM (10 mL) were added. The organic phase was separated, washed with saturated brine, dried over anhydrous sodium sulfate, and filtered. The filtrate was concentrated under reduced pressure to obtain a crude product, which was separated and purified by column chromatography (silica gel, EA:PE = 0~40%), to obtain the target compound (150 mg, yield 93.2%, colorless oil). LC-MS (ESI) *m*/*z* : 569.0 $[M+NH_4]^+$.

**Step 2**: Synthesis of *N*-(2,6-Dichloro-4'-(cyclopropylsulfonyl)-[1,1'-biphenyl]-4-yl)-2-(4-(ethylsulfonyl)phenyl)-3-hydroxypropionamide

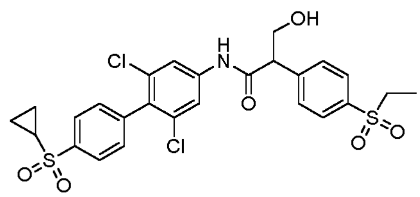

*N*-(2,6-Dichloro-4'-(cyclopropylsulfonyl)-[1,1'-biphenyl]-4-yl)-2-(4-(ethylsulfonyl)phenyl) acetamide (60 mg, 0.109 mmol) and paraformaldehyde (2.94 mg, 0.098 mmol) were added to anhydrous dimethylsulfoxide (2 mL). To the reaction mixture was added sodium methoxide (0.24 mg, 0.004 mmol). The reaction mixture was stirred at room temperature for 2 hours. EA (10 mL) and water (10 mL) were added. The organic phase was separated, the aqueous phase was extracted with EA (10 mL × 2), the combined organic phases were washed with saturated brine, dried over anhydrous sodium sulfate, and filtered. The filtrate was concentrated under reduced pressure to obtain a crude product, which was separated and purified by column chromatography (silica gel, EA/(DCM/PE = 1/2) = 0~100%), to obtain the target compound (30 mg, yield 47.6%, white solid). LC-MS (ESI) *m*/*z* : 599.0 $[M+NH_4]^+$.

$^1H$ NMR (500 MHz, DMSO-$d_6$) $\delta$ 10.68 (s, 1H), 7.99 (d, *J*= 8.4 Hz, 2H), 7.91-7.84 (m, 4H), 7.66 (d, *J*= 8.4 Hz, 2H), 7.55 (d, *J* = 8.4 Hz, 2H), 5.14 (t, *J* = 5.2 Hz, 1H), 4.11-4.06 (m, 1H), 4.00-3.95 (m, 1H), 3.70 -3.65 (m, 1H), 3.28 (q, *J* = 7.3 Hz, 2H), 2.99-2.94 (m, 1H), 1.20-1.15 (m, 2H), 1.13-1.06 (m, 5H).

**Example 33**: *N*-(2,6-Dichloro-4'-(cyclopropylsulfonyl)-[1,1'-biphenyl]-4-yl)-2-(4-(methylsulfonyl) phenyl)-3-hydroxypropionamide

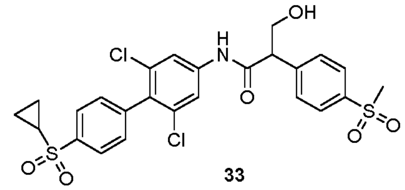

33

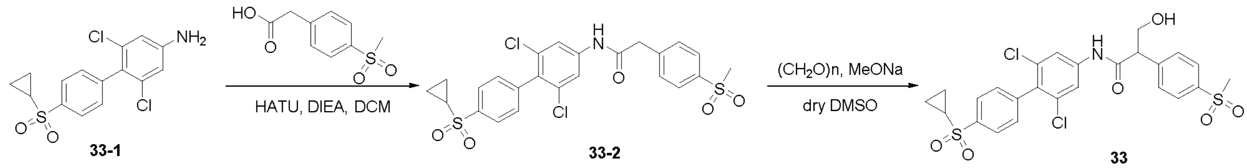


33-1

33-2

33

**Step 1:** Synthesis of *N*-(2,6-Dichloro-4'-(cyclopropylsulfonyl)-[1,1'-biphenyl]-4-yl)-2-(4-(methylsulfonyl)phenyl)acetamide

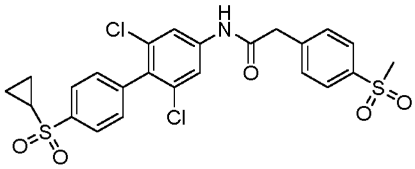

2,6-Dichloro-4'-(cyclopropylsulfonyl)-[1,1'-biphenyl]-4-amine (100 mg, 0.292 mmol) and 2-(4-(methylsulfonyl)phenyl)acetic acid (75.0 mg, 0.350 mmol) were added to DCM (5 mL). To the reaction mixture was successively added HATU (133 mg, 0.350 mmol) and DIEA (0.145 mL, 0.876 mmol). The reaction mixture was stirred at room temperature for 2 hours. Saturated aqueous sodium bicarbonate solution (10 mL) and DCM (10 mL) were added. The organic phase was separated, washed with saturated brine, dried over anhydrous sodium sulfate, and filtered. The filtrate was concentrated under reduced pressure to obtain a crude product, which was separated and purified by column chromatography (silica gel, EA : PE = 0 ~ 40 %), to obtain the target compound (145 mg, yield 92.4%, yellow solid). LC-MS (ESI) *m/z* : 555.0 $[M+NH_4]^+$.

**Step 2:** Synthesis of *N*-(2,6-Dichloro-4'-(cyclopropylsulfonyl)-[1,1'-biphenyl]-4-yl)-2-(4-(methylsulfonyl)phenyl)-3-hydroxypropionamide

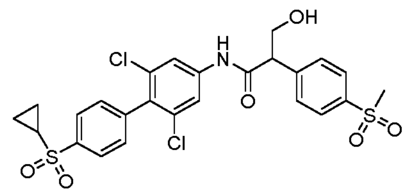

*N*-(2,6-Dichloro-4'-(cyclopropylsulfonyl)-[1,1'-biphenyl]-4-yl)-2-(4-(methylsulfonyl)phenyl) acetamide (60 mg, 0.111 mmol) and paraformaldehyde (3.0 mg, 0.100 mmol) were added to anhydrous dimethylsulfoxide (2 mL). To the reaction mixture was added sodium methoxide (0.24 mg, 0.004 mmol). The reaction mixture was stirred at room temperature for 2 hours. Ethyl acetate (10 mL) and water (10 mL) were added. The separated aqueous phase was extracted with EA (10 mL × 2). The combined organic phases were washed with saturated brine, dried over anhydrous sodium sulfate, and filtered. The filtrate was concentrated under reduced pressure to obtain a crude product, which was separated

and purified by column chromatography (silica gel, EA : (DCM:PE = 1:2) = 0~100%), to obtain the target compound (35 mg, yield 55.6%, white solid). LC-MS (ESI) *m/z* : 585.0 $[M+NH_4]^+$. $^1H$ NMR (500 MHz, DMSO-$d_6$) $\delta$ 10.68 (s, 1H), 8.00 (d, *J* = 8.4 Hz, 2H), 7.92 (d, *J* = 8.4 Hz, 2H), 7.86 (s, 2H), 7.65 (d, *J* = 8.4 Hz, 2H), 7.55 (d, *J* = 8.4 Hz, 2H), 5.14 (t, *J* = 5.2 Hz, 1H), 4.13-4.06 (m, 1H), 4.01-3.94 (m, 1H), 3.70-3.63 (m, 1H), 3.20 (s, 3H), 3.00-2.93 (m, 1H), 1.20-1.16 (m, 2H), 1.12-1.07 (m, 2H).

**Examples 34 and 35:** (S)-*N*-(2,6-dichloro-4'-(cyclopropylsulfonyl)-[1,1'-biphenyl]-4-yl)-3-methoxy-2-(4-(methylsulfonyl)phenyl)propanamide or (R)-*N*-(2,6-dichloro-4'-(cyclopropylsulfonyl)-[1,1'-biphenyl]-4-yl)-3-methoxy-2-(4-(methylsulfonyl)phenyl)propanamide

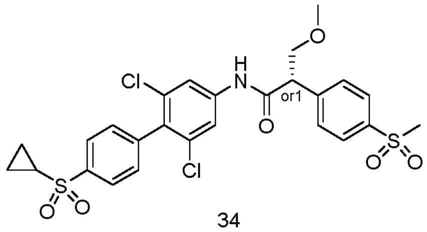

34

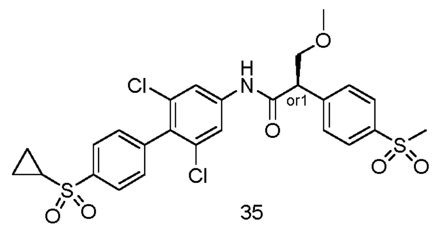

35

Racemate *N*-(2,6-Dichloro-4'-(cyclopropylsulfonyl)-[1,1'-biphenyl]-4-yl)-3-methoxy-2-(4-(methylsulfonyl)phenyl)propanamide (**Example 20,** 500mg dissolved in about 80mL methanol, injection volume 5.0mL) was separated through a Waters SFC 150 (room temperature, 100bar, 214nm) and 250*25mm 10μm Dr.maish Reprosil Chiral-OM (similar to DAICELCHIRALCEL® OD) (supercritical carbon dioxide:methanol, 50:50, 3.0min, 70 mL/min) to obtain Example 34 (212 mg, white solid, *Ret.*time = 1.284 min, ee 99%), LC-MS (ESI) *m/z* : 582.1 $[M+H]^+$/584.1 $[M+2+H]^+$; $^1H$ NMR (400 MHz, $CDCl_3$) $\delta$ 8.41 (s, 1H), 8.05 - 7.87 (m, 4H), 7.69 (s, 2H), 7.64 - 7.53 (m, 2H), 7.43 (d, *J*= 6.9 Hz, 2H), 4.09 - 3.91 (m, 2H), 3.85 - 3.75 (m, 1H), 3.51 (s, 3H), 3.07 (s, 3H), 2.60 - 2.49 (m, 1H), 1.47 - 1.36 (m, 2H), 1.16 - 1.03 (m, 2H); and **Example 35** (208 mg, white solid, *Ret.* time = 1.711 min, ee 9.8%); LC-MS (ESI) *m/z* : 582.1 $[M+H]^+$/584.1 $[M+2+H]^+$. $^1H$ NMR (400 MHz, $CDCl_3$) $\delta$ 8.46 (s, 1H), 8.00 - 7.91 (m, 4H), 7.69 (s, 2H), 7.59 (d, *J*= 8.3 Hz, 2H), 7.43 (d, *J*= 8.3 Hz, 2H), 4.07 - 4.00 (m, 1H), 3.99 - 3.93 (m, 1H), 3.82 - 3.75 (m, 1H), 3.50 (s, 3H), 3.06 (s, 3H), 2.59 - 2.50 (m, 1H), 1.45 - 1.38 (m, 2H), 1.13 - 1.06 (m, 2H).

**Examples 36 and 37:** (S)-*N*-(2,6-Dichloro-4'-(cyclopropylsulfonyl)-[1,1'-biphenyl]-4-yl)-2-(4-(methylsulfonyl)phenyl)-3-hydroxypropionamide or (R)-*N*-(2,6-dichloro-4'-(cyclopropylsulfonyl)-[1,1'-biphenyl]-4-yl)-2-(4-(methylsulfonyl)phenyl)-3-hydroxypropionamide

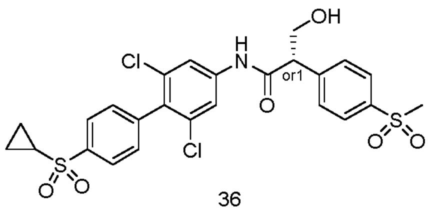

36

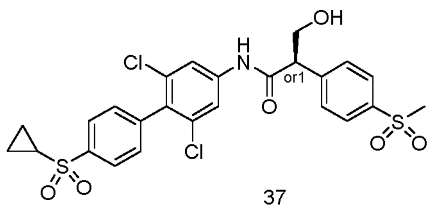

37

Racemate *N*-(2,6-dichloro-4'-1,1'-biphenyl]-4-yl)-2-(4-(methylsulfonyl)phenyl)-3-hydroxypropionamide (**Example 33,** 500 mg dissolved in about 120 mL methanol, injection volume 2.5 mL) was separated through a Waters SFC 150 (room temperature, 100bar, 214nm) and a 250*25mm 10μm Dr.maish Reprosil Chiral-OM (similar to DAICELCHIRALCEL® OD) (supercritical carbon dioxide : methanol, 55:45, 3.0 min, 70 mL/min) to obtain **Example 36** (158 mg, white solid, *Ret.*time = 1.373 min, ee 99%), LC-MS (ESI) *m/z* : 567.9 $[M+H]^+$; $^1H$ NMR (400 MHz, $CDCl_3$) $\delta$ 8.14 (s, 1H), 7.97 (d, *J* = 8.5 Hz, 2H), 7.91 (d, *J* = 8.4 Hz, 2H), 7.70 (s, 2H), 7.56 (d, *J*= 8.4 Hz, 2H), 7.43 (d, *J*= 8.5 Hz, 2H), 4.23 - 4.16 (m, 1H), 4.05 - 3.99 (m, 1H), 3.98 - 3.92 (m, 1H), 3.07 (s, 3H), 2.58 - 2.50 (m, 1H), 1.44 - 1.38 (m, 2H), 1.13 - 1.06 (m, 2H); and **Example 37** (102 mg, white solid, *Ret.*time = 1.701 min, ee 99%), LC-MS (ESI) *m/z* : 567.9 $[M+H]^+$; $^1H$ NMR (400 MHz, $CDCl_3$) $\delta$ 8.00 (s, 1H), 7.97 (d, *J* = 8.4 Hz, 2H), 7.93 (d, *J* = 8.2 Hz, 2H), 7.69 (s, 2H), 7.57 (d, *J* = 8.2 Hz, 2H), 7.43 (d, *J* = 8.3 Hz, 2H), 4.26 - 4.17 (m, 1H), 4.07 - 3.99 (m, 1H), 3.98 - 3.91 (m, 1H), 3.07 (s, 3H), 2.59 - 2.48 (m, 1H), 1.44 - 1.38 (m, 2H), 1.13 - 1.07 (m, 2H).

**Examples 38 and 39:** (S)-*N*-(2,6-dichloro-4'-(cyclopropylsulfonyl)-[1,1'-biphenyl]-4-yl)-2-(4-(ethylsulfonyl)phenyl)-3-hydroxypropionamide or (R)-*N*-(2,6-dichloro-4'-(cyclopropylsulfonyl)-[1,1'-biphenyl]-4-yl)-2-(4-(ethylsulfonyl)phenyl)-3-hydroxypropionamide

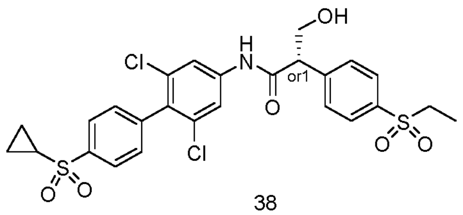
38

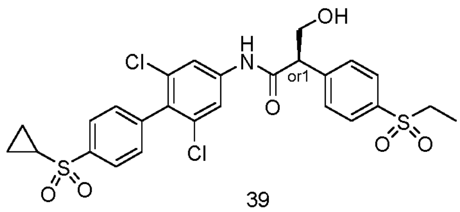
39

Racemate *N*-(2,6-Dichloro-4'-(cyclopropylsulfonyl)-[1,1'-biphenyl]-4-yl)-2-(4-(ethylsulfonyl) phenyl)-3-hydroxypropionamide (**Example 22,** 500 mg dissolved in about 150 mL methanol, injection volume 5.0 mL) was separated through a Waters SFC 150 (room temperature, 100 bar, 214 nm) and a 250*25 mm 10 μm Dr. maish Reprosil Chiral-OM (similar to DAICELCHIRALCEL®OD) (supercritical carbon dioxide : methanol, 55:45, 3.2 min, 70 mL/min) to obtain **Example 38** (165 mg, white solid, *Ret.* time = 1.370 min, ee 99%), LC-MS (ESI) *m/z* : 581.9 $[M+H]^+$/583.9 $[M+2+H]^+$; $^1$H NMR (400 MHz, $CDCl_3$) δ 8.01 (s, 1H), 7.97 (d, *J* = 8.4 Hz, 2H), 7.88 (d, *J* = 8.3 Hz, 2H), 7.70 (s, 2H), 7.56 (d, *J*= 8.3 Hz, 2H), 7.43 (d, *J*= 8.4 Hz, 2H), 4.24 - 4.16 (m, 1H), 4.06 - 3.99 (m, 1H), 3.99 - 3.91 (m, 1H), 3.14 (q, *J* = 7.4 Hz, 2H), 2.59 - 2.50 (m, 1H), 1.44 - 1.38 (m, 2H), 1.31 (t, *J* = 7.4 Hz, 3H), 1.13 - 1.06 (m, 2H); and **Example 39** (117 mg, white solid, *Ret.* time = 1.759 min, ee 99%), LC-MS (ESI) *m/z* 581.9 $[M+H]^+$/583.9 $[M+2+H]^+$; $^1$H NMR (400 MHz, $CDCl_3$) δ 8.08 (s, 1H), 7.97 (d, *J* = 8.4 Hz, 2H), 7.87 (d, *J* = 8.2 Hz, 2H), 7.71 (s, 2H), 7.56 (d, *J* = 8.3 Hz, 2H), 7.43 (d, *J* = 8.4 Hz, 2H), 4.24 - 4.17 (m, 1H), 4.06 - 3.99 (m, 1H), 3.98 - 3.90 (m, 1H), 3.13 (q, *J*= 7.4 Hz, 2H), 2.59 - 2.49 (m, 1H), 1.44 - 1.38 (m, 2H), 1.31 (t, *J* = 7.4 Hz, 3H), 1.13 - 1.06 (m, 2H).

**Examples 40 and 41:** (S)-*N*-(2,6-dichloro-4'-(cyclopropylsulfonyl)-[1,1'-biphenyl]-4-yl)-2-(4-(ethylsulfonyl)phenyl)-3-methoxypropionamide or (R)-*N*-(2,6-dichloro-4'-(cyclopropylsulfonyl)-[1,1'-biphenyl]-4-yl)-2-(4-(ethylsulfonyl)phenyl)-3-methoxypropionamide

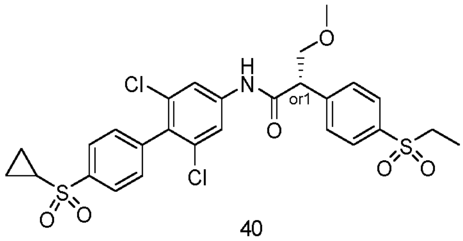
40

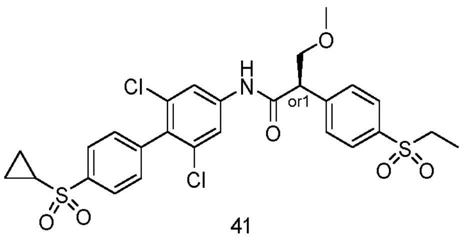
41

Racemate *N*-(2,6-Dichloro-4'-(cyclopropylsulfonyl)-[1,1'-biphenyl]-4-yl)-2-(4-(ethylsulfonyl) phenyl)-3-methoxypropionamide (**Example 16,** 500 mg dissolved in about 100 mL methanol, injection volume 8.0 mL) was separated through a Waters SFC 150 (room temperature, 100 bar, 214 nm) and 250*25 mm 10 μm Dr.maish Reprosil Chiral-OM (similar to DAICELCHIRALCEL® OD) (supercritical carbon dioxide : methanol, 50:50, 3.5 min, 70 mL/min) to obtain **Example 40** (175 mg, white solid, *Ret.* time = 1.287 min, ee 99%), LC-MS (ESI) *m/z* 596.1$[M+H]^+$/598.1 $[M+2+H]^+$; $^1$H NMR (400 MHz, $CDCl_3$) δ 8.40 (s, 1H), 7.97 (d, *J* = 8.3 Hz, 2H), 7.91 (d, *J* = 8.3 Hz, 2H), 7.69 (s, 2H), 7.58 (d, *J*= 8.3 Hz, 2H), 7.43 (d, *J*= 8.3 Hz, 2H), 4.06 - 3.99 (m, 1H), 3.97 - 3.92 (m, 1H), 3.82 - 3.77 (m, 1H), 3.50 (s, 3H), 3.13 (q, *J*= 7.4 Hz, 2H), 2.58 - 2.51 (m, 1H), 1.44 - 1.38 (m, 2H), 1.30 (t, *J* = 7.4 Hz, 3H), 1.12 - 1.06 (m, 2H); and **Example 41** (153 mg, white solid, *Ret.* time = 1.797 min, ee 99%), LC-MS (ESI) m /*z* 596.1 $[M+H]^+$/598.1 $[M+2+H]^+$; $^1$H NMR (400 MHz, $CDCl_3$) δ 8.52 (s, 1H), 7.97 (d, *J*= 8.3 Hz, 2H), 7.88 (d, *J*= 8.3 Hz, 2H), 7.71 (s, 2H), 7.58 (d, *J*= 8.3 Hz, 2H), 7.43 (d, *J*= 8.3 Hz, 2H), 4.07 - 4.00 (m, 1H), 4.00 - 3.94 (m, 1H), 3.80 - 3.73 (m, 1H), 3.48 (s, 3H), 3.13 (q, *J*= 7.4 Hz, 2H), 2.59 - 2.50 (m, 1H), 1.45 - 1.37 (m, 2H), 1.30 (t, *J*= 7.4 Hz, 3H), 1.15 - 1.06 (m, 2H).

**Example 42:** <u>*N*-(4'-(Cyclopropylsulfonyl)-2-(trifluoromethyl)-[1,1'-biphenyl]-4-yl)-3-methoxy-2-(4-(methylsulfonyl)phenyl)propionamide</u>

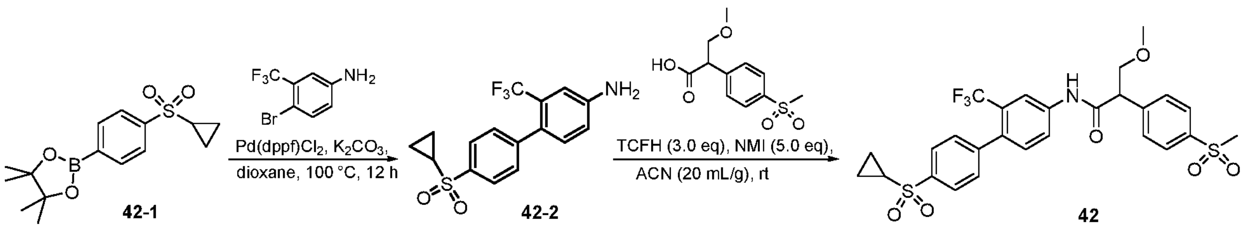


42-1 42-2 42

**Step 1**: Synthesis of 4'-(cyclopropylsulfonyl)-2-(trifluoromethyl)-[1,1'-biphenyl]-4-amine

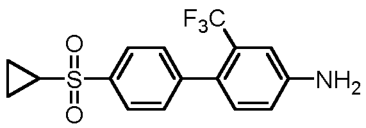

To a solution of 4-bromo-3-(trifluoromethyl)-aniline (1.00 g, 4.17 mmol) and 2-(4-(cyclopropylsulfonyl)phenyl)-4,4,5,5-tetramethyl-1,3,2-dioxaborolane (1.93 g, 6.23 mmol) in 1,4-dioxane (40.0 mL) was added Pd(dppf)$Cl_2$ (305 mg, 0.417 mmol) and potassium carbonate (1.15 g, 8.32 mmol). Under argon protection, the reaction mixture was stirred at 100°C for 12 hours, and then the reaction was stopped. After cooling the reaction solution to room temperature, the solvent was removed by rotary evaporation under reduced pressure, and the obtained residue was separated and purified by column chromatography (PE : EA = 10:1, 5:1-3:1) to obtain the target compound (820 mg, yield 57.7%, yellow solid). LC-MS (ESI) *m/z* 342.1 $[M+H]^+$.

$^1$H NMR (400 MHz, $CDCl_3$) $\delta$ 7.90 (d, *J*= 8.2 Hz, 2H), 7.48 (d, *J* = 8.1 Hz, 2H), 7.15-7.06 (m, 2H), 6.94 (d, *J*= 7.5 Hz, 1H), 2.63 - 2.39 (m, 1H), 1.45 - 1.34 (m, 2H), 1.10 - 1.05 (m, 2H).

**Step 2**: *N*-(4'-(Cyclopropylsulfonyl)-2-(trifluoromethyl)-[1,1'-biphenyl]-4-yl)-3-methoxy-2-(4-(methylsulfonyl)phenyl)propionamide

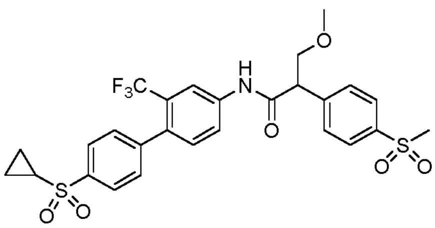

To a solution of 4'-(cyclopropylsulfonyl)-2-(trifluoromethyl)-[1,1'-biphenyl]-4-amine (200 mg, 0.586 mmol), 2-(4-(methylsulfonyl)phenyl)-3-methoxypropionic acid (303 mg, 1.17 mmol) and *N*-methylimidazole (241 mg, 2.93 mmol) in acetonitrile (4.00 mL) was added *N,N,N',N'*-tetra methylchloroformamidine hexafluorophosphate (493 mg, 1.76 mmol). The reaction solution was stirred at room temperature for 16 hours, and then the reaction was stopped. The reaction solution was directly preparatively purified by high pressure liquid chromatography (acetonitrile/water containing 0.05% formic acid) to obtain the target compound (91.4 mg, yield 26.8%, white solid). LC-MS (ESI) *m/z* : 581.9 $[M+H]^+$.

$^1$H NMR (400 MHz, $CDCl_3$) $\delta$ 8.45 (s, 1H), 7.98 - 7.90 (m, 5H), 7.81 (dd, *J*= 8.3, 2.1 Hz, 1H), 7.61 (d, *J*= 8.4 Hz, 2H), 7.48 (d, *J*= 8.3 Hz, 2H), 7.28-7.26 (d,*J*=8.4,1H),4.09 - 4.02 (m, 1H), 4.01 - 3.95 (m, 1H), 3.83-3.78 (m, 1H), 3.51 (s, 3H), 3.06 (s, 3H), 2.56 - 2.48 (m, 1H), 1.43 - 1.37 (m, 2H), 1.12 - 1.05 (m, 2H).

**Examples 43 and 44:** **(S)**-*N*-(4'-(cyclopropylsulfonyl)-2-(trifluoromethyl)-[1,1'-biphenyl]-4-yl)-3-methoxy-2-(4-(methylsulfonyl)phenyl)propionamide or **(R)**-*N*-(4'-(cyclopropylsulfonyl)-2-(trifluoromethyl)-[1,1'-biphenyl]-4-yl)-3-methoxy-2-(4-(methylsulfonyl)phenyl)propanamide

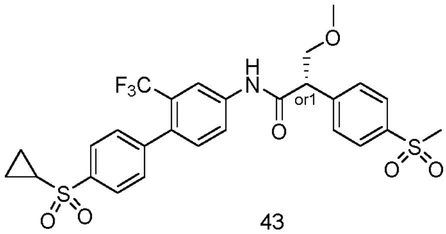
43

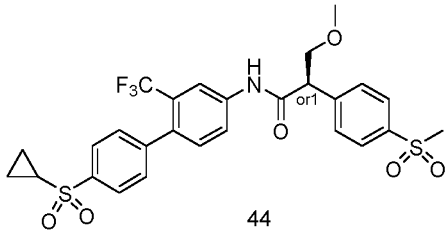
44

Racemate *N*-(4'-(cyclopropylsulfonyl)-2-(trifluoromethyl)-[1,1'-biphenyl]-4-yl)-3-methoxy-2-(4-(methylsulfonyl)phenyl)propanamide (**Example 42,** 90 mg dissolved in about 30 mL methanol, injection volume 2.0 mL) was separated through a Waters SFC 150 (room temperature, 100 bar, 214 nm) and 250*25 mm 10 μm Dr.maish Reprosil Chiral-OM (similar to DAICELCHIRALCEL® OD) (supercritical carbon dioxide : methanol, 55:45, 4.2 min, 70 mL/min) to obtain **Example 43** (37.7 mg, white solid, *Ret.time* = 2.080 min, ee 100%), LC-MS (ESI) *m/z* : 581.9 $[M+H]^+$; $^1H$ NMR (400 MHz, $CD_3OD$) δ 8.15 (d, *J*= 2.0 Hz, 1H), 7.97 - 7.93 (m, 4H), 7.86 (dd, *J* = 8.4, 1.9 Hz, 1H), 7.72 (d, *J* = 8.4 Hz, 2H), 7.55 (d, *J*= 8.2 Hz, 2H), 7.33 (d, *J*= 8.4 Hz, 1H), 4.15 - 4.07 (m, 2H), 3.69 - 3.65 (m, 1H), 3.39 (s, 3H), 3.11 (s, 3H), 2.77 - 2.70 (m, 1H), 1.30 - 1.24 (m, 2H), 1.13 - 1.06 (m, 2H); and **Example 44** (32.3 mg, white solid, *Ret.*time = 2.972 min, ee 98%), LC-MS (ESI) *m/z* : 581.9 $[M+H]^+$; $^1H$ NMR (400 MHz, $CD_3OD$) δ 8.15 (d, *J* = 1.9 Hz, 1H), 7.98 - 7.92 (m, 4H), 7.86 (dd, *J* = 8.4, 1.9 Hz, 1H), 7.72 (d, *J* = 8.4 Hz, 2H), 7.55 (d, *J* = 8.1 Hz, 2H), 7.34 (d, *J* = 8.4 Hz, 1H), 4.16 - 4.06 (m, 2H), 3.69 - 3.65 (m, 1H), 3.39 (s, 3H), 3.11 (s, 3H), 2.77 - 2.69 (m, 1H), 1.30 - 1.25 (m, 2H), 1.13 - 1.07 (m, 2H).

**Activity Examples**

**Activity Example 1:** In vitro determination of agonistic activity of compound on RORγ receptors

A time-resolved fluorescence resonance energy transfer (TR-FRET) assay was used for the compounds of the present disclosure, to determine the agonistic activity of the compounds on the RORγ protein receptor. The agonistic activity is expressed by the concentration for 50% of maximal effect ($EC_{50}$). The experiment was performed basically with refernece to the methods described in ONCOIMMUNOLOGY, 2016, VOL. 5, NO. 12, e1254854.

**Experimental methods:**

1. Preparation of RORγ basal buffer
A 100mL basal buffer was prepared, mixed homogeneously and stored for use;

| | |
|---|---|
| Tris-HCl, pH7.5 | 20 mM |
| EDTA, pH8.0 | 1 mM |
| CHAPS | 0.01% |
| KF | 100mM |
| BSA | 0.01% |
| DTT | 5mM |

2. Preparation of solutions of test compounds

The compounds of the examples shown in the table below were prepared into solutions with DMSO as the solvent, serially diluted at 3-fold dilution to 10 concentrations starting from 10 mM, and added to the corresponding wells of a 384-well plate with each concentration of each compound in duplicate;

The solution of reference substance LYC-55716 was similarly prepared, starting from 10 mM, serially diluted at 3-fold dilution to 10 concentrations, and added to the corresponding control wells of 384-well plate;

3. Preparation of protein solution mixture

a. A 75 nM solution of GST-RORγ LBD ((263-509) (produced at ChemPartner)) and a 4 nM solution of Biotin-SRC1 (Glsbiochem) were prepared with the above basal buffer. The two solutions were 1:1 mixed evenly, and 10 μL of the mixed solution was added into each well of the above 384-well plate. The plate was immediately centrifugated at 1000 rpm for 10 seconds;

b. A 1 nM solution of Anti-GST Eu (Cisbio, #61GSTKLA) and a 50 nM solution of SA-APC (Cisbio, #61GSTKLA) were prepared with the above basal buffer. The two solutions were 1:1 mixed evenly, and 10 μL of the mixed solution was added into each well of the above 384-well plate. The plate was immediately centrifugated at 1000 rpm for 10 seconds;

c. The resulting 384-well plate was incubated at room temperature for 15 minutes. Data were read on an Envision microplate reader, $EC_{50}$ values and E max% were calculated.

4. Analysis of experimental results

a. Readings were taken at 665 nm and 615 nm wavelengths respectively on the Envision Microplate Reader, and the ratios between them were normalized.
b. Calculation was carried out with the following equation:

$$\%Activation = \left(\frac{X - Min}{Max - Min}\right) \times 100\%$$

wherein X is the reading of compounds, Min is the reading of the blank solution, and Max is the reading of 10 μM SRC.
c. The concentration-response curve data were fitted by a four-parameter logistic nonlinear regression model, and Emax was the TOP value of the fitted curve.

Table 1 RORyt protein receptor agonistic activity of Example compounds

| Examples | EC50(nM) | Emax% |
|---|---|---|
| 1 | 14.5 | 212.7 |
| 2 | 9.1 | 209.9 |
| 3 | 14 | 222.9 |
| 4 | 9.6 | 219.9 |
| 5 | 8.5 | 222 |
| 6 | 3.7 | 171.4 |
| 7 | 10 | 168.8 |
| 8 | 10.1 | 169.8 |
| 9 | 22.2 | 159.7 |
| 10 | 114.4 | 158.8 |
| 11 | 6.8 | 167.4 |
| 12-1 | 6.8 | 185.6 |
| 13 | 12.1 | 170.8 |
| 16 | 16.6 | 186.6 |
| 20 | 19.5 | 192.3 |

(continued)

| Examples | EC50(nM) | Emax% |
|---|---|---|
| 22-1 | 7.0 | 179.0 |
| 33 | 13.3 | 201.9 |
| 34 | 5.1 | 191.0 |
| 36 | 4.3 | 200.0 |
| 37 | 27.4 | 197.0 |
| 38 | 5.9 | 190.2 |
| 39 | 13.5 | 181.3 |
| 40 | 9.9 | 199.3 |
| 41 | 24.6 | 174.7 |
| 42 | 12.6 | 194.0 |
| 43 | 6.2 | 193.8 |
| LYC-55716 (Control compound) | 27.9 | 226.2 |
| Note: Emax% is the maximum activation rate. | | |

The test results in the above table show that the compounds of the present disclosure have good agonistic activity on the RORγ protein receptor.

**Activity Example 2:** In vitro assay of agonistic activity of compounds on RORyt in luciferase reporter gene assay

This assay was basically carried out according to the method described in the literature ONCOIMMUNOLOGY, 2016, VOL. 5, NO. 12, e1254854.

The RORγ-LBD coding sequence was inserted into pBIND plasmid (Promega, E1581). This expression vector and a reporter vector (pGL4.35 carrying a stably integrated luciferase reporter gene driven by GAL4 promoter) were co-expressed in HEK293T host cells. When the agonist binds to the corresponding chimeric receptor, the chimeric receptor binds to the GAL4 binding site on the reporter gene carrier and stimulates reporter gene expression. The agonistic activity of the compound on RORγ was determined according to the intensity of the chemiluminescence signal.

Experimental procedure:

1. Preparation of test compounds

1.1 All test compounds were serially diluted at 3-fold dilution with DMSO, with 10 serial dilutions starting from 10mM; LIT-00093 solution was prepared similarly.

1.2 The positive control LYC-55716 was serially diluted at 3-fold dilution with DMSO, with 10 serial dilutions starting from 5 mM.

1.3 1000× positive control (5mM LYC-55716) and 1000× negative control (100% DMSO) were prepared.

1.4 The compound plate was sealed and shaken for 5 min.

2. Test procedure

Cells were cultured according to ATCC standard operation, and $6*10^6$ HEK293T cells were seeded into 100 mm cell culture dishes, cultured overnight in a 37°C, 5% $CO_2$ incubator, and then transfected. Compounds of corresponding concentrations were added for treatment, and the Steady-Glo™ kit (Lot E 2520) was used for further detection.

Chemiluminescence values were detected with Envision 2104; EC50 values were calculated. %Activity was calculated with the following equation:

$$\% \, Activity = \left| \frac{RLU_{cmpd} - \overline{RLU}_{vehicle}}{\overline{RLU}_{positive} - \overline{RLU}_{vehicle}} \right| * 100$$

RLU: Fluorescence value

$\overline{RLU}$ *positive*: average value of positive control

$\overline{RLU}$ *vehicle*: average value of negative control

The log values of the compound concentration and %Activity were fitted using Graphad 8.0, to calculate EC50s of the compounds. The results are shown in Table 2.

Table 2. Luciferase reporter gene assay of RORyt agonistic activity of Example compounds

| Example | EC50 ( nM ) | Emax% |
|---|---|---|
| 1 | 47 | 87.07 |
| 2 | 19 | 43.04 |
| 3 | 14 | 50.53 |
| 4 | 17 | 63.96 |
| 5 | 37 | 69.62 |
| 6 | 13 | 42.08 |
| 7 | 41 | 39.44 |
| 8 | 44 | 37.89 |
| 9 | 53 | 51.47 |
| 10 | 1461 | 58.96 |
| 11 | 15 | 45.04 |
| 12-1 | > 3000 | |
| 13 | 69 | 71.86 |
| 14 | 225 | 40.7 |
| 15 | 202 | 79.49 |
| 16 | 181 | 85.57 |
| 17 | 25 | 33.86 |
| 18 | 784 | 136.9 |
| 19 | 123 | 91.37 |
| 20 | 81 | 63.49 |
| 21 | 72 | 51.25 |
| 22-1 | 139 | 73.84 |
| 33 | 93 | 69.77 |
| 34 | 67 | 70.7 |
| 36 | 54 | 71.28 |
| 38 | 124 | 103.3 |
| 40 | 93 | 77.5 |
| 42 | 105 | 60.71 |
| 43 | 57 | 64.08 |

(continued)

| Example | EC50 ( nM ) | Emax% |
|---|---|---|
| LIT-00093 | 116 | 83.86 |
| LYC-55716 | 157 | 94.19 |
| Note: Emax% is the relative maximum activation rate relative to LYC-55716 at 5 μM. | | |

The test results in the above table show that the compounds of the present disclosure have good agonistic activity on the RORγt luciferase reporter gene.

**Activity Example 3:** Th17 differentiation and activation by the present compounds in mouse lymphocytes

Experiment materials:

| Materials | Supplier | Cat# | Lot# |
|---|---|---|---|
| mouse | C57BL/6J | male | / |
| RPMI1640 | Gibco | A10491-01 | 2037571 |
| Mouse Lymphocyte Isolation Kit | Haoyang Biologicals | LTS1092PK | 2018-12-12 |
| Purified NA/LE hamster anti-mouse CD3 | BD | 553057 | 8172649 |
| Purified NA/LE mouse anti-mouse CD28 | BD | 553294 | 8072791 |
| Recombinant mouse TGF-beta 1 | R&D | 7666-MB/CF | DCKL0218101 |
| Recombinant mouse IL-6 | R&D | 406-ML-005/CF | NUQ2618011 |
| Recombinant mouse IL-23 | R&D | 1887-ML-010/CF | DFXU0418021 |
| Ursolic acid (UA) | Sigma | U6753 | BCBQ8542V |
| Quantikine ® ELISA Mouse IL-17 Immunoassay | R&D | SM1700 | P198693 |

Test procedure: the mouse spleen lymphocytes were first isolated, and the lymphocytes were subject to the conditions of stimulating factors (anti-mCD28: 5 μg/mL; rmTGF-β1: 1 ng/mL; rmIL-6: 50 ng/mL; rmIL- 23: 5 ng/mL) and differentiated to Th17, while adding different concentrations of compounds. All test compounds were serially diluted at 3-fold dilution with DMSO, with 8 serial dilutions starting from the maximum concentration 3 μM. The supernatants were collected after 48 hours for IL-17 ELISA detection, and compared with the solvent group, to determine the activation rate of the compounds on Th17 cells to secrete IL-17. $EC_{50}$ values were fitted with Graphad 8.0, and the TOP values fitted by the software was Emax%.

The test results show that the compounds of the present disclosure have good ability to increase the differentiation of Th17 cells to secrete IL-17 on mouse lymphocytes (as shown in Table 3).

Table 3. Compound stimulated Th17 cells differentiate and secretion of IL-17

| Example | EC50 (nM) | Emax% |
|---|---|---|
| 2 | 114.2 | 203.2 |
| 4 | 111.8 | 156.4 |
| 5 | 11.4 | 147.3 |
| 8 | 370.1 | 85 |
| 9 | 942.8 | 65.4 |
| 11 | 317.7 | 123.0 |

(continued)

| Example | EC50 (nM) | Emax% |
|---|---|---|
| 13 | 5.7 | 133.5 |
| Note: Emax% is the maximum activation rate. | | |

**Activity Example 4:** Th17 differentiation and activation by the present compounds in human PBMC

Experiment materials:

| Materials and Reagents | supplier | Item# | batch number# |
|---|---|---|---|
| hPBMC_ | TPCS | PB100C | A19Z018001 |
| RPMI1640 | Gibco | A10491-01 | 2037571 |
| penicillin-streptomycin | Gibco | 15140-122 | 1953104 |
| fetal bovine serum | Gibco | 10099-141C | 2045686CP |
| Phosphate Buffered Saline (PBS) | Gibco | 10010-031 | 2003918 |
| DMSO | Sigma | D8418-1L | SHBG3288V |
| Purified NA/LE mouse anti-human CD3 | BD | 555336 | 8152611 |
| Purified NA/LE mouse anti-human CD28 | BD | 555725 | 8152601 |
| Recombinant human TGF-beta 1 | R&D | 240-B | A V7117011 |
| Recombinant human IL-6 | R&D | 7270-IL-025/CF | DAOM0318061 |
| Recombinant human IL-23 | R&D | 1290-IL-010/CF | GBI6218031 |
| Ursolic acid (UA) | Sigma | U6753 | BCBQ8542V |
| Quantikine® ELISA Human IL-17 Immunoassay | R&D | S1700 | P192117 |

Test procedure: PBMC cells were first thawed and plated, and then stimulated with stimulating factors (anti-hCD28: 5 μg/mL; rhTGF-β1: 5 ng/mL; rhIL-6: 20 ng/mL; rhIL-23: 10 ng/Ml) to differentiate to Th17, while adding different concentrations of compounds. All test compounds were serially diluted at 3-fold dilution with DMSO, with 8 serial dilutions starting from the maximum concentration 3 μM. The supernatants were collected after 48 hours for IL-17 ELISA detection, and compared with the solvent group, to determine the activation rate of the compounds on Th17 cells to secrete IL-17. $EC_{50}$ values were fitted with Graphad8.0, and the TOP values fitted by the software was Emax%.

The test results show that the compounds of the present disclosure have good ability to increase the differentiation of Th17 cells to secrete IL-17 on on human PBMCs (as shown in Table 4).

Table 4. Compound stimulated Th17 cells differentiate and secretion of IL-17

| Example | EC50 (nM)_ | Emax% |
|---|---|---|
| 2 | 61.61 | 137.8 |
| 4 | 30.4 | 152.4 |
| 5 | 33.2 | 122.9 |
| 6 | 49.23 | 254.0 |
| 8 | 346.9 | 128.9 |
| 9 | 359.7 | 167.1 |
| 11 | 52.76 | 149.2 |
| 13 | 6.2 | 138.8 |

(continued)

| Example | EC50 (nM)_ | Emax% |
|---|---|---|
| 15 | 45.1 | 196.7 |
| 16 | 27.3 | 202.3 |
| 17 | 112.6 | 294.6 |
| 18 | 29.4 | 193.1 |
| 19 | 32.1 | 232.5 |
| 20 | 18.9 | 227.8 |
| 21 | 182.9 | 296.8 |
| 34 | 57.2 | 195.2 |
| 36 | 36.2 | 168.7 |
| 38 | 22.8 | 178.6 |
| 40 | 25.8 | 224.1 |
| 43 | 134.9 | 264.6 |
| Note: Emax% is the maximum activation rate. | | |

**Activity Example 5:** Metabolic stability of human and mouse liver microsomes

According to standard methods of in vitro metabolic stability studies in the art, eg., the methods described in Kerns, Edward H. and Di Li (2008). Drug-like Properties: Concepts, Structure Design and Methods: from ADME to Toxicity Optimization. San Diego: Academic Press; Di, Li et al., Optimization of a Higher Throughput Microsomal Stability Screening Assay for Profiling Drug Discovery Candidates, J. Biomol. Screen. 2003, The method described in 8(4), 453, the liver microsomal metabolic stability test of the compounds of the present disclosure was carried out analogously as follows.

The incubation system contained 0.5 mg protein/mL microsomes, cofactors, and PBS, was preincubated at 37°C for 3 min, and then added the substrate (i.e., the test compounds) to initiate the reaction. Samples were taken at 0, 1, 5, 10, 15, 20, 30 and 60 min from the start of the reaction, and an appropriate terminator was added to terminate the reaction.

The terminator was an ice acetonitrile solution containing 100ng/mL tolbutamide and 100ng/mL labetalol.

| Species | Product information | Supplier | Abbreviation |
|---|---|---|---|
| Human | Cat No. 452117 | Corning | HLM |
| | Lot No. 38292 | | |
| CD-1 mice | Cat No. BQM1000 | Biopredic | MLM |
| | Lot No. MIC255036 | | |

Sample treatment (n = 3): each appropriate sample was added, vortexed and centrifuged at high speed. The supernatants were collected, and the substrate was detected by HPLC-MS/MS. The peak area at the 0 min was taken as 100%. The peak areas at other time points were converted to percentage of the reamining amount. The natural logarithms of the percentage of remaining amount at each time point was plotted against the incubation time, and the slope (-k) was calculated by linear regression. Then, Clint (uL/min/mg) and half-life (T1/2, min) of the compounds were calculated following the equation of the inherent clearance rate (Clint) = (k* volume of incubation solution)/mass of liver microsomes. The results are shown in Table 5.

Table 5. Test results of metabolic stability of human and mouse liver microsomes

| | | human liver microsomes | | | mouse liver microsomes | | |
|---|---|---|---|---|---|---|---|
| Example | cLogP | Clint (uL /min/mg) | T1/2 (min) | % Remaining (T=60min) | Clint (uL /min/mg) | T1/2 (min) | % Remaining (T=60min) |
| 1 | 3.7 | 33.1 | 41.8 | 32.4% | 67.6 | 20.5 | 11.70% |
| 2 | 3.1 | < 9.6 | >145 | 75.7% | < 9.6 | >145 | 89.30% |
| 3 | 4.0 | 9 9.5 | 13.9 | 4.6% | | | |
| 4 | 3.1 | < 9.6 | >145 | 83.4% | 4 9.1 | 28.2 | 22.20% |
| 5 | 2.8 | < 9.6 | >145 | 76.7% | 3 2.7 | 42.4 | 35.50% |
| 6 | 3.7 | < 9.6 | > 145 | 82% | < 9.6 | >145 | 78.60% |
| 7 | 2.6 | < 9.6 | > 145 | 80.1% | < 9.6 | >145 | 88.20% |
| 8 | 2.3 | < 9.6 | > 145 | 95.6% | < 9.6 | >145 | 107.80% |
| 9 | 2.3 | < 9.6 | > 145 | 102.5% | 1 4.1 | 98.6 | 64.20% |
| 10 | 1.7 | < 9.6 | > 145 | 92.5% | < 9.6 | >145 | 89.90% |
| 11 | 3.2 | < 9.6 | > 145 | 110.5% | < 9.6 | >145 | 97.10% |
| 12-1 | 2.8 | < 9.6 | > 145 | 107.4% | 1 0.9 | 126.8 | 71% |
| 13 | 3.6 | 1 1.1 | 124.4 | 71.4% | 4 2.6 | 32.6 | 28% |
| 14 | 2.1 | 14.9 | 92.8 | 61.7% | <9.6 | >145 | 87.3% |
| 15 | 3.0 | 17.8- | 77.9 | 58.7% | < 9.6 | >145 | 82.60% |
| 16 | 3.6 | < 9.6 | >145 | 100.7% | 10.7 | 134.3 | 68.90% |
| 17 | 3.6 | 415 - | 3.3 | 0% | 61.2 - | 22.6 | 15.30% |
| 18 | 3.5 | 120.9 | 11.5 | 2% | 57.6 | 24.1 | 15.10% |
| 19 | 3.0 | 13.0 | 106.6 | 60% | 47.4 | 29.2 | 23.10% |
| 20 | 3.1 | 17.3 | 80.2 | 53.9% | 10.5 | 132.6 | 75.50% |
| 21 | 2.8 | <9.6 | > 145 | 79.6% | 17.3 | 80.1 | 56% |
| 34 | 3.1 | 15.6 | 88.6 | 62.4% | <9.6 | > 145 | 74.5% |
| 36 | 2.3 | 13.7 | 101.2 | 62.3% | <9.6 | > 145 | 83.9% |
| 38 | 2.9 | <9.6 | > 145 | 77.2% | 12.9 | 107.7 | 70.7% |
| 40 | 3.6 | 9.6 | 143.8 | 71.1% | 11.8 | 117.1 | 71.8% |
| 43 | 3.2 | 13.8 | 100.8 | 69% | 14.8 | 93.4 | 64.4% |
| LIT-00093 (control) | 5.8 | 295.4 | 4.7 | 0% | | | |

**Activity Example 6:** Pharmacokinetic (PK) assay of the compounds of the disclosure in mice

The PK of each compound was determined as follows: 6 CD-1 mice (obtained from Shanghai Lingchang Biotechnology Co., Ltd.) were divided into two groups, 3 mice in each group. One group was administered intravenously at a dose of 1 mg/kg, and the vehicle was 5% DMSO/40% PEG400/55% (20% β-CD); one group was administered by oral gavage at a dose of 5 mg/kg, and the vehicle was DMSO/1% methylcellulose (1:99). Blood samples were collected from saphenous vein of lower leg in each group at 0, 0.083, 0.25, 0.5, 1, 2, 4, 6, 8 and 24 h after administration. Approximately 40 μL of the blood samples were collected into anticoagulant tubes containing EDTA-K2. Immediately after collection, the tubes were inverted at least 5 times to ensure uniform mixing, and then placed on ice. The blood samples collected at each time point were centrifuged at 4°C, 8000 rpm for 5 minutes to obtain plasma. Another 1.5 mL centrifuge tube was marked with the compound name, animal number, and time point, and the plasma was transferred

to this tube. The plasma was stored at -80°C until analysis.

Compound concentrations in plasma were determined by UPLC-MS/MS method, and the pharmacokinetic parameters were calculated by Phoenix WinNolin 6.4 pharmacokinetic software on the obtained data.

The specific test results are as follows, showing that the compounds have good pharmacokinetic absorption and have the PK advantages.

Table 6. In vivo PK results of Example compounds

| Ex. | IV 剂量 1 mg/kg | | | | | Po 剂量 5 mg/kg | | | |
|---|---|---|---|---|---|---|---|---|---|
| | AUCO-last (ng/mL*hr) | Cmax (ng/mL) | t1/2 (hr) | Vss (ml/kg) | CL_obs (mL/hr/kg) | AUCO-last (ng/mL*hr) | Cmax (ng/mL) | t1/2 (hr) | F% |
| 2 | 2250 | 737 | 1.76 | 1086 | 426.2 | 12622.9 | 3033.3 | 2.46 | 112.2 |
| 5 | 730.2 | 1360 | 0.45 | 892.9 | 1367 | 1415.97 | 1770 | 0.5 | 38.8 |
| 11 | 7246.9 | 709.3 | 6.33 | 1040.9 | 128.6 | 31190.6 | 3810 | 4.52 | 86.1 |
| 13 | 785.7 | 1323.3 | 0.63 | 712.7 | 1264.7 | 951.7 | 1218.7 | 0.54 | 24.2 |
| 15 | 2301 | 1435 | 1.23 | 0.71* | 7.17** | 4239 | 1061 | 2.32 | 40 |
| 16 | 3662 | 1169 | 3.88 | 1.10* | 4.51** | 10328 | 1417 | 3.3 | 56.3 |
| 19 | 647 | 932 | 0.63 | 1.09* | 26.0** | 924 | 419 | 1.51 | 29.7 |
| 20 | 8280 | 1164 | 4.85 | 0.75* | 1.98** | 21589 | 1657 | 6.95 | 57.3 |
| 21 | 3631 | 716 | 4.19 | 1.45* | 4.55** | 5629 | 611 | 3.83 | 30.9 |
| 35 | 6882 | 1335 | 3.56 | 0.83* | 2.40** | 9140 | 700 | 12.3 | 32.8 |
| 37 | 2170 | 1325 | 2.20 | 1.27* | 7.21** | 1440 | 294 | 2.60 | 15.8 |
| 39 | 2048 | 1084 | 3.70 | 1.53* | 8.22** | 1717 | 400 | 3.00 | 17.1 |
| 41 | 1061 | 601 | 1.70 | 2.02* | 15.4** | 6375 | 1450 | 2.72 | 116 |
| 43 | 3810 | 787 | 4.14 | 1.38* | 4.31** | 17145 | 3037 | 3.51 | 89.1 |
| LYC-55716 | 256.5 | 352.7 | 2.63 | 7231.5 | 3654.6 | 269.3 | 52.6 | 5.35 | 21.0 |
| *: in L/kg<br>**: in mL/min/kg | | | | | | | | | |

The structures of the control compounds used in the above experiments are as follows:

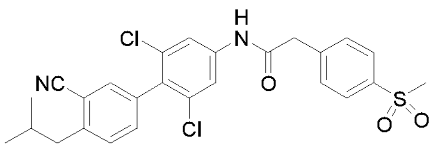

LIT-00093

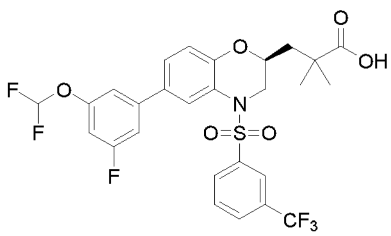

LYC-55716.

## Claims

1. A compound of formula (I) or a pharmaceutically acceptable salt thereof,

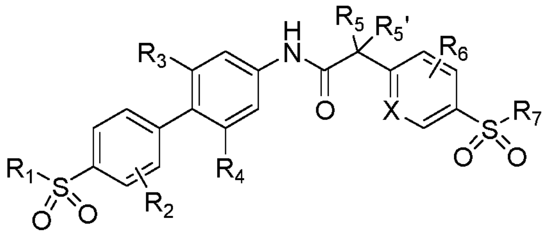

**(I)**

wherein:

$R_1$ is selected from optionally substituted $C_1$-$C_6$ alkyl, optionally substituted $C_3$-$C_6$ cycloalkyl and optionally substituted 3-6 membered heterocycloalkyl, each of the substitutents is independently selected from hydrogen, halogen, cyano, nitro, hydroxyl, mercapto, $C_1$-$C_6$ alkyl, $C_1$-$C_6$ alkoxy, $C_1$-$C_6$ alkylthio and $C_3$-$C_6$ cycloalkyl;
$R_2$, $R_3$, $R_4$ are each independently selected from hydrogen, halogen, cyano, nitro, hydroxyl, mercapto, optionally substituted $C_1$-$C_6$ alkyl, optionally substituted $C_1$-$C_6$ alkoxy and optionally substituted $C_1$-$C_6$ alkylthio, each of the substitutents is independently selected from hydrogen, halogen, hydroxyl, mercapto, nitro and cyano;
$R_5$ and $R_5$' are each independently selected from hydrogen, hydroxy, halogen, nitro, cyano, optionally substituted $C_1$-$C_6$ alkyl, optionally substituted $C_1$-$C_6$ alkoxy, optionally substituted $C_1$-$C_6$ alkylthio and optionally substituted $C_3$-$C_6$ cycloalkyl, each of the substitutents is independently selected from hydrogen, halogen, nitro, cyano, hydroxyl, mercapto, $C_1$-$C_6$ alkoxy and $C_1$-$C_6$ alkylthio;
$R_6$ is selected from hydrogen, halogen, cyano, hydroxyl, mercapto, nitro, optionally substituted $C_1$-$C_6$ alkyl, optionally substituted $C_1$-$C_6$ alkoxy and optionally substituted $C_1$-$C_6$ alkylthio, each of the substituents is independently selected from hydrogen, halogen, nitro, cyano, hydroxyl and mercapto;
$R_7$ is selected from optionally substituted $C_1$-$C_6$ alkyl, optionally substituted $C_3$-$C_6$ cycloalkyl, optionally substituted 3-6 membered heterocycloalkyl, and an amino optionally substituted by $C_1$-$C_6$ alkyl, $C_3$-$C_6$ cycloalkyl or 3-6 membered heterocycloalkyl, each of the substitutents is independently selected from hydrogen, halogen, cyano, nitro, hydroxyl, mercapto, $C_1$-$C_6$ alkoxy, $C_1$-$C_6$ alkylthio, $C_3$-$C_6$ cycloalkyl and an amino optionally substituted with $C_1$-$C_6$ alkyl; and
X is selected from CH and N.

**2.** The compound of formula (I) according to claim 1 or a pharmaceutically acceptable salt thereof, wherein $R_1$ is selected from optionally substituted $C_1$-$C_6$ alkyl and optionally substituted $C_3$-$C_6$ cycloalkyl, each of the substitutents is independently selected from halogen, $C_3$-$C_6$ cycloalkyl and $C_1$-$C_6$ alkoxy; preferably, $R_1$ is selected from $C_1$-$C_6$ alkyl and $C_3$-$C_6$ cycloalkyl; more preferably, $R_1$ is selected from $C_1$-$C_3$ alkyl and $C_3$-$C_5$ cycloalkyl; or more preferably, $R_1$ is selected from methyl, ethyl, propyl and cyclopropyl.

**3.** The compound of formula (I) according to any one of claims 1 to 2 or a pharmaceutically acceptable salt thereof, wherein $R_2$ is selected from H and halogen, preferably H and fluorine.

**4.** The compound of formula (I) according to any one of claims 1 to 3 or a pharmaceutically acceptable salt thereof, wherein $R_3$ and $R_4$ are each independently selected from hydrogen, halogen, cyano, $C_1$-$C_6$ alkyl optionally substituted by halogen, and $C_1$-$C_6$ alkoxy optionally substituted by halogen; more preferably, $R_3$ and $R_4$ are each independently selected from hydrogen, halogen, methyl, trifluoromethyl, trifluoromethoxy and difluoromethoxy; further preferably, $R_3$ and $R_4$ are each independently selected from halogen; most preferably, both $R_3$ and $R_4$ are chlorine.

**5.** The compound of formula (I) according to any one of claims 1 to 4 or a pharmaceutically acceptable salt thereof, wherein $R_5$ and $R_5$' are each independently selected from hydrogen, halogen, substituted $C_1$-$C_3$ alkyl and substituted $C_3$-$C_6$ cycloalkyl, each of the substituents is independently selected from hydroxy, $C_1$-$C_3$ alkoxy and halogen.

**6.** The compound of formula (I) according to claim 5 or a pharmaceutically acceptable salt thereof, wherein $R_5$ is selected from hydrogen, $-CH_2OH$, $-CH_2OCH_3$,

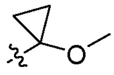 and 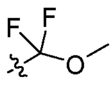 ;

and $R_5$' is H; more preferably, $R_5$ is selected from hydrogen and $-CH_2OCH_3$, and $R_5$' is H.

7. The compound of formula (I) according to any one of claims 1 to 6 or a pharmaceutically acceptable salt thereof, wherein $R_6$ is selected from hydrogen and halogen, preferably hydrogen.

8. The compound of formula (I) according to any one of claims 1 to 7 or a pharmaceutically acceptable salt thereof, wherein $R_7$ is selected from $C_1$-$C_6$ alkyl, $C_1$-$C_6$ alkyl substituted by hydroxyl or $C_3$-$C_6$ cycloalkyl, and $C_3$-$C_6$ cycloalkyl; more preferably, $R_7$ is selected from methyl, ethyl, cyclopropylmethyl and $-CH_2CH_2$-OH; most preferably $R_7$ is selected from methyl, ethyl and - $CH_2CH_2$-OH.

9. The compound of formula (I) according to any one of claims 1 to 8 or a pharmaceutically acceptable salt thereof, wherein the compound of formula (I) has the formula (II):

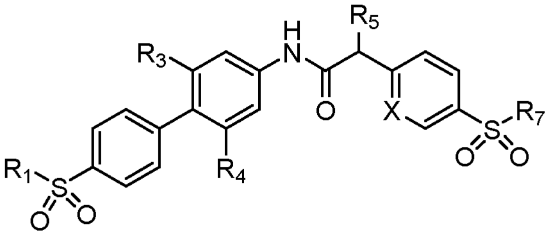

**(II)**

wherein Ri, $R_3$, $R_4$, $R_5$ and $R_7$ have the meanings set forth in any of the preceding claims.

10. The compound of formula (I) according to claim 1 or a pharmaceutically acceptable salt thereof, selected from the following compounds:

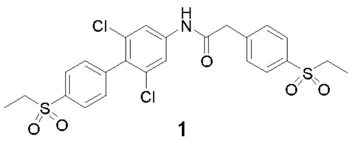

1

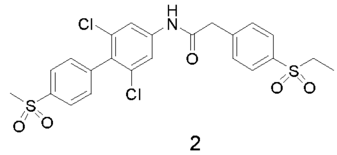

2

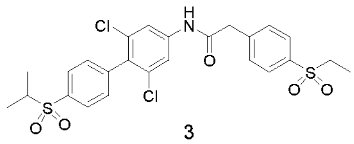

3

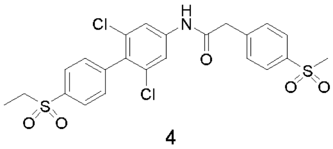

4

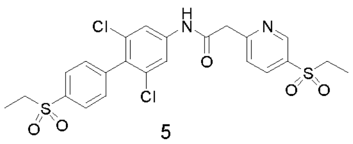

5

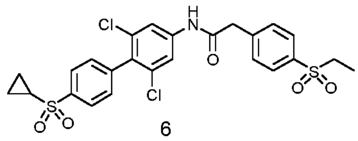

6

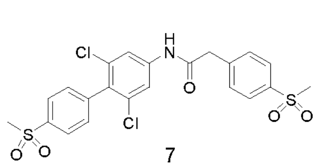

7

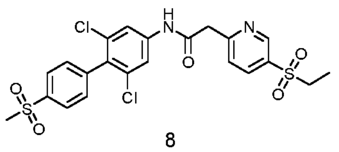

8

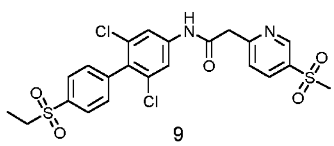

9

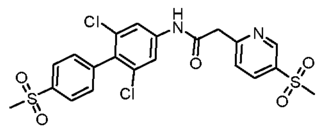
10

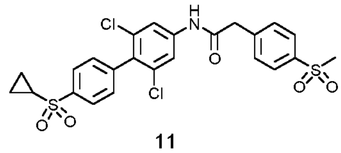
11

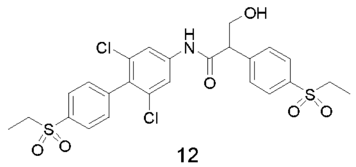
12

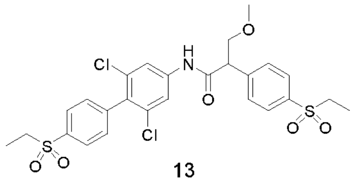
13

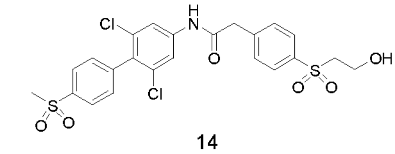
14

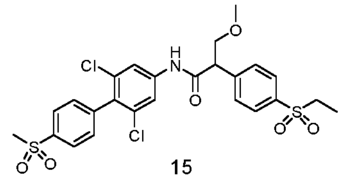
15

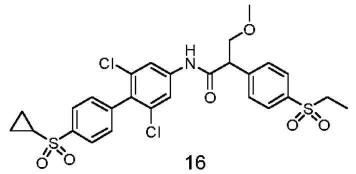
16

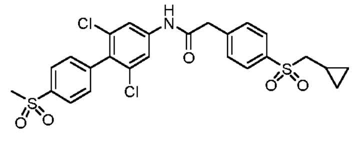
17

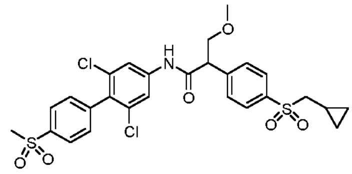
18

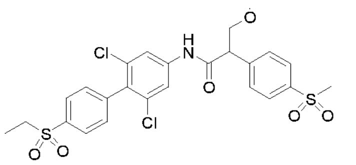
19

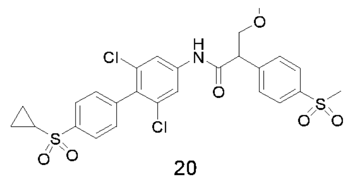
20

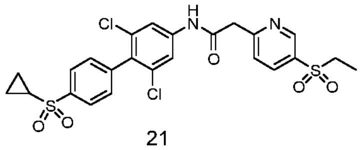
21

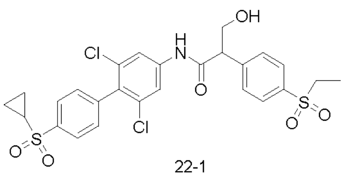
22-1

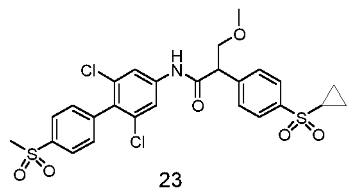
23

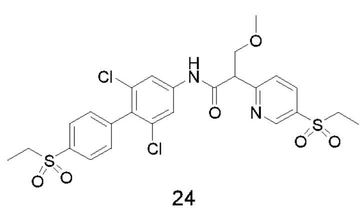
24

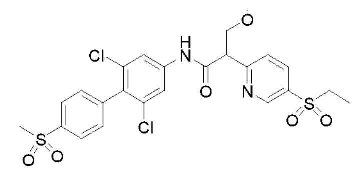
25

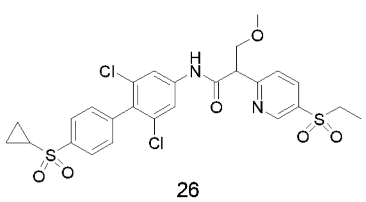
26

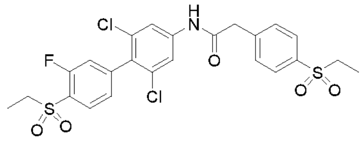
27

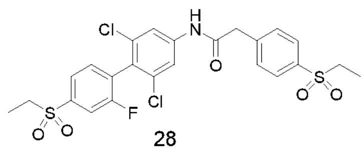
28

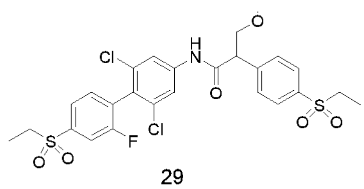
29

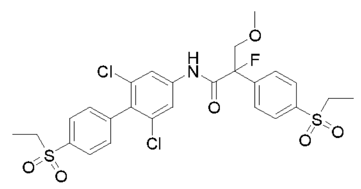

30

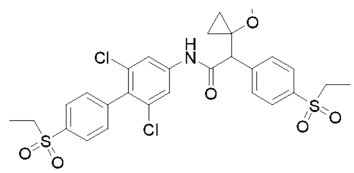

31

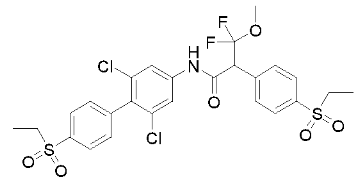

32

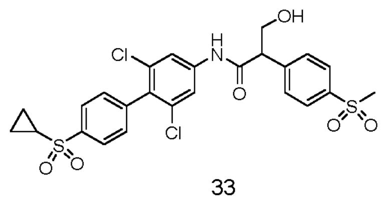

33

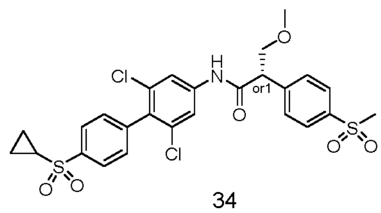

34

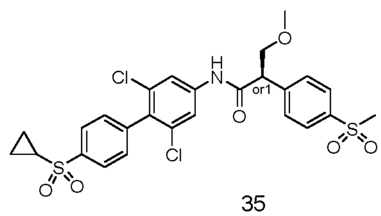

35

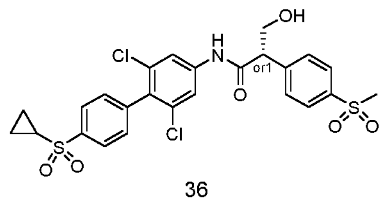

36

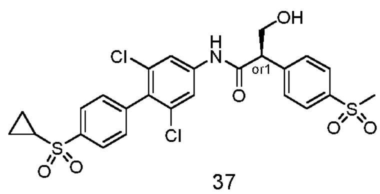

37

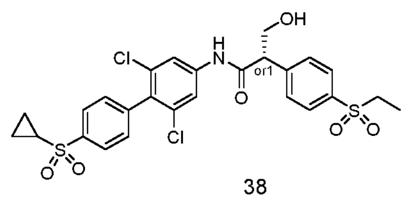

38

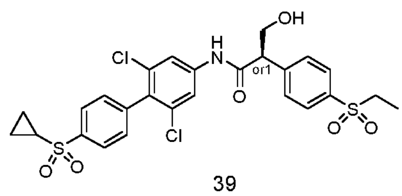

39

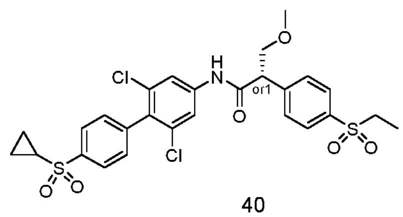

40

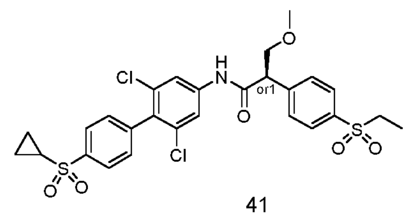

41

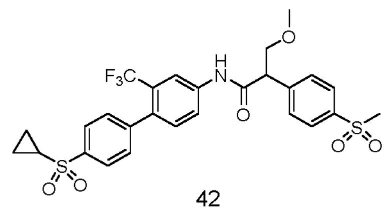

42

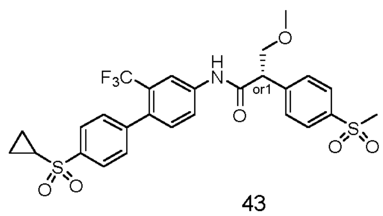

43

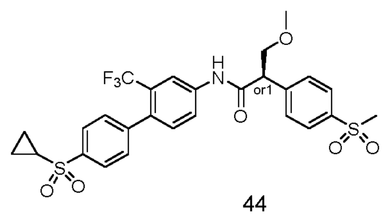

44

.

**11.** The compound according to any one of claims 1 to 10 or a pharmaceutically acceptable salt thereof, for use as a medicament, preferably as a RORγt agonist.

**12.** A pharmaceutical composition comprising a compound according to any one of claims 1 to 10 or a pharmaceutically acceptable salt thereof, and one or more pharmaceutically acceptable carriers.

**13.** Use of a compound according to any one of claims 1 to 10 or a pharmaceutically acceptable salt thereof or a pharmaceutical composition according to claim 12 in the manufacture of a medicament having RORγt receptor agonistic activity, preferably for use in treatment or prevention of a disease associated with RORγt, such as a disease treatable or preventable by activation of RORγt.

**14.** A method of preventing or treating a disease associated with RORγt, such as a disease treatable or preventable by activation of RORγt, comprising administering to an individual in need thereof an effective dose of a compound according to any one of claims 1 to 10 or a pharmaceutically acceptable salt thereof or a pharmaceutical composition according to claim 12.

**15.** The use according to claim 13 or the method according to claim 14, wherein the disease associated with RORγt is selected from tumor or cancer, including but not limited to colon cancer, pancreatic cancer, breast cancer, ovarian cancer, prostate cancer, squamous cell carcinoma, basal cell carcinoma, adenocarcinoma, sweat gland carcinoma, sebaceous gland carcinoma, lung cancer, leukemia, bladder cancer, stomach cancer, cervical cancer, testicular cancer, skin cancer, rectal cancer, thyroid cancer, kidney cancer, pemphigus cancer, liver cancer, acoustic neuroma, oligodendroglioma, meningioma, neuroblastoma and eye cancer.

**INTERNATIONAL SEARCH REPORT**

International application No. **PCT/CN2020/118905**

**A. CLASSIFICATION OF SUBJECT MATTER**

C07C 309/32(2006.01)i; C07D 213/71(2006.01)i; A61K 31/4412(2006.01)i; A61K 31/167(2006.01)i; A61P 31/12(2006.01)i; A61P 35/00(2006.01)i; A61P 37/02(2006.01)i

According to International Patent Classification (IPC) or to both national classification and IPC

**B. FIELDS SEARCHED**

Minimum documentation searched (classification system followed by classification symbols)

C07C309/-; C07D213/-; A61K31/-; A61P31/-; A61P35/-; A61P37/-

Documentation searched other than minimum documentation to the extent that such documents are included in the fields searched

Electronic data base consulted during the international search (name of data base and, where practicable, search terms used)

VEN; CNABS; REGISTRY; CAPLUS; MARPAT; CNKI: 上海辉启, 程耀邦, 黄亚飞, 周娟, 董志强, 视黄酸受体相关孤儿受体, 激动剂, 肿瘤, 癌, retinoid acid receptor-related orphan receptor, ROR+, agonist+, cancer, tumor, structural formula search according to structural formulae (I), (II)

**C. DOCUMENTS CONSIDERED TO BE RELEVANT**

| Category* | Citation of document, with indication, where appropriate, of the relevant passages | Relevant to claim No. |
|---|---|---|
| X | CN 108863850 A (FUDAN UNIVERSITY) 23 November 2018 (2018-11-23) description page 20 embodiment 17, paragraph 0628 table 1, paragraphs 0008-0020, 0071, 0078-0080 | 1-15 |
| A | WO 2013029338 A1 (GLAXO GROUP LTD. et al.) 07 March 2013 (2013-03-07) entire document | 1-15 |
| A | WO 2017157332 A1 (JIANGSU HENGRUI MEDICINE CO., LTD. et al.) 21 September 2017 (2017-09-21) entire document | 1-15 |
| A | WO 2019007382 A1 (JIANGSU HENGRUI MEDICINE CO., LTD. et al.) 10 January 2019 (2019-01-10) entire document | 1-15 |
| A | WO 2015171558 A2 (LYCERA CORP.) 12 November 2015 (2015-11-12) entire document | 1-15 |
| A | CA 2572334 A1 (NEW YORK UNIVERSITY) 19 January 2006 (2006-01-19) entire document | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 November 2020** | **04 January 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/118905**

**C. DOCUMENTS CONSIDERED TO BE RELEVANT**

| Category* | Citation of document, with indication, where appropriate, of the relevant passages | Relevant to claim No. |
|---|---|---|
| A | WO 2016179343 A1 (LYCERA CORP.) 10 November 2016 (2016-11-10)<br>entire document | 1-15 |

Form PCT/ISA/210 (second sheet) (January 2015)

**INTERNATIONAL SEARCH REPORT**

International application No.
**PCT/CN2020/118905**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14-15**
because they relate to subject matter not required to be searched by this Authority, namely:

[1] Claim 14-15 relate to a treatment method applied to the human or animal body (PCT Rule 39.1(iv)). Nevertheless, a search has still been performed on the basis of the technical subject matter of a use of the compound/composition in preparing a corresponding drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/118905**

| Patent document cited in search report | | | Publication date (day/month/year) | Patent family member(s) | | | Publication date (day/month/year) |
|---|---|---|---|---|---|---|---|
| CN | 108863850 | A | 23 November 2018 | JP | 2020508981 | A | 26 March 2020 |
| | | | | US | 2020002280 | A1 | 02 January 2020 |
| | | | | WO | 2018145653 | A1 | 16 August 2018 |
| | | | | EP | 3581561 | A1 | 18 December 2019 |
| WO | 2013029338 | A1 | 07 March 2013 | | None | | |
| WO | 2017157332 | A1 | 21 September 2017 | TW | 201733982 | A | 01 October 2017 |
| | | | | CN | 107531679 | A | 02 January 2018 |
| WO | 2019007382 | A1 | 10 January 2019 | AU | 2018298193 | A1 | 02 January 2020 |
| | | | | TW | 201906816 | A | 16 February 2019 |
| | | | | US | 2020131123 | A1 | 30 April 2020 |
| | | | | KR | 20200024880 | A | 09 March 2020 |
| | | | | BR | 112019026945 | A2 | 30 June 2020 |
| | | | | CA | 3068083 | A1 | 10 January 2019 |
| | | | | EP | 3650448 | A1 | 13 May 2020 |
| | | | | CN | 109952298 | A | 28 June 2019 |
| WO | 2015171558 | A2 | 12 November 2015 | EP | 3209641 | A4 | 06 June 2018 |
| | | | | EP | 3209641 | A2 | 30 August 2017 |
| | | | | US | 2017190659 | A1 | 06 July 2017 |
| | | | | US | 10189777 | B2 | 29 January 2019 |
| | | | | WO | 2015171558 | A3 | 20 July 2017 |
| | | | | JP | 6523337 | B2 | 29 May 2019 |
| | | | | JP | 2017528415 | A | 28 September 2017 |
| CA | 2572334 | A1 | 19 January 2006 | WO | 2006007486 | A2 | 19 January 2006 |
| | | | | JP | 2008505080 | A | 21 February 2008 |
| | | | | US | 2017159057 | A1 | 08 June 2017 |
| | | | | EP | 1771204 | A4 | 13 August 2008 |
| | | | | US | 2015218563 | A1 | 06 August 2015 |
| | | | | EP | 1771204 | A2 | 11 April 2007 |
| | | | | US | 2007154487 | A1 | 05 July 2007 |
| | | | | WO | 2006007486 | A3 | 13 December 2007 |
| WO | 2016179343 | A1 | 10 November 2016 | CA | 2982847 | A1 | 10 November 2016 |
| | | | | JP | 2018515491 | A | 14 June 2018 |
| | | | | US | 10421751 | B2 | 24 September 2019 |
| | | | | AU | 2016257997 | A1 | 09 November 2017 |
| | | | | EP | 3292119 | A1 | 14 March 2018 |
| | | | | US | 2018111922 | A1 | 26 April 2018 |
| | | | | EP | 3292119 | A4 | 03 October 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- The Most Important Scientific Breakthrough. *Science,* 2013
- **DONG.** *Immunity,* 2009, vol. 31, 787-798
- *ACS Chem. Biol.,* 2016, vol. 11, 1012-1018
- *ONCOIMMUNOLOGY,* 2016, vol. 5, e1254854-1, e1254854-13
- *Journal of Medicinal Chemistry,* 2018
- *J. Med. Chem.,* 2018, vol. 61, 5794-5804
- *ONCOIMMUNOLOGY,* 2016, vol. 5 (12), e1254854
- **KERNS ; EDWARD H ; DI LI.** Drug-like Properties: Concepts, Structure Design and Methods: from ADME to Toxicity Optimization. Academic Press, 2008
- **DI, LI et al.** Optimization of a Higher Throughput Microsomal Stability Screening Assay for Profiling Drug Discovery Candidates. *J. Biomol. Screen,* 2003, vol. 8 (4), 453